# EUROPEAN PATENT APPLICATION

(11) **EP 4 245 767 A1**
(43) Date of publication of application: **20.09.2023**
(21) Application number: 21891223.6
(22) Date of filing: 12.11.2021
(51) Int. Cl.: C07K 14/005, C07K 16/10, A61K 39/12, A61K 39/21

(54) **MODIFIED MEMBRANE PROTEIN OF HUMAN IMMUNODEFICIENCY VIRUS AND USE THEREOF**

(30) Priority: 12.11.2020 CN 202011260750
(71) Applicant: Xiamen University, Xiamen, Fujian 361005 (CN); Xiamen Innovax Biotech Co., Ltd., Xiamen, Fujian 361022 (CN)
(72) Inventor: GU, Ying, Xiamen, Fujian 361005 (CN); DENG, Tingting, Xiamen, Fujian 361005 (CN); ZHANG, Hui, Xiamen, Fujian 361005 (CN); HUANG, Fang, Xiamen, Fujian 361005 (CN); CHEN, Gege, Xiamen, Fujian 361005 (CN); LIN, Yanling, Xiamen, Fujian 361005 (CN); LI, Shaowei, Xiamen, Fujian 361005 (CN); XIA, Ningshao, Xiamen, Fujian 361005 (CN)
(74) Representative: Meissner Bolte Partnerschaft mbB
(86) International application number: PCT/CN2021/130414
(87) International publication number: WO 2022/100703

(57) **Abstract**

Provided are a newly designed HIV-1 Env trimer protein, and an HIV-1 pseudovirus and virus expressing the Env trimer protein, and the use thereof for the prevention and/or treatment of HIV infection.

## Description

### Technical Field

The present invention relates to the field of virology. Specifically, the present invention relates to a novel designed HIV-1 Env trimer protein, HIV-1 pseudovirus and virus expressing the Env trimer protein, and use thereof for preventing and/or treating HIV infection.

### Background Art

Human immunodeficiency virus type 1 HIV-1 (Human immunodeficiency virus-1) is the main pathogen that induces AIDS, and belongs to the genus of *Lentivirus,* the family of *Retrovirus.* Its virus particle is composed of core/envelope proteins and genetic materials of diploid single-stranded positive-sense RNA. The single-stranded genome is about 9.8kb, encoding three structural proteins: Env, Gag, Pol, and six auxiliary proteins: vpr, vif, vpu, nef, tat, rev. The auxiliary proteins coordinate with each other to form a regulatory network for HIV-1 viral replication. The Pol gene encodes a polymerase precursor protein, which forms protease (PR), reverse transcriptase (RT) and integrase (IN) after cleavage, which are key enzymes for virus maturation, replication and infection. The Gag protein encoded by the Gag gene is the main structural protein of the virus and is responsible for the assembly process of the virus, accounting for 50% of the entire virus mass; the Gag protein has MA, CA, NC and p6 in sequence from the N-terminal to the C-terminal. MA is a matrix protein, which aggregates to the plasma membrane of infected cells to recruit another structural protein Env so as to start the virus assembly process; CA is a capsid protein, and the capsid protein of mature HIV-1 virus is cone-shaped, which drives virus maturation through protein interaction; NC is a nucleocapsid protein, which can capture the genome during the packaging process of the virus and make it a complete virus particle; p6 has binding sites for other proteins including auxiliary proteins, which are closely related to the process such as replication and transcription of HIV-1. There are PR cleavage sites between different components of Gag protein, and mature particles are gradually formed through its action (Sundquist, W.I. and H.G. Krausslich, HIV-1 assembly, budding, and maturation. Cold Spring Harb Perspect Med, 2012.). Env is the only antigen on the surface of HIV-1. The number of Env trimers on the surface of each virus is very small, about 7-14, but they can effectively trigger the infection of host cells, which shows the importance thereof. Env is a heterotrimeric protein, its precursor is gp160 protein with a molecular weight of 160KD, which is digested by furin enzyme into gp120 and gp41 during the process of virus maturation, and gp120 and gp41 form a heterotrimer through non-covalent interaction; gp120 exists extracellularly, and during virus infection, gp120 recognizes the receptor molecule CD4 on the host cell to trigger virus infection. There are key epitopes such as CD4bs, V1V2, V3, and CD4i on gp120, in which CD4bs epitope recognizes the receptor molecule; gp41 comprises an extracellular region, a membrane-proximal external region (MPER), a transmembrane region (TM) and an intracellular region (CT), on which there are epitopes such as fusion peptide (FP), membrane-proximal external region (MPER), interface between gp120 and gp41; after gp120 recognizes the CD4 molecule, it will induce a change in the conformation of gp120, exposing the co-receptor binding site and binding to the co-receptor on the cell, which will further lead to a conformational change, so that the FP epitope of gp41 is exposed from the viral membrane instantaneously, and inserted into host cells to mediate virus infection (Guttman, Garcia et al. CD4-induced activation in a soluble HIV-1 Env trimer, structure. 2014.).

Env is the main component of many HIV-1 vaccine clinical studies. Early AIDS vaccine researches mostly used gp120 monomer as the antigen, which retained some known Nab epitopes, but it proved difficult to induce Tier2 neutralizing antibodies, and Phase III clinical trials proved that it had no clinical protective effect. The researchers thought that it might because that there are many epitopes on the gp120 monomer that do not exist on the natural Env, and gradually shifted the focus to the native-like Env trimer. However, because the transmembrane region of gp41 is extremely hydrophobic and has low antigenicity as the intracellular region, the researchers increased the soluble expression of Env by removing its transmembrane region and intracellular region, and this type of HIV-1 antigen molecule was named as gp140 (Sanders, R.W. and J.P. Moore. Native-like Env trimers as a platform for HIV-1 vaccine design. Immunol Rev, 2017.). BG505 SOSIP, NFL2P, UFO, etc. are all designed on the basis of gp140. By introducing disulfide bonds in the extracellular region of gp120 and gp41, introducing a mutation I559P for stabilizing into gp41 or replacing the furin cleavage site between gp120 and gp41 with linker, the interaction between subunits and the stability of the trimer are enhanced. These designs can effectively expose the Nab epitope without exposing non-neutralizing epitopes, and thus are considered to well mimic the conformation of the natural trimer on virus. This type of antigen and its virus-like particle antigen prepared by nanotechnology have been used in animal experiments one after another, and it has been confirmed that it can only induce neutralizing antibodies against the same type of virus, with limited broad-spectrum. Therefore, HIV-1 immunogen design still needs to find another way.

The physical and chemical properties of the modified gp140 are optimized. Taking BG505-SOSIP as an example, its trimer content is significantly increased, its thermal stability is about 14°C higher than that of monomer gp120, its binding ability to many kinds of bNabs is maintained, while its binding to some antibodies against non-neutralizing epitopes such as CD4i is weak (Sanders, Derking et al. A next-generation cleaved, soluble HIV-1 Env trimer, BG505 SOSIP.664 gp140, expresses multiple epitopes for broadly neutralizing but not non-neutralizing antibodies, 2013.). Immunization of mice with BG505-SOSIP trimer can induce stronger IgG, Tfh and GC responses, but it cannot induce the production of Tier2 neutralizing antibodies, and the epitope map analysis shows that the induced antibodies are mostly directed to the base region of the trimer (Hu, Crampton et al. 2015, Murine Antibody Responses to Cleaved Soluble HIV-1 Envelope Trimers Are Highly Restricted in Specificity, 2015.), while this region is less masked by glycosyl, but does not exist on the surface of natural virus. This result suggests that the design of Env trimer immunogen should minimize the exposure of non-neutralizing antibodies, and stabilize the conformation of the trimer, which will further advance the HIV-1 vaccine research, but it still has certain limitations to use the Env trimer as a vaccine candidate molecule, for example, it is difficult to induce Tier2 neutralization response, and it is difficult to induce cross-neutralization effect between different strains.

Inactivated or attenuated viruses are the main active ingredients of many vaccines, but so far there is no effective vaccine and radical cure for AIDS. Inactivated or attenuated viruses contain all the antigenic components on the viruses, and can induce the body to produce humoral immunity and cellular immunity simultaneously, induce a strong cytotoxic T lymphocyte response (CTL), and the SIV live attenuated vaccine has been proven to successfully protect rhesus macaques from SIV infection (Protection by Live, Attenuated Simian Immunodeficiency Virus against Heterologous Challenge. Journal of Virology, 1999.). However, due to the pathogenicity and incurability of HIV-1, attenuated or inactivated HIV-1 vaccines have the potential to infect the human body, which limits the application of HIV-1 inactivated or attenuated viruses in the development of AIDS vaccines. It is a very novel and safe vaccine design to obtain a modified HIV-1 virus particle that has completely lost its pathogenicity and cannot regain its infectivity through reverse mutation, and thereby preparing an HIV-1 virus vaccine without potential infection risk, which will bring breakthroughs in the field of AIDS vaccine research and development.

### Contents of the present invention

### Modified gp140/gp160 protein and trimer

In a first aspect, the present invention provides a recombinant protein, which comprises gp120 and gp41 ectodomain (gp41ECTO), wherein the gp120 is located between β27 and α8 of the gp41ECTO. In certain embodiments, the recombinant protein of the present invention is a modified gp140 protein.

Herein, the expression "gp120 is located between β27 and α8 of gp41ECTO" means that the position of gp120 in the recombinant protein of the present invention is between β27 and α8 of gp41ECTO, and is not intended to limit to any specific way of introducing it between β27 and α8. For example, gp120 can be inserted directly between adjacent amino acids between β27 and α8 of gp41ECTO, or gp120 can be used to replace one or more consecutive amino acids between β27 and α8 of gp41ECTO. Furthermore, gp120 can be native or modified, and gp41ECTO can be native or modified. It is easy to understand that gp41ECTO described in the technical solution of the present invention being natural means that the gp41ECTO does not contain other artificial modifications except for the modification of containing gp120 between β27 and α8. Herein, the term "modification" preferably refers to deletion, addition or substitution of one or more amino acid residues.

In some embodiments, the recombinant protein comprises: α6, α7, β27 of gp41ECTO; gp120; α8, α9 of gp41ECTO from the N-terminal to the C-terminal.

In certain embodiments, one or more (e.g., 1-12, 5-12, 5-10; for example 1, 2, 3, 4, 5, 6, 7, 8, 9, 10, 11, or 12) consecutive amino acids in the linkage region between β27 and α8 of the gp41ECTO are substituted with gp120. In certain embodiments, the linkage region corresponds to amino acid positions 607-618 of a gp160 sequence of isolate HXB2.

In certain embodiments, one or more (e.g., 1-7, for example 5-7; for example 1, 2, 3, 4, 5, 6, or 7) consecutive amino acids in a region of the gp41ECTO corresponding to amino acid positions 610-616 of the gp160 sequence of isolate HXB2 are substituted with gp120.

In certain exemplary embodiments, 7 consecutive amino acids in a region of the gp41ECTO corresponding to amino acid positions 610-616 of gp160 sequence of isolate HXB2 are substituted with gp120. In certain embodiments, the linkage region between β27 and α8 of the gp41ECTO comprises a deletion of a sequence set forth in SEQ ID NO: 26 (WNSSWSN) or an amino acid sequence at corresponding position thereof. The expression "amino acid sequence at corresponding position" refers to an amino acid sequence located at a position equivalent to WNSSWSN when the gp160 sequences of different strains are optimally aligned, i.e., when the sequences are aligned for the highest percentage identity. In certain embodiments, the gp120 is located between amino acid positions corresponding to positions 606 and 619 of the gp160 sequence of isolate HXB2.

In certain embodiments, the gp120 is inserted between adjacent amino acids in the linkage region between β27 and α8 of the gp41ECTO. In certain embodiments, the linkage region corresponds to amino acid positions 607-618 of the gp160 sequence of isolate HXB2. In certain embodiments, the gp120 is located between amino acid positions corresponding to positions 609 and 610 of the gp160 sequence of isolate HXB2. In certain embodiments, the gp120 is located between amino acid positions corresponding to positions 616 and 617 of the gp160 sequence of isolate HXB2.

### Alteration of gp120

In some embodiments, the gp120 inserted in gp41ECTO may be a natural gp120. In certain embodiments, the natural gp120 protein corresponds to amino acid positions 31-511 of the gp160 sequence of isolate HXB2.

In other embodiments, the gp120 inserted in gp41ECTO may be a modified gp120, which may comprise a mutation (e.g., substitution, deletion, or insertion) of one or more amino acids compared to the natural gp120.

In certain embodiments, the gp120 is a modified gp120 that comprises a mutation at the furin cleavage site compared to a natural gp120 to prevent the cleavage. The method of modifying the cleavage site to remove furin dependence is well known to those skilled in the art, for example, amino acid substitution, insertion or deletion can be introduced into the furin cleavage site sequence, or the furin cleavage site sequence or part thereof can be replaced with another sequence (e.g., linker sequence).

In certain embodiments, the furin cleavage site corresponds to amino acid positions 508-511 of the gp160 sequence of isolate HXB2. In certain exemplary embodiments, the furin cleavage site is set forth in SEQ ID NO: 41 (REKR).

In certain embodiments, the furin cleavage site in the modified gp120 is deleted compared to the natural gp120.

In certain embodiments, the gp120 is a modified gp120 with 1-11 (e.g., 4-11; for example 1, 2, 3, 4, 5, 6, 7, 8, 9, 10, or 11) amino acids truncated at the C-terminal compared to the natural gp120.

In certain embodiments, the modified gp120 comprises a deletion of one or more (e.g., 1-11, 4-11; for example, 1, 2, 3, 4, 5, 6, 7, 8, 9, 10, or 11) consecutive amino acids in the region corresponding to amino acid positions 501-511 of the gp160 sequence of isolate HXB2.

In certain exemplary embodiments, the modified gp120 has a deletion of a region corresponding to amino acid positions 501-511 of the gp160 sequence of isolate HXB2. In certain embodiments, the modified gp120 is truncated at C-terminal by a sequence set forth in SEQ ID NO: 27 (AKRRVVGREKR) or an amino acid sequence at corresponding position thereof. The expression "amino acid sequence at corresponding position" refers to an amino acid sequence at a position equivalent to AKRRVVGREKR when the gp160 sequences of different strains are optimally aligned, i.e., when the sequences are aligned for the highest percentage identity.

In certain exemplary embodiments, the modified gp120 has a deletion of a region corresponding to amino acid positions 508-511 of the gp160 sequence of isolate HXB2. In certain embodiments, the modified gp120 is truncated at C-terminal with a deletion of a sequence set forth in SEQ ID NO: 41 (REKR) or an amino acid sequence at corresponding position thereof. The expression "amino acid sequence at corresponding position" refers to an amino acid sequence at a position equivalent to REKR when the gp160 sequences of different strains are optimally aligned, i.e., when the sequences are aligned for the highest percentage identity.

In certain embodiments, the gp120 is a modified gp120 with 1-5 (e.g., 1-4; for example 1, 2, 3, 4, or 5) amino acids truncated at the N-terminal, compared to the natural gp120.

In certain embodiments, the modified gp120 has a deletion of a region corresponding to amino acid positions 31-34 of the gp160 sequence of isolate HXB2. In certain embodiments, the modified gp120 comprises a replacement of a region corresponding to amino acid positions 31-34 of the gp160 sequence of isolate HXB2 with an exogenous nucleic acid sequence. In certain embodiments, the exogenous nucleic acid sequence consists of 4 amino acid residues.

In certain embodiments, the modified gp120 has a deletion of an amino acid residue corresponding to amino acid position 499 of the gp160 sequence of isolate HXB2.

In certain embodiments, the modified gp120 is located between amino acid positions corresponding to positions 606 and 610 of the gp160 sequence of isolate HXB2, that is, the modified gp120 is substituted for a region in the gp41ECTO corresponding to amino acid positions 607-609 of the gp160 sequence of isolate HXB2.

In certain embodiments, a disulfide bond is contained between the gp120 and gp41ECTO. In certain embodiments, the recombinant protein has a disulfide bond between amino acid positions corresponding to positions 501 and 605 of the gp160 sequence of isolate HXB2. In certain embodiments, the recombinant protein has a replacement of residues corresponding to amino acid positions 501 and 605 of the gp160 sequence of isolate HXB2 with residue Cys.

In certain embodiments, the gp120 inserted into the gp41ECTO may further comprise a mutation of one or more amino acids to increase the exposure of epitope recognized by broad-spectrum neutralizing antibody and/or stabilize the trimer structure of HIV envelope protein.

In certain embodiments, the gp120 comprises one or more of the following mutations:
(1) the gp120 comprises a substitution of T332N to increase the exposure of epitope recognized by broad-spectrum neutralizing antibody;
(2) the gp120 comprises substitutions of E64K and H66R to stabilize the trimer conformation so that it is not easy to change into open conformation, so that it is not easy to expose CD4i non-neutralizing epitope;
(3) the gp120 comprises a substitution of A316W to enhance the hydrophobic interaction between gp120 subunits, hinder the movement of V3 region, and avoid the exposure of non-neutralizing epitope in the V3 region;
(4) a N-linked glycosylation site (PNGS) near the CD4bs epitope of the gp120 is substituted to prevent glycosylation; preferably, the PNGS is selected from the group consisting of N276, N301, N360, N463;
(5) the gp120 comprises an internal disulfide bond; preferably, the gp120 comprises an internal disulfide bond between I201C and A433C;
(6) a disulfide bond is further contained between the gp120 and gp41ECTO; for example, the recombinant protein has a disulfide bond between E49C and L555C;
the numbering of the above positions is based on the numbering in the gp160 of HIV-1 isolate HXB2.

### Alteration of gp41ECTO

In the recombinant protein of the present invention, the gp41ECTO contains a gp120 between β27 and α8. Other parts of the gp41ECTO can be the corresponding sequences of a natural gp41, or can also contain an artificial modification.

In certain embodiments, the gp41ECTO may comprise a stabilization mutation. Mutations for stabilizing the trimer structure of the HIV envelope protein are known to those skilled in the art, see for example, WO 03/022869. In certain embodiments, the stabilization mutation is I559P, and the numbering of the position is according to the numbering in gp160 of the HIV-1 isolate HXB2.

In the recombinant protein of the present invention, the gp120 inserted into the gp41ECTO can be directly linked to the gp41ECTO, or through a peptide linker.

In certain embodiments, the N-terminal and/or C-terminal of the gp120 is linked to the gp41ECTO optionally via a peptide linker. In certain embodiments, the peptide linker comprises or consists of a sequence represented by (GₘS)ₙ, wherein m is an integer selected from 1-4, and n is an integer selected from 1-3. In certain exemplary embodiments, the peptide linker comprises or consists of a sequence represented by (GₘS)ₙ, wherein m is 4 and n is 1, 2 or 3, preferably 1 or 2.

In the recombinant protein of the present invention, the gp41ECTO and gp120 can be from the same or different HIV-1 strains. In certain embodiments, the gp41ECTO and gp120 are from the same HIV-1 strain.

In the recombinant protein of the present invention, the gp41ECTO and gp120 can be from any subtype of HIV-1, such as group M, N, O or P, or subtype A, B, C, D, F, G, H, J or K etc. In certain embodiments, the HIV-1 strain is selected from the group consisting of subtypes A, B, C, G, BC, AE, DC. In certain exemplary embodiments, the HIV-1 strain is selected from the group consisting of BG505, NL4-3, 246F3, 25710, CH119, CNE8, X1632, Bal.26.

In certain embodiments, the gp160 of BG505 has the sequence set forth in SEQ ID NO: 18. In certain embodiments, the gp160 of NL4-3 has the sequence set forth in SEQ ID NO: 19. In certain embodiments, the gp160 of 25710 has the sequence set forth in SEQ ID NO:20. In certain embodiments, the gp160 of X1632 has the sequence set forth in SEQ ID NO:21. In certain embodiments, the gp160 of CH119 has the sequence set forth in SEQ ID NO:22. In certain embodiments, the gp160 of CNE8 has the sequence set forth in SEQ ID NO:23. In certain embodiments, the gp160 of 246F3 has the sequence set forth in SEQ ID NO:24. In certain embodiments, the gp160 of Bal.26 has the sequence set forth in SEQ ID NO:25.

In certain exemplary embodiments, the recombinant protein of the present invention comprises an amino acid sequence selected from:
(1) an amino acid sequence consisting of amino acid residues at positions 40 to 651 of the sequence set forth in SEQ ID NO: 1;
(2) an amino acid sequence consisting of amino acid residues at positions 40 to 652 of the sequence set forth in SEQ ID NO:2;
(3) an amino acid sequence consisting of amino acid residues at positions 40 to 651 of the sequence set forth in SEQ ID NO:3;
(4) an amino acid sequence consisting of amino acid residues at positions 40 to 652 of the sequence set forth in SEQ ID NO:4;
(5) an amino acid sequence consisting of amino acid residues at positions 40 to 665 of the sequence set forth in SEQ ID NO:5;
(6) an amino acid sequence consisting of amino acid residues at positions 40 to 678 of the sequence set forth in SEQ ID NO:6;
(7) an amino acid sequence consisting of amino acid residues at positions 40 to 661 of the sequence set forth in SEQ ID NO:7;
(8) an amino acid sequence consisting of amino acid residues at positions 40 to 666 of the sequence set forth in SEQ ID NO:8;
(9) an amino acid sequence consisting of amino acid residues at positions 40 to 671 of the sequence set forth in SEQ ID NO:9;
(10) an amino acid sequence consisting of amino acid residues at positions 40 to 676 of the sequence set forth in SEQ ID NO: 10;
(11) an amino acid sequence consisting of amino acid residues at positions 36 to 607 of the sequence set forth in SEQ ID NO: 11;
(12) an amino acid sequence consisting of amino acid residues at positions 36 to 646 of the sequence set forth in SEQ ID NO: 12;
(13) an amino acid sequence consisting of amino acid residues at positions 36 to 648 of the sequence set forth in SEQ ID NO: 13;
(14) an amino acid sequence consisting of amino acid residues at positions 36 to 638 of the sequence set forth in SEQ ID NO: 14;
(15) an amino acid sequence consisting of amino acid residues at positions 36 to 639 of the sequence set forth in SEQ ID NO:15;
(16) an amino acid sequence consisting of amino acid residues at positions 36 to 836 of the sequence set forth in SEQ ID NO:16;
(17) an amino acid sequence consisting of amino acid residues at positions 36 to 673 of the sequence set forth in SEQ ID NO:30;
(18) an amino acid sequence consisting of amino acid residues at positions 36 to 678 of the sequence set forth in SEQ ID NO:31;
(19) an amino acid sequence consisting of amino acid residues at positions 36 to 683 of the sequence set forth in SEQ ID NO:32;
(20) an amino acid sequence consisting of amino acid residues at positions 36 to 678 of the sequence set forth in SEQ ID NO:33;
(21) an amino acid sequence consisting of amino acid residues at positions 36 to 688 of the sequence set forth in SEQ ID NO:34;
(22) an amino acid sequence consisting of amino acid residues at positions 36 to 670 of the sequence set forth in SEQ ID NO:35;
(23) an amino acid sequence consisting of amino acid residues at positions 36 to 676 of the sequence set forth in SEQ ID NO:36;
(24) an amino acid sequence consisting of amino acid residues at positions 36 to 680 of the sequence set forth in SEQ ID NO:37;
(25) an amino acid sequence consisting of amino acid residues at positions 36 to 673 of the sequence set forth in SEQ ID NO:38;
(26) an amino acid sequence consisting of amino acid residues at positions 36 to 678 of the sequence set forth in SEQ ID NO:39;
(27) an amino acid sequence consisting of amino acid residues at positions 36 to 683 of the sequence set forth in SEQ ID NO:40; or
(28) a variant of the sequence described in any one of (1) to (27), in which the variant has a substitution, deletion or addition of one or several amino acids (e.g., a substitution, deletion or addition of 1, 2, 3, 4, 5, 6, 7, 8, 9 or 10 amino acids) or has a sequence identity of at least 80%, at least 85%, at least 90%, at least 91%, at least 92%, at least 93%, at least 94%, at least 95%, at least 96%, at least 97%, at least 98%, or at least 99% compared to the sequence from which it is derived, and the variant retains the properties of the sequence from which it is derived.

In certain embodiments, the recombinant protein of the present invention optionally comprises one or more sequences selected from the following at its N-terminal or C-terminal: signal peptide, translation initiation sequence (e.g., Kozak consensus sequence), tag sequence.

In certain embodiments, the signal peptide is tPA.

In certain embodiments, the translation initiation sequence is a Kozak consensus sequence (Kozak M., Nucleic Acids Research, 1984, 12, 857-872), and the consensus sequence may comprise at least part of the sequence CCRGCCAUGG, where R may be A or G. The position-3 (i.e., position of the 3^{rd} nucleotide upstream of ATG codon) and the position+4 have the greatest effect on translation (Kozak M., Nucleic Acids Research, 1987, 15, 8125-8148). Thus, the consensus sequence may also be RXXAUGG, XXAUGG or RXXAUG.

In certain embodiments, the tag sequence is a purification tag, such as a polyhistidine tag, myc tag, or HA tag.

In certain embodiments, the recombinant protein optionally comprises a signal peptide and/or a translation initiation sequence (e.g., a Kozak consensus sequence) at its N-terminal.

In certain embodiments, the recombinant protein optionally comprises a tag sequence at its C-terminal.

In the second aspect, the present invention provides a fusion protein, which comprises the recombinant protein described in the first aspect and transmembrane region and intracellular region sequences of gp41 linked to its C-terminal. In certain embodiments, the fusion protein of the present invention is a modified gp160 protein.

In certain embodiments, the transmembrane region and intracellular region sequences of gp41 and the gp41ECTO in the recombinant protein are from the same HIV-1 strain.

In certain exemplary embodiments, the fusion protein of the present invention comprises an amino acid sequence selected from:
(1) an amino acid sequence consisting of amino acid residues at positions 33 to 836 of the sequence set forth in SEQ ID NO: 16;
(2) an amino acid sequence consisting of amino acid residues at positions 34 to 837 of the sequence set forth in SEQ ID NO: 17;
(3) a variant of the sequence described in any one of (1) to (2), in which the variant has a substitution, deletion or addition of one or several amino acids (e.g., a substitution, deletion or addition of 1, 2, 3, 4, 5, 6, 7, 8, 9 or 10 amino acids) or has a sequence identity of at least 80%, at least 85%, at least 90%, at least 91%, at least 92%, at least 93%, at least 94%, at least 95%, at least 96%, at least 97%, at least 98%, or at least 99% compared to the sequence from which it is derived, and the variant retains the properties of the sequence from which it is derived.

In certain embodiments, the fusion protein of the present invention optionally comprises one or more sequences selected from the following at its N-terminal or C-terminal: signal peptide, translation initiation sequence (e.g., Kozak consensus sequence), tag sequence.

In certain embodiments, the signal peptide is the signal peptide contained in a natural gp160. In certain embodiments, the signal peptide, the transmembrane region and intracellular region sequences of gp41, and the gp41ECTO and gp120 in the recombinant protein are from the same HIV-1 strain.

In certain embodiments, the tag sequence is a purification tag, such as a polyhistidine tag, myc tag, or HA tag.

In certain embodiments, the recombinant protein optionally comprises a signal peptide and/or a translation initiation sequence (e.g., a Kozak consensus sequence) at its N-terminal.

In certain embodiments, the recombinant protein optionally comprises a tag sequence at its C-terminal.

In a third aspect, the present invention provides a multimer comprising a plurality of monomers, wherein each monomer is independently selected from the recombinant protein described in the first aspect, or independently selected from the fusion protein described in the second aspect.

In certain embodiments, the individual monomers are identical to each other.

In certain embodiments, the multimer is a trimer or a dimer.

### Preparation of envelope protein and trimer

The recombinant protein or fusion protein or multimer thereof of the present invention can be prepared by various methods known in the art, for example, genetic engineering methods (recombinant technology), or chemical synthesis methods (e.g., Fmoc solid phase method). The recombinant protein or fusion protein or multimer thereof of the present invention is not limited by its production method.

In a fourth aspect, the present invention provides an isolated nucleic acid molecule, which comprises a nucleotide sequence encoding the recombinant protein described in the first aspect, the fusion protein described in the second aspect or the multimer described in the third aspect.

In a fifth aspect, the present invention provides a vector, which comprises the isolated nucleic acid molecule as described above. In certain embodiments, the vector is, for example, a plasmid, cosmid, phage, and the like.

In a sixth aspect, the present invention provides a host cell, which comprises the isolated nucleic acid molecule or the vector as described above. Such host cells include, but are not limited to, prokaryotic cell such as E. coli cell, and eukaryotic cell such as yeast cell, insect cell, plant cell, and animal cell (e.g., mammalian cell, such as mouse cell, human cell, etc.). Preferably, the host cell is a mammalian cell, such as a human cell.

In the seventh aspect, the present invention provides a method for preparing the recombinant protein described in the first aspect, the fusion protein described in the second aspect, or the multimer described in the third aspect, which comprises culturing the host cell of the sixth aspect under suitable conditions, and recovering the recombinant protein, the fusion protein or the multimer from a cell culture. In some embodiments, the recombinant protein or the fusion protein exists in the form of multimer (e.g., trimer or dimer).

### Display platform

The protein described in the first or second aspect of the present invention or the multimer described in the third aspect can be displayed on particles such as liposomes, virus-like particles (VLPs), nanoparticles, virosomes or exosomes to enhance in vivo antigen presentation efficacy.

Therefore, in the eighth aspect, the present invention provides a particle, displaying on its surface the recombinant protein described in the first aspect, the fusion protein described in the second aspect or the multimer described in the third aspect on its surface.

In certain embodiments, the particle is a liposome or a nanoparticle.

In certain embodiments, the recombinant protein, the fusion protein or the multimer of the present invention is fused to and/or displayed on a liposome. The liposome is a spherical vesicle with at least one lipid bilayer. In certain embodiments, the multimer protein (e.g., trimer or dimer protein) of the present invention can for instance be non-covalently coupled to such liposomes by electrostatic interactions, e.g. by adding a His-tag to the C-terminus of the multimer and a bivalent chelating atom such as Ni2+ or Co2+ incorporated into the head group of derivatized lipids in the liposome. In certain embodiments, the multimer protein (e.g., trimer or dimer protein) of the present invention is covalently coupled to the surface of liposome, for example, via a maleimide functional group integrated in the liposome surface. In certain embodiments, the multimer protein (e.g., trimer or dimer protein) of the present invention can be coupled thereto, for example, via a C-terminal cysteine added in the multimer protein. Methods for preparing HIV Env trimers coupled to liposomes and their characterization, are known and described in detail, for example, in Bale S et al. J Virol. 2017;91(16):e00443-17, and this document is incorporated herein by reference.

In certain embodiments, the recombinant protein, the fusion protein or the multimer of the present invention is fused to a self-assembling particle or displayed on a nanoparticle. Antigen nanoparticles are assemblies of polypeptides that present multiple copies of antigen (e.g., the HIV Env protein of the present invention), resulting in multiple binding sites (avidity) and providing an improved antigen stability and immunogenicity. The preparation of self-assembling protein nanoparticle and use thereof in vaccines are well known to those skilled in the art, see for example, Zhao L et al. (2014) Vaccine 32:327-337, López-Sagaseta J et al. (2016) Computational and Struct Biotechnol J 14:58-68. As non-limiting examples, the self-assembling nanoparticle may be based on ferritin, bacterial ferritin or DPS. DPS nanoparticles displaying proteins on the surface are described in, for example, WO 2011/082087. The description of trimeric HIV-1 antigens on such particles has been described in, for example, He L et al. (2016) Nat Commun. 2016 Jun 28;7:1 2041. Other self-assembling protein nanoparticles and their preparation are disclosed in, for example, WO 2014/124301 and US 2016/0122392, which are hereby incorporated by reference.

### Pseudoviral particle

In the ninth aspect, the present invention provides a pseudoviral particle, comprising on its surface the recombinant protein described in the first aspect, the fusion protein described in the second aspect or the multimer described in the third aspect.

The pseudoviral particle of the present invention presents a typical form of pseudoviral particle, but has completely lost its infection ability. Compared with traditional protein vaccines, this type of pseudoviral particle not only has components such as envelope and capsid proteins of HIV-1 virus, etc., with high similarity to the natural virus in shape, but also has completely lost the infection ability, has good immunogenicity, and thus can be used as a virus-based vaccine.

In certain embodiments, the pseudoviral particle is produced by a lentiviral packaging system or a retroviral packaging system.

In certain embodiments, the pseudoviral particle is obtained by co-expressing (i) a vector comprising the nucleic acid molecule described in the fourth aspect, and (ii) a packaging vector (e.g., a backbone plasmid) in a host cell.

In certain embodiments, the packaging vector is capable of expressing gag, pol, tat, rev genes. In certain embodiments, the packaging vector is a vector comprising an HIV-1 genome with a deletion of env gene. In certain embodiments, the packaging vector is a plasmid. In certain embodiments, the backbone plasmid includes, for example, pSPAX, pNL4-3.Luc.R-E-, pSG3Δenv, pfNL43-dGPE-EGFP.

In certain embodiments, the vector described in (i) is an eukaryotic expression vector, including but not limited to, VRC8400, PTT5, pCDN3.1 and the like.

In another aspect, the present invention provides a method for preparing the pseudoviral particle of the present invention, which comprises: (1) co-transfecting a host cell with the expression vector comprising the nucleic acid molecule described in the fourth aspect and the packaging vector; (2) expressing the proteins encoded by the expression vector and the packaging vector in the host cell, in which the proteins are capable of self-assembling to form an HIV pseudovirus; and (3) collecting the HIV pseudovirus.

In certain preferred embodiments, the host cell is an eukaryotic cell, such as a mammalian cell, such as a primate cell, such as a human cell.

In another aspect, the present invention provides a packaging system for producing the above-mentioned pseudoviral particle, which comprises: (i) an expression vector comprising the nucleic acid molecule described in the fourth aspect, (ii) a packaging vector.

### Modified HIV virus

In the tenth aspect, the present invention provides a modified HIV virus, which expresses the fusion protein or multimer (e.g., trimer or dimer) thereof described in the second aspect as its envelope protein. In certain embodiments, the HIV virus is HIV-1 virus.

The modified HIV virus of the present invention will lose the ability to infect cells and animals, and as an immunogen of vaccine, it can simulate the immune response process of HIV-1 infection of the human body to the greatest extent, thus may produce protective immunity, and at the same time has good safety. The modified HIV virus of the present invention can also simulate a natural variation of HIV-1 virus when it is cultured in cells, and this kind of variation cannot be back-mutated to form an infectious virus particle, because the viral replication process cannot complete the rearrangement with the gene fragment encoding the Env protein of the present invention. Therefore, this type of live virus vaccine will have the immunogenicity of the natural virus to the body, but it is impossible to have infectivity and pathogenicity, so that it is also a feasible vaccine strategy.

In certain embodiments, the genome of the modified HIV virus comprises the following modification: the wild-type env gene is replaced with the nucleotide sequence encoding the fusion protein described in the second aspect.

In an eleventh aspect, the present invention provides an isolated nucleic acid molecule or vector, which comprises a nucleotide sequence encoding the genome of the modified HIV virus described above.

### Composition

In the twelfth aspect, the present invention provides a composition, which comprises the recombinant protein described in the first aspect, the fusion protein described in the second aspect or the multimer described in the third aspect, the isolated nucleic acid molecule described in the fourth aspect, the vector described in the fifth aspect, the host cell described in the sixth aspect, the particle described in the eighth aspect, the pseudoviral particle described in the ninth aspect, the modified HIV virus described in the tenth aspect, or the isolated nucleic acid molecule or vector described in the eleventh aspect.

In certain embodiments, the composition further comprises a pharmaceutically acceptable carrier and/or excipient.

In certain embodiments, the composition is an immunogenic composition or vaccine. In such embodiments, the recombinant protein described in the first aspect, the fusion protein described in the second aspect or the multimer described in the third aspect, the isolated nucleic acid molecule described in the fourth aspect, the vector described in the fifth aspect, the host cell described in the sixth aspect, the particle described in the eighth aspect, the pseudoviral particle described in the ninth aspect, the modified HIV virus described in the tenth aspect, or the isolated nucleic acid molecule or vector described in the eleventh aspect is used as an immunogen.

In certain embodiments, the composition is a protein vaccine, which comprises the recombinant protein described in the first aspect, the fusion protein described in the second aspect, or the multimer described in the third aspect, or the particle described in the eighth aspect as an immunogen.

In some embodiments, the composition is a virus-based vaccine, which comprises the pseudoviral particle described in the ninth aspect, or the modified HIV virus described in the tenth aspect as an immunogen.

In certain embodiments, the composition is a nucleic acid vaccine, which comprises the isolated nucleic acid molecule described in the fourth aspect, the vector described in the fifth aspect, or the isolated nucleic acid molecule or vector described in the eleventh aspect as an immunogen. As used herein, the term "nucleic acid vaccine" refers to a vaccine based on DNA or RNA (e.g., plasmid, such as expression plasmid), optionally further comprising an adjuvant.

In certain embodiments, the nucleic acid vaccine comprises DNA or RNA. In certain embodiments, the DNA or RNA can be naked or can be packaged within a shell with delivery and/or protection functions. In some embodiments, the shell can be a shell of adenovirus, adeno-associated virus, lentivirus, retrovirus, etc., or other material that is synthesized by a chemical method and capable of performing similar function.

In certain embodiments, the pharmaceutically acceptable carrier and/or excipient comprises an adjuvant. Adjuvants for co-administration with or being included in the composition according to the present invention should preferably be those that are potentially safe, well tolerated and effective in humans. Such adjuvants are well known to those skilled in the art, and non-limiting examples thereof include: QS-21, Detox-PC, MPL-SE, MoGM-CSF, TiterMax-G, CRL-1005, GERBU, TER amide, PSC97B, Adjumer, PG-026, GSK-I, GcMAF, B-alethine, MPC-026, Adjuvax, CpG ODN, Betafectin, aluminum salts such as aluminum phosphate (e.g., AdjuPhos) or aluminum hydroxide and MF59.

The immunogenic composition or vaccine of the present invention can also be used in combination with an additional agent known in the art for the treatment or prevention of an HIV infection. Thus, in certain embodiments, the composition of the present invention may also comprise an antiretroviral agent. In certain embodiments, the antiretroviral agent comprises: a nucleoside reverse transcriptase inhibitor, such as abacavir, AZT, didanosine, emtricitabine, lamivudine, stavudine, tenofovir, zalcitabine, zidovudine, etc.; a non-nucleoside reverse transcriptase inhibitor, such as delavirdine, efavirenz, nevirapine; a protease inhibitor, such as amprenavir, atazanavir, indinavir, lopinavir, nelfinavir, osamprenavir, ritonavir, saquinavir, tipranavir, etc., and a fusion protein inhibitor, such as enfuvirtide and so on.

In certain embodiments, the immunogenic composition or vaccine of the present invention and the antiretroviral agent are present as separate components or as a single formulation. In certain embodiments, the immunogenic composition or vaccine of the present invention and the antiretroviral agent are present as separate components or as a single formulation. In certain embodiments, the immunogenic composition or vaccine of the present invention and the antiretroviral agent may be administered simultaneously, separately or sequentially.

The composition (e.g., immunogenic composition) of the present invention may be formulated into any dosage form known in the medical art, for example, tablets, pills, suspensions, emulsions, solutions, gels, capsules, powders, granules, elixirs, lozenges, suppositories, injections (including injections, sterile powders for injection and concentrated solutions for injection), inhalants, sprays, etc. The preferred dosage form depends on the intended mode of administration and therapeutic use. The composition of the present invention should be sterile and stable under the conditions of manufacture and storage. A preferred dosage form is injection. Such injection can be a sterile injectable solution. In addition, the sterile injectable solution can be prepared as a sterile lyophilized powder (e.g., by vacuum drying or freeze-drying) for ease of storage and use. Such sterile lyophilized powder can be dispersed in a suitable vehicle before use, such as water for injection (WFI), bacteriostatic water for injection (BWFI), sodium chloride solution (e.g., 0.9% (w/v) NaCl), dextrose solution (e.g., 5% dextrose), surfactant-containing solution (e.g., 0.01% polysorbate 20), pH buffer solution (e.g., phosphate buffered saline), Ringer's solution and any combination thereof.

The composition (e.g., immunogenic composition) of the present invention may be administered by any suitable method known in the art, including, but not limited to, oral, buccal, sublingual, ocular, topical, parenteral, rectal, intrathecal, intra-cisterna, inguinal, intravesical, topical (e.g., powder, ointment, or drop), or nasal routes. However, for many therapeutic uses, the preferred route/mode of administration is parenteral administration (e.g., intravenous or bolus injection, subcutaneous injection, intraperitoneal injection, intramuscular injection). The skilled artisan will understand that the route and/or mode of administration will vary depending on the intended purpose.

The immunogenic composition of the present invention should be administered in an amount sufficient to induce an immune response against HIV-1. The appropriate amount of immunogen can be determined according to the particular disease or condition to be treated or prevented, its severity, the age of the subject, and other personal attributes of the particular subject (e.g., the general state of the subject's health and the robustness of the subject's immune system). Determination of effective doses is also guided by animal model studies followed by human clinical trials, and by administration regimens that significantly reduce the occurrence or severity of the target disease symptom or condition in subjects.

### Applications

The composition (e.g., immunogenic composition) of the present invention can readily be used in a variety of therapeutic or prophylactic applications for treating an HIV-1 infection or inducing an immune response to HIV-1 in a subject. For example, the composition can be administered to a subject to induce an immune response to HIV-1, for example, to induce broad-spectrum neutralizing antibodies against HIV-1. To subjects at risk of developing HIV infection, the immunogenic composition of the present invention can be administered to provide prophylactic protection against viral infection.

Accordingly, in another aspect, the present invention provides a method for inducing an immune response against HIV in a subject or for preventing and/or treating an HIV infection in a subject, which comprises administering to the subject in need thereof an immunologically effective amount of the recombinant protein described in the first aspect, the fusion protein described in the second aspect or the multimer described in the third aspect, the isolated nucleic acid molecule described in the fourth aspect, the vector described in the fifth aspect, the host cell described in the sixth aspect, the particle described in the eighth aspect, the pseudoviral particle described in the ninth aspect, the modified HIV virus described in the tenth aspect, or the isolated nucleic acid molecule or vector described in the eleventh aspect.

In certain embodiments, the method may further comprise administering to the subject an additional agent in combination for the treatment or prevention of an HIV infection. In certain embodiments, the method comprises administering an antiretroviral agent, such as a nucleoside reverse transcriptase inhibitor, a non-nucleoside reverse transcriptase inhibitor, a protease inhibitor, or a fusion protein inhibitor, among others.

For prophylactic applications, the recombinant protein, the fusion protein, the multimer, the isolated nucleic acid molecule, the vector, the host cell, the particle, the pseudoviral particle, the modified HIV virus, or the composition (e.g., immunogenic composition) of the present invention is provided before any symptoms, such as before infection. The prophylactic administration of the immunogenic composition is used for the prevention or amelioration of any subsequent infection, to attenuate the expected severity, duration or extent of infection and/or associated disease symptoms following the exposure or suspected exposure to the virus or after actual infection. Thus, in some embodiments, the subject to be treated is a subject who has an HIV infection or is at risk of developing an HIV infection, for example, being or likely being exposed to HIV. Following the administration of a therapeutically effective amount of the disclosed therapeutic composition, HIV-1 infection, or symptoms associated with HIV-1 infection of the subject can be monitored.

For therapeutic applications, the recombinant protein, the fusion protein, the multimer, the isolated nucleic acid molecule, the vector, the host cell, the particle, the pseudoviral particle, the modified HIV virus, or the composition (e.g., immunogenic composition) of the present invention is provided at or after the onset of symptoms of a disease or infection, for example, after the onset of symptoms of HIV-1 infection or after a diagnosis of HIV-1 infection.

In another aspect, the present invention also relates a use of the recombinant protein described in the first aspect, the fusion protein described in the second aspect or the multimer described in the third aspect, the isolated nucleic acid molecule described in the fourth aspect, the vector described in the fifth aspect, the host cell described in the sixth aspect, the particle described in the eighth aspect, the pseudoviral particle described in the ninth aspect, the modified HIV virus described in the tenth aspect, or the isolated nucleic acid molecule or vector described in the eleventh aspect in the manufacture of a medicament for inducing an immune response against HIV in a subject and/or for preventing and/or treating an HIV infection in a subject. Preferably, the medicament is a vaccine. Preferably, the subject is a human.

In another aspect, the present invention also relates to a use of the recombinant protein described in the first aspect, the fusion protein described in the second aspect or the multimer described in the third aspect, or the particle described in the eighth aspect in the manufacture of a protein vaccine, the protein vaccine is used for inducing an immune response against HIV in a subject and/or for preventing and/or treating an HIV infection in a subject.

In another aspect, the present invention also relates to a use of the pseudoviral particle described in the ninth aspect, or the modified HIV virus described in the tenth aspect, in the manufacture of a virus-based vaccine, and the virus-based vaccine is used for inducing an immune response against HIV in a subject and/or for preventing and/or treating an HIV infection in a subj ect.

In another aspect, the present invention also relates to a use of the isolated nucleic acid molecule described in the fourth aspect, the vector described in the fifth aspect, or the isolated nucleic acid molecule or vector described in the eleventh aspect in the manufacture of a nucleic acid vaccine, the nucleic acid vaccine is used for inducing an immune response against HIV in a subject and/or for preventing and/or treating an HIV infection in a subject.

### Definition of Terms

In the present invention, unless otherwise specified, the scientific and technical terms used herein have the meanings commonly understood by those skilled in the art. In addition, the laboratory procedures of virology, biochemistry, nucleic acid chemistry, and immunology used herein are routine procedures widely used in the corresponding fields. Meanwhile, in order to better understand the present invention, definitions and explanations of related terms are provided below.

As used herein, the term "Env" refers to an envelope protein (envelope) on the surface of HIV-1 virus. Env is synthesized on the surface of HIV-1 in the form of full-length gp160 (full-length molecular weight 160KD), and the precursor protein formed by translation is cleaved by protease into gp120 (with a molecular weight of 120KD) and gp41 after the viral maturation, in which gp 120 is located on the surface of virus and is a molecule that recognizes CD4 receptor and induces infection, while gp41 comprises an extracellular region transmembrane region and an intracellular region and has a molecular weight of 41KD, gp120 and gp41 form a heterodimer through non-covalent interaction between subunits, and the heterodimer forms a typical trimer structure through non-covalent interaction. This trimer protein is Env.

As used herein, the term "gp140" refers to a protein composed of gp120 and gp41 extracellular segment in which the gp41 intracellular segment and transmembrane region have been deleted, and the trimer formed by the monomeric protein can be called as gp140 trimer. The gp140 trimer includes trimer proteins whose conformation is further stabilized by introducing a disulfide bond, a point mutation, etc. into monomers. The "BG505 SOSIP" herein refers to that on the basis of the full-length Env of the BG505 strain, the intracellular segment and the transmembrane region sequences are deleted, and cys mutations are introduced at the amino acid position 501and the amino acid position 605 to form a disulfide bond between gp120 and gp41 extracellular regions, and Ile at the amino acid position 559 is further mutated into pro to stabilize the pre-fusion conformation of gp140 trimer, this modification method is named as SOSIP, and this modified protein is BG505 SOSIP. The deletion of the intracellular and transmembrane regions of gp160 will facilitate the soluble expression of membrane proteins.

As used herein, the term "furin" refers to a major protein convertase in the secretory pathway that catalyzes the cleavage of the carboxy-terminal peptide bond of Arg-X-Y-Arg in proteins (X is any amino acid, Y is Arg or Lys) to produce mature proteins. Furin recognizes the REKR sequence between the immature HIV-1 Env gp120 and gp41 subunits and cleaves the full-length gp160 into gp120 and gp41, allowing them to undergo conformational rearrangement and maturation.

As used herein, the HXB2 numbering system is a reference numbering system for HIV proteins and nucleic acid sequences, using the HIV-1 HXB2 strain sequence as a reference for all other HIV strain sequences. Those of ordinary skill in the art are familiar with the HXB2 numbering system. The amino acid numbers described herein, such as 559, 501, 605, etc., are given according to the HXB2 numbering system, which can be determined by using the amino acid numbering of the strain HXB2 as a template and performing sequence homologous alignment, and those of ordinary skill in the art will understand that the amino acid numbers described herein do not refer to the actual amino acid number in the protein. Herein, the expression "a region corresponding to specific amino acid positions of gp160 sequence of isolate HXB2" refers to the positions in a strain to be compared that are equivalent to the specific amino acid positions in isolate HXB2 when the gp160 sequence of the strain to be compared is optimally aligned with the gp160 sequence of isolate HXB2, that is, when the sequences are aligned to obtain the highest percent identity.

As used herein, "subtype," "strain," etc. of HIV are well known to those skilled in the art. It is now known that HIV-1 has at least 13 subtypes, including A, B, C, D, E, F, G, H, I, J, K, O, and N subtypes, which are classified as three groups M, O and N, in which A to K belong to the group M. There are many strains in each of different subtypes, and one strain corresponds to one Env sequence. For example, BG505 used in the present invention is a strain in the subtype A, and NL4-3 and Bal.26 are strains in the subtype B.

As used herein, the term "vector" refers to a nucleic acid delivery vehicle into which a polynucleotide can be inserted. When the vector is capable of achieving expression of the protein encoded by the inserted polynucleotide, the vector is called an expression vector. A vector can be introduced into a host cell by transformation, transduction or transfection, so that the genetic material elements it carries can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC) or P1-derived artificial chromosome (PAC); phages such as λ phage or M13 phage and animal viruses, etc. Animal viruses that can be used as vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40). A vector may contain a variety of elements that control expression, including but not limited to, promoter sequence, transcription initiation sequence, enhancer sequence, selection element, and reporter gene. In addition, the vector may also contain an origin of replication.

In the present invention, the term "packaging vector" refers to a vector capable of expressing proteins necessary for the formation of HIV virus-like particle (VLP) other than the envelope protein, including gag, pol, tat and vpu proteins of HIV. Usually, the packaging vector can be constructed by modifying or deleting the coding gene and regulatory gene of envelope protein in the complete HIV genome. Furthermore, packaging vectors for assembly of HIV VLP/pseudovirus and methods for constructing such packaging vectors are known in the art.

In the present invention, the term "backbone plasmid" refers to a plasmid used to encode structural proteins such as gag and pol in the production of pseudovirus. When using the lentivirus system to produce pseudovirus, packaging plasmid, envelope plasmid, transfer plasmid and regulatory plasmid are required to be co-transfected into a packaging cell to produce a pseudoviral particle for easy detection; the transfer plasmid generally contains a reporter gene that can indicate the infection and entry of pseudovirus into the cell; the reporter gene can be integrated into the packaging plasmid; the regulatory gene can regulate the transcription and translation of the pseudovirus structural gene, and can also be integrated into the packaging plasmid.

As used herein, the term "host cell" refers to a cell into which a vector can be introduced, which includes but is not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As used herein, the term "pseudovirus" refers to a virus-like particle formed by viral capsid protein or envelope protein, which generally does not encapsulate nucleic acid or encapsulate viral nucleic acid after gene deletion or modification. Generally speaking, because the pseudovirus does not contain nucleic acid or the viral nucleic acid genome contained therein is incomplete, the pseudovirus only has the ability of single-round infection, but does not have the ability to replicate to produce progeny viruses, and thus has high biological safety.

As used herein, the term "Tier2 strain" is well known to those skilled in the art. HIV-1 strains can be neutralized by some specific antibodies. According to the difficulty of being neutralized, HIV-1 strains are divided into three levels: Tier1, Tier2 and Tier3. Tier1 refers to a strain that is most easily neutralized, and Tier2 refers to a strain that is least easily neutralized.

As used herein, the term "identity" refers to the match degree between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The percent identity between two sequences is a function of the number of identical sites shared by the two sequences over the total number of sites for comparison x 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison of two sequences is conducted in a manner to produce maximum identity. Such alignment can be conducted by using a computer program such as Align program (DNAstar, Inc.) which is based on the method of Needleman, et al. (J. Mol. Biol. 48:443-453, 1970). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

The writing of the twenty conventional amino acids referred to herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which are well known in the art (see for example. Remington's Pharmaceutical Sciences. Edited by Gennaro AR, 19th ed. Pennsylvania: Mack Publishing Company, 1995), and include, but are not limited to: pH adjuster, surfactant, ionic strength enhancer, agent for maintaining osmotic pressure, agent for delaying absorption, diluent, adjuvant, preservative, stabilizer, etc. For example, the pH adjusting agent includes, but is not limited to, phosphate buffer. The surfactant includes but is not limited to cationic, anionic or nonionic surfactant such as Tween-80. the ionic strength enhancer includes, but is not limited to, sodium chloride. The agent for maintaining osmotic pressure includes, but is not limited to, sugar, NaCl, and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The adjuvant includes, but is not limited to, aluminum adjuvant (e.g., aluminum hydroxide), Freund's adjuvant (e.g., complete Freund's adjuvant), and the like. The preservative includes, but is not limited to, various antibacterial and antifungal agents, such as thimerosal, 2-phenoxyethanol, paraben, chlorobutanol, phenol, sorbic acid, and the like. The stabilizer has the meaning generally understood by those skilled in the art, which can stabilize a desired activity of the active ingredient in the drug (e.g., the inhibitory activity on PSD-95 ubiquitination), including but not limited to sodium glutamate, gelatin, SPGA, saccharide (e.g., sorbitol, mannitol, starch, sucrose, lactose, dextran, or glucose), amino acid (e.g., glutamic acid, glycine), protein (e.g., dried whey, albumin, or casein) or degradation product thereof (e.g., lactalbumin hydrolyzate), etc.

### Beneficial effects of the present invention

Env, as the main antigen protein of HIV-1 virus surface recognition receptor molecule, is a key molecule in the development of HIV-1 vaccine. Env on the surface of natural viruses is a non-covalently linked heterotrimer. When the virus binds to the CD4 molecule on the host cell, the conformation of Env gradually changes from a closed pre-fusion conformation to an opened conformation. Studies have shown that Env has more bNab epitopes exposed in the pre-fusion conformation. Based on the instability of the natural Env trimer, it is particularly important to perform HIV-1 Env immunogen design to stabilize its conformation.

The Env trimer protein of the present invention can be efficiently expressed in mammalian cells, with increased trimer content, improved uniformity, and significantly improved thermal stability. The Env trimeric protein of the present invention can effectively expose key epitopes such as CD4bs/V3, and is not conducive to the exposure of non-neutralizing epitopes, thus has the potential as a protein vaccine. In addition, the HIV pseudovirus and virus obtained based on the Env trimer protein are highly similar to the natural virus in shape, so they have the immunogenicity of the natural virus to the body, but they have lost the ability to infect and cause disease, and thus show the potential as virus-based vaccines, thereby having important clinical value.

Embodiments of the present invention will be described in detail below with reference to the drawings and examples, but those skilled in the art will understand that the following drawings and examples are only for illustrating the present invention, rather than limiting the scope of the present invention. Various objects and advantages of the present invention will become apparent to those skilled in the art from the accompanying drawings and the following detailed description of the preferred embodiment.

### Brief Description of the Drawings

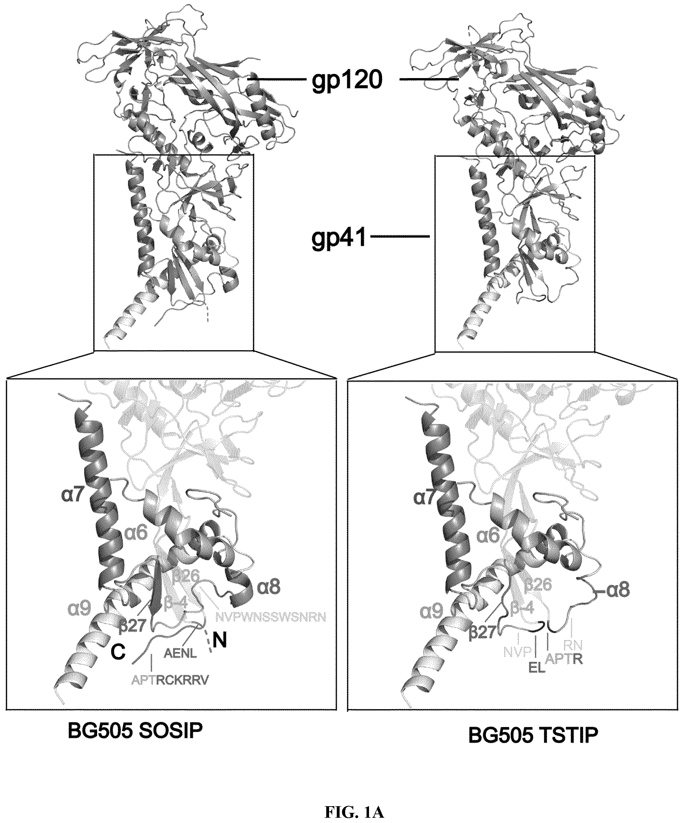

FIGS. 1A to 1B show the hypothetical spatial structure (1A) and secondary structure topology (1B) of monomeric protein of the gp140 trimer before modification (BG505 SOSIP, PDB No. 4TVP) and the trimer after modification (BG505 TSTIP).

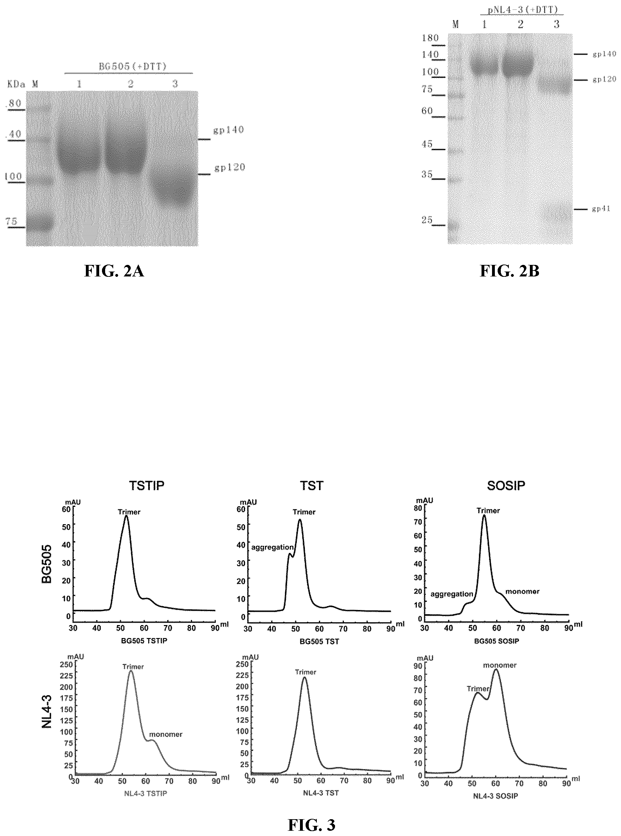

FIGS. 2A to 2B show the results of reducing SDS polyacrylamide gel electrophoresis of the modified gp140 trimer after one-step Ni Sepharose excel purification. FIG. 2A shows the BG505 strain, and FIG. 2B shows the NL4-3 strain. 1 represents TSTIP protein, 2 represents TST protein, 3 represents SOSIP protein. Both TSTIP and TST showed a single band under reducing conditions, with a molecular weight of about 140KD, while SOSIP showed two bands under reducing conditions, which were gp120 and gp41 extracellular segment. This shows that the protein of the present invention contains a stable covalent linkage between subunits as expected.
FIG. 3 shows the molecular sieve superdex200-16/600 purification results of the modified gp140 trimer, in which A represents BG505 strain, B represents NL4-3 strain. The molecular sieve chromatography results of TSTIP protein and TST protein showed a single elution peak, while the chromatographic result of SOSIP protein showed three elution peaks, indicating that the components of the proteins TSTIP and TST of the present invention were more uniform than those of SOSIP.

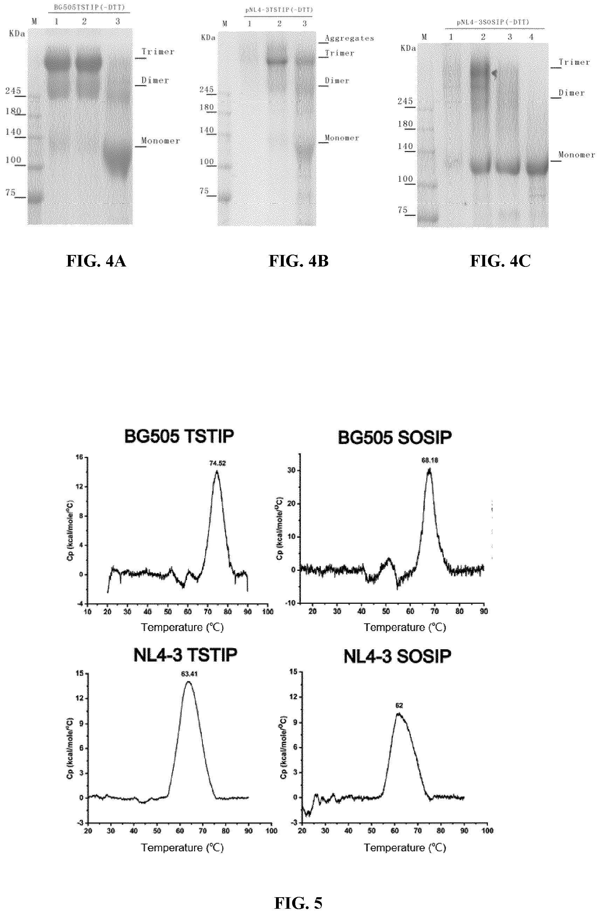

FIGS. 4A to 4C show the results of non-reducing polyacrylamide gel electrophoresis of the modified gp140 trimer after molecular sieve purification. A represents BG505 TSTIP, B represents NL4-3 TSTIP, C represents BG505 SOISP. The results showed that after molecular sieve purification, the main component of the protein TSTIP of the present invention was trimer, and had a trimer content higher than that of SOSIP.
FIG. 5 shows the Tm measurement results of the modified gp140 trimer by differential scanning calorimetry (DSC). The results showed that the Tm of the protein BG505 TSTIP of the present invention was 74.5°C, and the Tm of NL4-3 TSTIP was 63.41°C, which was higher than the Tm of the control protein BG505 SOSIP: 68.2°C and the Tm of the control protein NL4-3 SOSIP: 62°C. This shows that the thermal stability of the modified protein is higher than that of the corresponding control protein SOSIP.

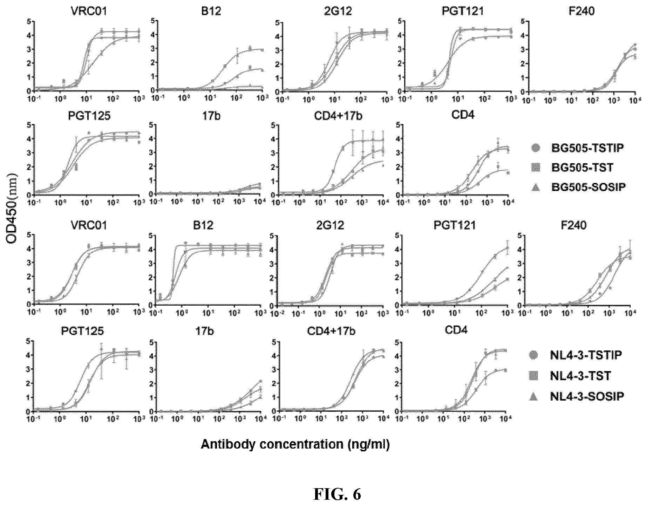

FIG. 6 shows the enzyme-linked immunosorbent assay (ELISA) results of the modified gp140 trimer with various reported human monoclonal antibodies. The antibodies used include broad-spectrum neutralizing antibodies VRC01, B12, PGT121, PGT125, 2G12, non-broad-spectrum neutralizing antibodies F105, F240, 17b, and HIV-1 receptor molecule CD4. The results showed that the proteins BGTSTIP and NL4-3 TSTIP of the present invention had a binding ability to various reported broad-spectrum neutralizing antibodies and non-broad-spectrum neutralizing antibodies that was basically equivalent to that of the control proteins BG505 SOSIP and NL4-3 SOSIP, and could recognize and bind well to CD4 receptor molecule. In addition, like SOSIP, the recognition of the modified protein by CD4 would induce the conformational change of the protein of the present invention, thereby exposing the 17b binding site.

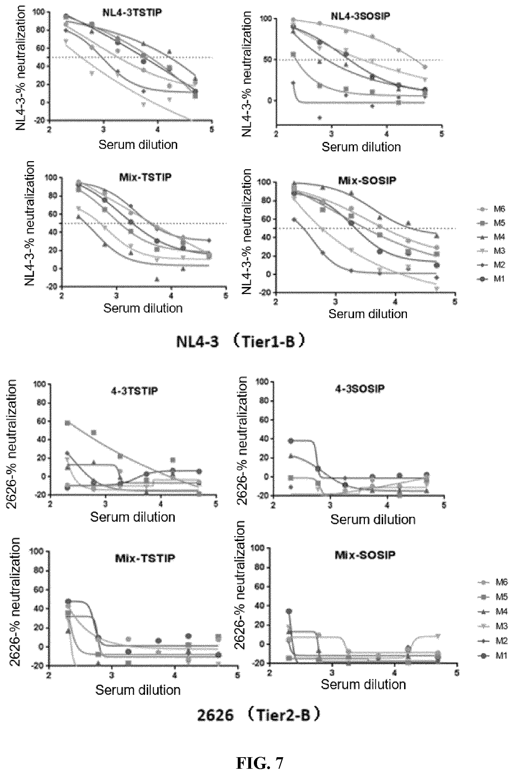

FIG. 7 shows the results of the virus neutralization experiment of the immune serum samples obtained by immunizing BALB/C mice with the modified gp140 trimer on the B subtype NL4-3 and B subtype 2626 strain viruses; wherein, M1 to M6 referred to different mice, respectively. The results showed that the modified protein TSTIP and the control protein could induce a strong neutralizing response against the corresponding strain after immunizing mice, but could not induce neutralizing response against the Tier2 strain of the same subtype. The modified protein TSTIP and the control protein SOSIP showed comparable immunogenicity.

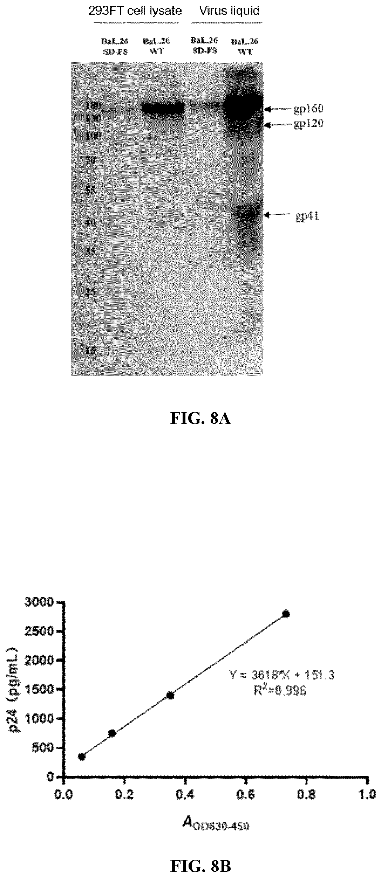

FIG. 8A shows the results of western blot of the pseudovirus based on the modified gp140 trimer. FIG. 8B shows the quantitative results of p24 of the pseudovirus based on the modified gp140 trimer. The results showed that both the transfected cells and the collected virus supernatant had the expression of full-length TSTIP protein, and the content of p24 in the supernatant of the pseudovirus of the present invention was decreased in some extent compared with the wild-type pseudovirus, but certain amounts of p24 and Env could still be detected, indicating that the pseudovirus of the present invention could be normally packaged to form a complete pseudoviral particle, but the yield might be reduced to a certain extent.

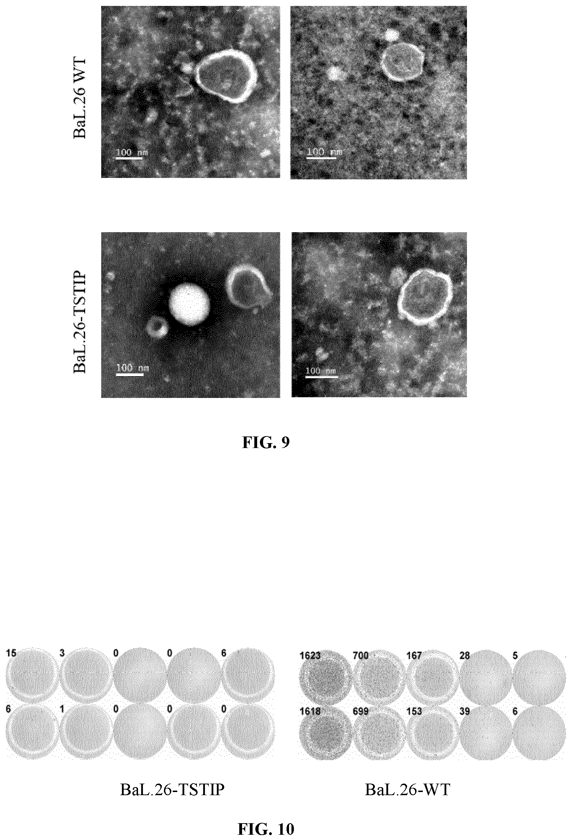

FIG. 9 shows the results of negative staining electron microscopy of the pseudoviral particles based on the modified gp140 trimer. It showed that the pseudoviral particles similar to the wild-type pseudovirus could be seen under the negative staining electron microscope.
FIG. 10 shows the infectivity detection results of the pseudovirus based on the modified gp140 trimer by using the Elispot method with LacZ as reporter gene. It showed that the wild-type pseudovirus maintained normal infectivity, while the pseudovirus of the present invention had lost the infectivity, which was in line with the inventor's expectation.

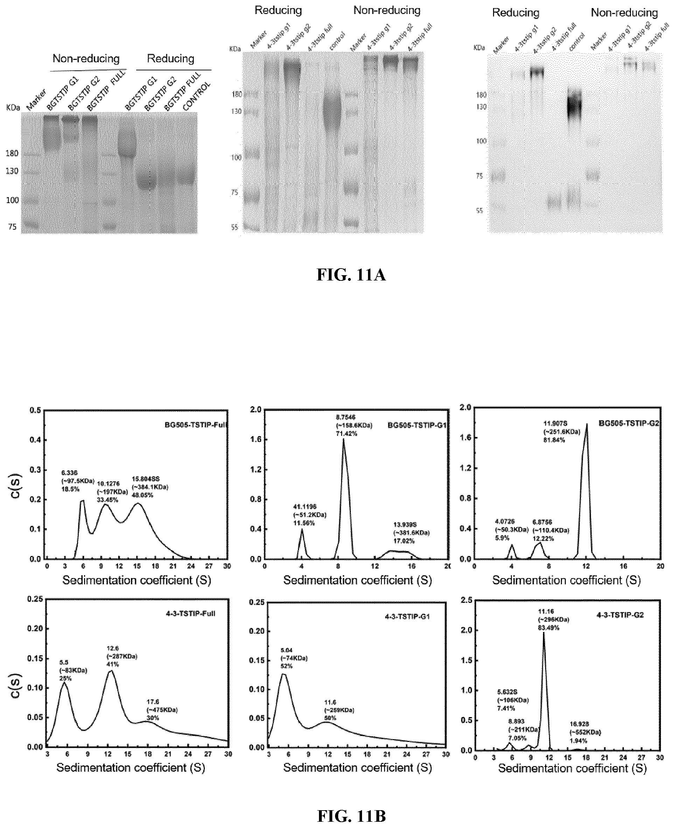

FIG. 11A shows the results of reducing SDS polyacrylamide gel electrophoresis of the modified gp140 trimers BG505/NL4-3 TSTIP G1/G2/Full. It showed that the molecular weights of BG505 TSTIP-G2 and NL4-3 TSTIP-G1 were larger than expected, the two proteins could still be dimers under reducing conditions, while no target proteins with high purity were available for BGTSTIP-Full, NL4-3-G1 and NL4-3-Full after purification. In contrast, the protein TSTIP of the present invention had a broader range of application.
FIG. 11B shows the AUC results of the modified gp140 trimers BG505/NL4-3 TSTIP G1/G2/Full, which were consistent with the results of SDS-PAGE.

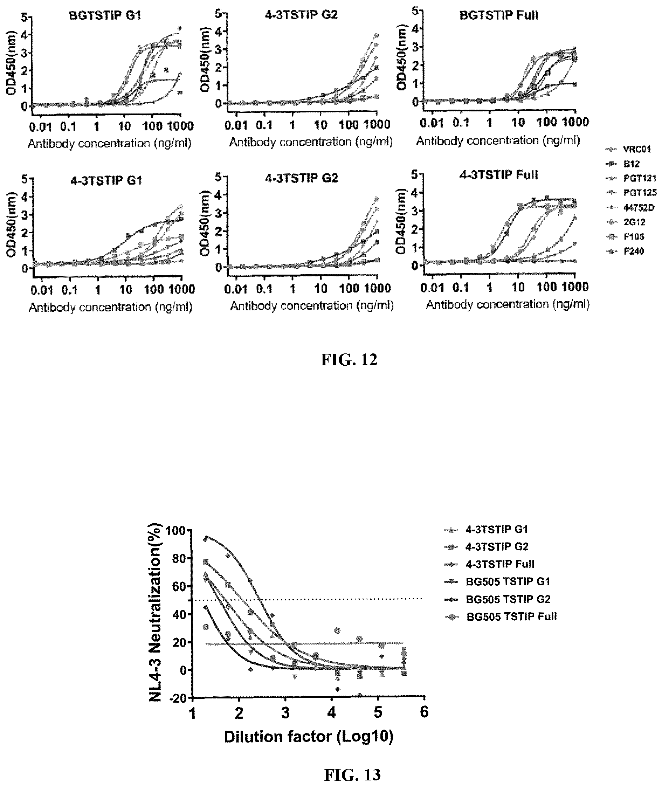

FIG. 12 shows the results of enzyme-linked immunosorbent assay of the modified gp140 trimers BG505/NL4-3 TSTIP G1/G2/Full with various reported antibodies. It showed that the six proteins could all react with various antibodies and had certain activities, but the reactivity of some proteins was weaker than that of the protein TSTIP of the present invention.
FIG. 13 shows the virus neutralization experiment of the immune serum samples obtained by immunizing BALB/C mice with various modified gp140 trimers on B subtype NL4-3 strain. The results showed that all six proteins could induce neutralizing antibody responses in mice.

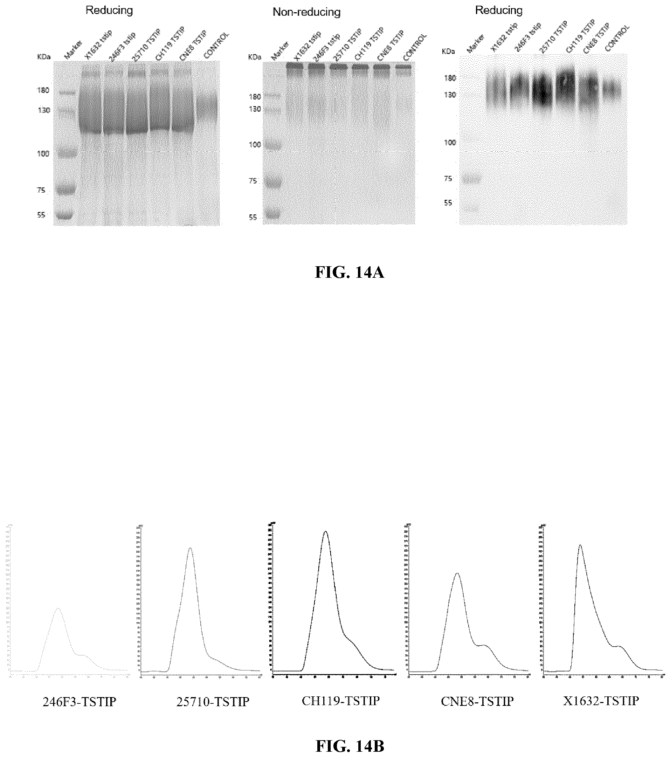

FIG. 14A shows the SDS polyacrylamide gel electrophoresis results of the modified gp140 trimers of various strains. The results showed that the modified proteins of the various strains were well expressed, and could be purified to reach a higher purity. Under the reducing SDS-PAGE, the proteins of the present invention showed a single band, which proved that the subunits thereof were linked by a stable covalent linkage.
FIG. 14B shows the molecular sieve superdex200 16/600 purification results of TSTIP protein of the five strains, showing that the molecular sieve peaks of the five proteins of the present invention were relatively single, indicating better protein uniformity, and the proteins obtained by applying the strategy of the present invention to different strains formed trimers as main component.

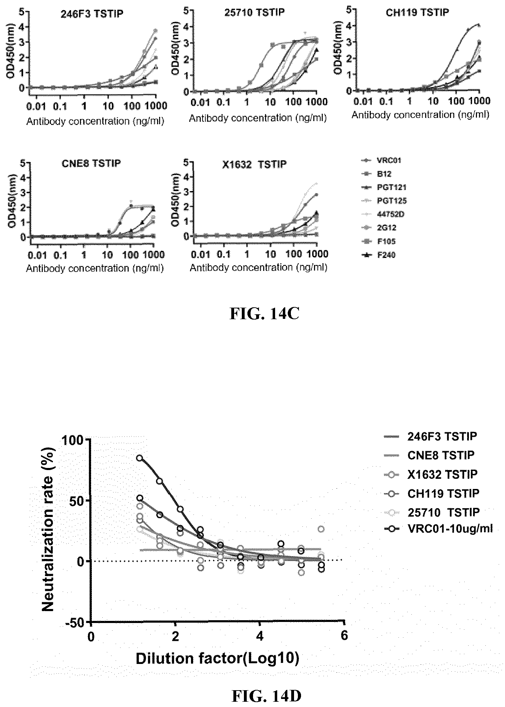

FIG. 14C shows the results of enzyme-linked immunosorbent assay of the proteins of the five strains with various antibodies, showing that the five proteins of the present invention had certain reactivity with various reported antibodies, proving their good activities.
FIG. 14D shows the neutralization test results of pseudovirus by the serum samples obtained by immunization with TSTIP proteins of the five strains, wherein the strains detected by immune serum samples of CH119 TSTIP and X1632 TSTIP were the corresponding CH119 and X1632 strains respectively; the strain detected by immune serum samples of 25710 and 246F3 TSTIP was BJOX200; the strain detected by immune serum sample of CNE8 TSTIP was TRO11. The results showed that the immune serum samples of the fourth injection of TSTIP protein of the five strains all detected virus neutralization to some extent, indicating that the immunization of TSTIP protein of different strains could stimulate neutralization responses in mice.

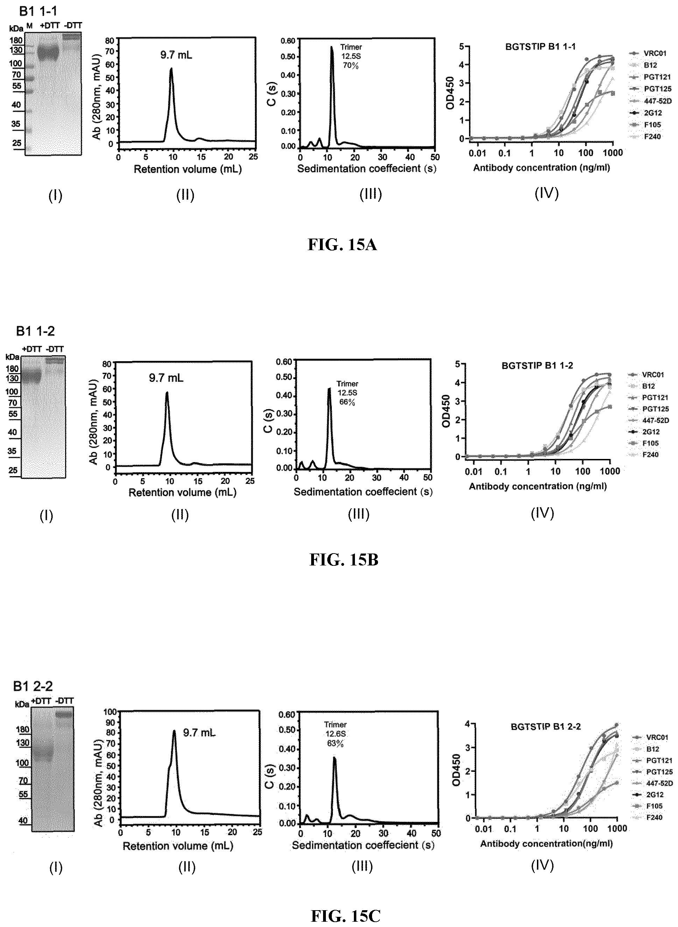

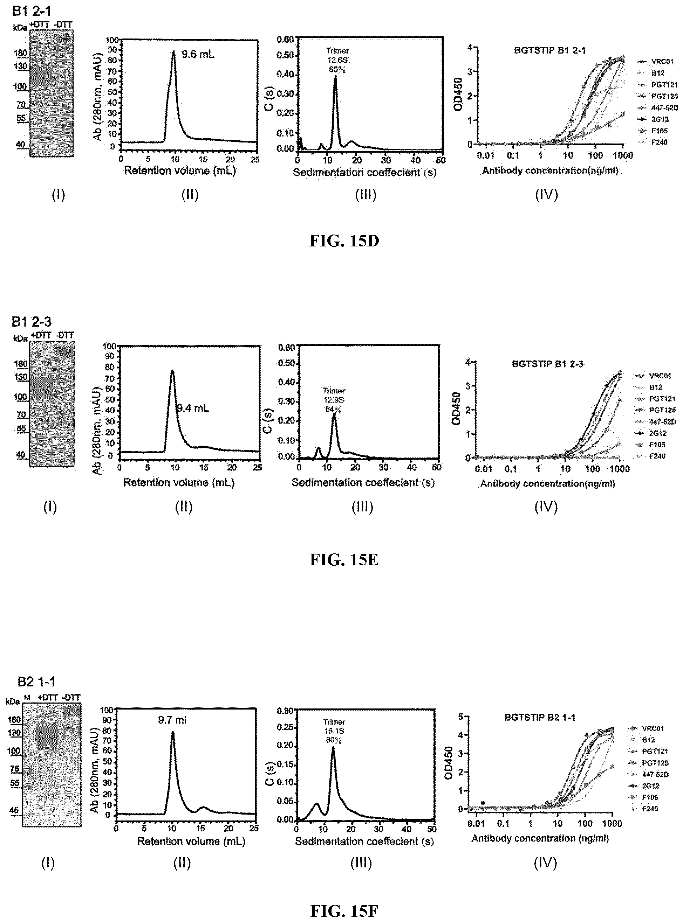

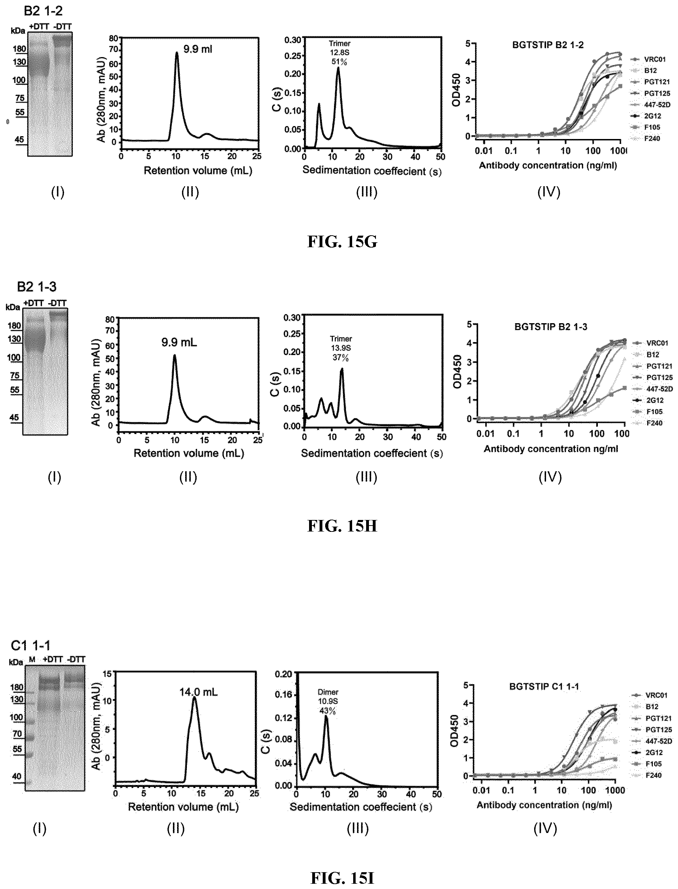

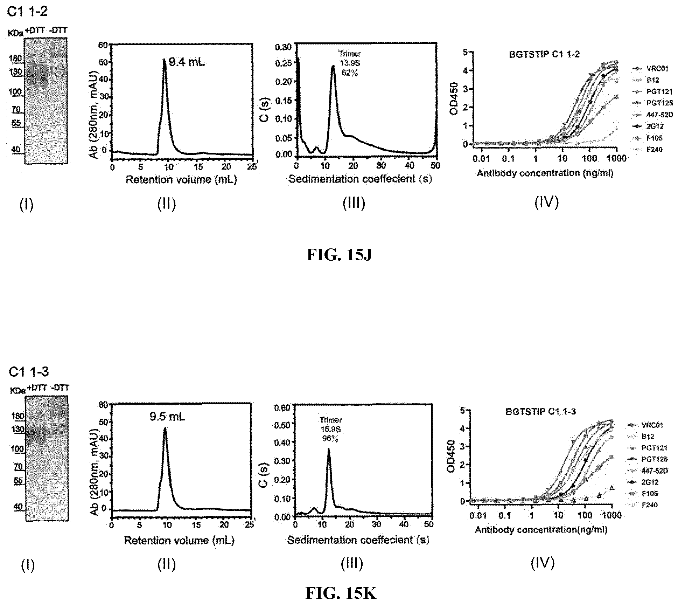

FIGS. 15A to 15K show the experimental results of electrophoresis (I), molecular sieve purification (II), analytical ultracentrifugation (III), and ELISA (IV) of each TSTIP protein construct in Example 11. FIG. 15A: BG-B1 (1/1); FIG. 15B: BG-B1 (1/2); FIG. 15C: BG-B 1 (2/2); FIG. 15D: BG-B1 (2/1); FIG. 15E: BG-B1(2/3); FIG. 15F: BG-B2(1-1); FIG. 15G: BG-B2(1-2); FIG. 15H: BG-B2(1-3); FIG. 15I: BG-C1 (1/1); FIG. 15J: BG-C1 (1/2); FIG. 15K: BG-C1 (1/3).

### Sequence information

Information on some sequences involved in the present invention is provided in Table 1 below.

**Table 1: Description of sequences**

| SEQ ID NO | Description | Sequence information |
|---|---|---|
| 1 | BG505 TST | MDAMKRGLCCVLLLCGAVFVSPSQEIHARFRRGARAENLAVGIGA VFLGFLGAAGSTMGAASMTLTVQARNLLSGIVQQQSNLLRAIEAQQ HLLKLTVWGIKQLQARVLAVERYLRDQQLLGIWGCSGKLICTTNVP NLWVTVYYGVPVWKDAETTLFCASDAKAYETEKHNVWATHACV PTDPNPQEIHLENVTEEFNMWKNNMVEQMHTDIISLWDQSLKPCV KLTPLCVTLQCTNVTNNITDDMRGELKNCSFNMTTELRDKKQKVY SLFYRLDVVQINENQGNRSNNSNKEYRLINCNTSAITQACPKVSFEPI PIHYCAPAGFAILKCKDKKFNGTGPCPSVSTVQCTHGIKPVVSTQLL LNGSLAEEEVMIRSENITNNAKNILVQFNTPVQINCTRPNNNTRKSIR IGPGQAFYATGDIIGDIRQAHCNVSKATWNETLGKVVKQLRKHFGN NTIIRFANSSGGDLEVTTHSFNCGGEFFYCNTSGLFNSTWISNTSVQG SNSTGSNDSITLPCRIKQIINMWQRIGQAMYAPPIQGVIRCVSNITGLI LTRDGGSTNSTTETFRPGGGDMRDNWRSELYKYKVVKIEPLGVAPT RRNLSEIWDNMTWLQWDKEISNYTQIIYGLLEESQNQQEKNEQDLL ALDHHHHHHHH* |
| 2 | NL4-3 TST | MDAMKRGLCCVLLLCGAVFVSPSQEIHARFRRGARAENLAVGIGA LFLGFLGAAGSTMGAASMTLTVQARQLLSDIVQQQNNLLRAIEAQQ HLLQLTVWGIKQLQARILAVERYLKDQQLLGIWGCSGKLICTTAVP NLWVTVYYGVPVWKEATTTLFCASDAKAYDTEVHNVWATHACV PTDPNPQEVVLVNVTENFNMWKNDMVEQMHEDIISLWDQSLKPCV KLTPLCVSLKCTDLKNDTNTNSSSGRMIMEKGEIKNCSFNISTSIRDK VQKEYAFFYKLDIVPIDNTSYRLISCNTSVITQACPKVSFEPIPIHYCA PAGFAILKCNNKTFNGTGPCTNVSTVQCTHGIRPVVSTQLLLNGSLA EEDVVIRSANFTDNAKTIIVQLNTSVEINCTRPNNNTRKSIRIQRGPG RAFVTIGKIGNMRQAHCNISRAKWNATLKQIASKLREQFGNNKTIIF KQSSGGDPEIVTHSFNCGGEFFYCNSTQLFNSTWFNSTWSTEGSNNT EGSDTITLPCRIKQFINMWQEVGKAMYAPPISGQIRCSSNITGLLLTR DGGNNNNGSEIFRPGGGDMRDNWRSELYKYKVVKIEPLGVAPTKK SLEQIWNNMTWMEWDREINNYTSLIHSLIEESQNQQEKNEQELLEL DKHHHHHHHH* |
| 3 | BG505 TSTIP | MDAMKRGLCCVLLLCGAVFVSPSQEIHARFRRGARAENLAVGIGA VFLGFLGAAGSTMGAASMTLTVQARNLLSGIVQQQSNLLRAPEAQ QHLLKLTVWGIKQLQARVLAVERYLRDQQLLGIWGCSGKLICTTN VPNLWVTVYYGVPVWKDAETTLFCASDAKAYETEKHNVWATHA CVPTDPNPQEIHLENVTEEFNMWKNNMVEQMHTDIISLWDQSLKPC VKLTPLCVTLQCTNVTNNITDDMRGELKNCSFNMTTELRDKKQKV YSLFYRLDVVQINENQGNRSNNSNKEYRLINCNTSAITQACPKVSFE PIPIHYCAPAGFAILKCKDKKFNGTGPCPSVSTVQCTHGIKPVVSTQL LLNGSLAEEEVMIRSENITNNAKNILVQFNTPVQINCTRPNNNTRKSI RIGPGQAFYATGDIIGDIRQAHCNVSKATWNETLGKVVKQLRKHFG |
| | | NNTIIRFANSSGGDLEVTTHSFNCGGEFFYCNTSGLFNSTWISNTSVQ GSNSTGSNDSITLPCRIKQIINMWQRIGQAMYAPPIQGVIRCVSNITG LILTRDGGSTNSTTETFRPGGGDMRDNWRSELYKYKVVKIEPLGVA PTRRNLSEIWDNMTWLQWDKEISNYTQIIYGLLEESQNQQEKNEQD LLALDHHHHHHHH* |
| 4 | NL4-3 TSTIP | MDAMKRGLCCVLLLCGAVFVSPSQEIHARFRRGARAENLAVGIGA LFLGFLGAAGSTMGAASMTLTVQARQLLSDIVQQQNNLLRAPEAQ QHLLQLTVWGIKQLQARILAVERYLKDQQLLGIWGCSGKLICTTAV PNLWVTVYYGVPVWKEATTTLFCASDAKAYDTEVHNVWATHAC VPTDPNPQEVVLVNVTENFNMWKNDMVEQMHEDIISLWDQSLKPC VKLTPLCVSLKCTDLKNDTNTNSSSGRMIMEKGEIKNCSFNISTSIRD KVQKEYAFFYKLDIVPIDNTSYRLISCNTSVITQACPKVSFEPIPIHYC APAGFAILKCNNKTFNGTGPCTNVSTVQCTHGIRPVVSTQLLLNGSL AEEDVVIRSANFTDNAKTIIVQLNTSVEINCTRPNNNTRKSIRIQRGP GRAFVTIGKIGNMRQAHCNISRAKWNATLKQIASKLREQFGNNKTII FKQSSGGDPEIVTHSFNCGGEFFYCNSTQLFNSTWFNSTWSTEGSNN TEGSDTITLPCRIKQFINMWQEVGKAMYAPPISGQIRCSSNITGLLLT RDGGNNNNGSEIFRPGGGDMRDNWRSELYKYKVVKIEPLGVAPTK KSLEQIWNNMTWMEWDREINNYTSLIHSLIEESQNQQEKNEQELLE LDKHHHHHHHH* |
| 5 | BG505 TSTIP full | MDAMKRGLCCVLLLCGAVFVSPSQEIHARFRRGARAENLAVGIGA VFLGFLGAAGSTMGAASMTLTVQARNLLSGIVQQQSNLLRAPEAQ QHLLKLTVWGIKQLQARVLAVERYLRDQQLLGIWGCSGKLICTTN VPWNSSWSNNLWVTVYYGVPVWKDAETTLFCASDAKAYETEKHN VWATHACVPTDPNPQEIHLENVTEEFNMWKNNMVEQMHTDIISLW DQSLKPCVKLTPLCVTLQCTNVTNNITDDMRGELKNCSFNMTTELR DKKQKVYSLFYRLDVVQINENQGNRSNNSNKEYRLINCNTSAITQA CPKVSFEPIPIHYCAPAGFAILKCKDKKFNGTGPCPSVSTVQCTHGIK PVVSTQLLLNGSLAEEEVMIRSENITNNAKNILVQFNTPVQINCTRPN NNTRKSIRIGPGQAFYATGDIIGDIRQAHCNVSKATWNETLGKVVK QLRKHFGNNTIIRFANSSGGDLEVTTHSFNCGGEFFYCNTSGLFNST WISNTSVQGSNSTGSNDSITLPCRIKQIINMWQRIGQAMYAPPIQGVI RCVSNITGLILTRDGGSTNSTTETFRPGGGDMRDNWRSELYKYKVV KIEPLGVAPTRAKRRVVGRNLSEIWDNMTWLQWDKEISNYTQIIYG LLEESQNQQEKNEQDLLALDHHHHHHHH* |
| 6 | NL4-3 TSTIP full | MDAMKRGLCCVLLLCGAVFVSPSQEIHARFRRGARAENLREKRAV GIGALFLGFLGAAGSTMGAASMTLTVQARQLLSDIVQQQNNLLRAP EAQQHLLQLTVWGIKQLQARILAVERYLKDQQLLGIWGCSGKLICT TAVPWNASWSNNLWVTVYYGVPVWKEATTTLFCASDAKAYDTEV HNVWATHACVPTDPNPQEVVLVNVTENFNMWKNDMVEQMHEDII SLWDQSLKPCVKLTPLCVSLKCTDLKNDTNTNSSSGRMIMEKGEIK NCSFNISTSIRDKVQKEYAFFYKLDIVPIDNTSYRLISCNTSVITQACP KVSFEPIPIHYCAPAGFAILKCNNKTFNGTGPCTNVSTVQCTHGIRPV VSTQLLLNGSLAEEDVVIRSANFTDNAKTIIVQLNTSVEINCTRPNNN TRKSIRIQRGPGRAFVTIGKIGNMRQAHCNISRAKWNATLKQIASKL REQFGNNKTIIFKQSSGGDPEIVTHSFNCGGEFFYCNSTQLFNSTWFN STWSTEGSNNTEGSDTITLPCRIKQFINMWQEVGKAMYAPPISGQIR CSSNITGLLLTRDGGNNNNGSEIFRPGGGDMRDNWRSELYKYKVV KIEPLGVAPTKAKRRVVQKSLEQIWNNMTWMEWDREINNYTSLIH SLIEESQNQQEKNEQELLELDKGSGSGGSGHHHHHHHH* |
| 7 | BG505 TSTIP G1 | MDAMKRGLCCVLLLCGAVFVSPSQEIHARFRRGARAENLAVGIGA VFLGFLGAAGSTMGAASMTLTVQARNLLSGIVQQQSNLLRAPEAQ QHLLKLTVWGIKQLQARVLAVERYLRDQQLLGIWGCSGKLICTTN VPGGGGSNLWVTVYYGVPVWKDAETTLFCASDAKAYETEKHNV WATHACVPTDPNPQEIHLENVTEEFNMWKNNMVEQMHTDIISLWD QSLKPCVKLTPLCVTLQCTNVTNNITDDMRGELKNCSFNMTTELRD KKQKVYSLFYRLDVVQINENQGNRSNNSNKEYRLINCNTSAITQAC |
| | | PKVSFEPIPIHYCAPAGFAILKCKDKKFNGTGPCPSVSTVQCTHGIKP VVSTQLLLNGSLAEEEVMIRSENITNNAKNILVQFNTPVQINCTRPN NNTRKSIRIGPGQAFYATGDIIGDIRQAHCNVSKATWNETLGKVVK QLRKHFGNNTIIRFANSSGGDLEVTTHSFNCGGEFFYCNTSGLFNST WISNTSVQGSNSTGSNDSITLPCRIKQIINMWQRIGQAMYAPPIQGVI RCVSNITGLILTRDGGSTNSTTETFRPGGGDMRDNWRSELYKYKVV KIEPLGVAPTRGGGGSRNLSEIWDNMTWLQWDKEISNYTQIIYGLL EESQNQQEKNEQDLLALDHHHHHHHH* |
| 8 | NL4-3 TSTIP G1 | MDAMKRGLCCVLLLCGAVFVSPSQEIHARFRRGARAENLREKRAV GIGALFLGFLGAAGSTMGAASMTLTVQARQLLSDIVQQQNNLLRAP EAQQHLLQLTVWGIKQLQARILAVERYLKDQQLLGIWGCSGKLICT TAVPGGGGSNLWVTVYYGVPVWKEATTTLFCASDAKAYDTEVHN VWATHACVPTDPNPQEVVLVNVTENFNMWKNDMVEQMHEDIISL WDQSLKPCVKLTPLCVSLKCTDLKNDTNTNSSSGRMIMEKGEIKNC SFNISTSIRDKVQKEYAFFYKLDIVPIDNTSYRLISCNTSVITQACPKV SFEPIPIHYCAPAGFAILKCNNKTFNGTGPCTNVSTVQCTHGIRPVVS TQLLLNGSLAEEDVVIRSANFTDNAKTIIVQLNTSVEINCTRPNNNTR KSIRIQRGPGRAFVTIGKIGNMRQAHCNISRAKWNATLKQIASKLRE QFGNNKTIIFKQSSGGDPEIVTHSFNCGGEFFYCNSTQLFNSTWFNST WSTEGSNNTEGSDTITLPCRIKQFINMWQEVGKAMYAPPISGQIRCS SNITGLLLTRDGGNNNNGSEIFRPGGGDMRDNWRSELYKYKVVKIE PLGVAPTKGGGGSKSLEQIWNNMTWMEWDREINNYTSLIHSLIEES QNQQEKNEQELLELDKGSGSGGSGHHHHHHHH* |
| 9 | BG505 TSTIP G2 | MDAMKRGLCCVLLLCGAVFVSPSQEIHARFRRGARAENLAVGIGA VFLGFLGAAGSTMGAASMTLTVQARNLLSGIVQQQSNLLRAPEAQ QHLLKLTVWGIKQLQARVLAVERYLRDQQLLGIWGCSGKLICTTN VPGGGGSGGGGSNLWVTVYYGVPVWKDAETTLFCASDAKAYETE KHNVWATHACVPTDPNPQEIHLENVTEEFNMWKNNMVEQMHTDII SLWDQSLKPCVKLTPLCVTLQCTNVTNNITDDMRGELKNCSFNMT TELRDKKQKVYSLFYRLDVVQINENQGNRSNNSNKEYRLINCNTSA ITQACPKVSFEPIPIHYCAPAGFAILKCKDKKFNGTGPCPSVSTVQCT HGIKPVVSTQLLLNGSLAEEEVMIRSENITNNAKNILVQFNTPVQINC TRPNNNTRKSIRIGPGQAFYATGDIIGDIRQAHCNVSKATWNETLGK VVKQLRKHFGNNTIIRFANSSGGDLEVTTHSFNCGGEFFYCNTSGLF NSTWISNTSVQGSNSTGSNDSITLPCRIKQIINMWQRIGQAMYAPPIQ GVIRCVSNITGLILTRDGGSTNSTTETFRPGGGDMRDNWRSELYKY KVVKIEPLGVAPTRGGGGSGGGGSRNLSEIWDNMTWLQWDKEISN YTQIIYGLLEESQNQQEKNEQDLLALDHHHHHHHH* |
| 10 | NL4-3 TSTIP G2 | MDAMKRGLCCVLLLCGAVFVSPSQEIHARFRRGARAENLREKRAV GIGALFLGFLGAAGSTMGAASMTLTVQARQLLSDIVQQQNNLLRAP EAQQHLLQLTVWGIKQLQARILAVERYLKDQQLLGIWGCSGKLICT TAVPGGGGSGGGGSNLWVTVYYGVPVWKEATTTLFCASDAKAYD TEVHNVWATHACVPTDPNPQEVVLVNVTENFNMWKNDMVEQMH EDIISLWDQSLKPCVKLTPLCVSLKCTDLKNDTNTNSSSGRMIMEKG EIKNCSFNISTSIRDKVQKEYAFFYKLDIVPIDNTSYRLISCNTSVITQ ACPKVSFEPIPIHYCAPAGFAILKCNNKTFNGTGPCTNVSTVQCTHGI RPVVSTQLLLNGSLAEEDVVIRSANFTDNAKTIIVQLNTSVEINCTRP NNNTRKSIRIQRGPGRAFVTIGKIGNMRQAHCNISRAKWNATLKQIA SKLREQFGNNKTIIFKQSSGGDPEIVTHSFNCGGEFFYCNSTQLFNST WFNSTWSTEGSNNTEGSDTITLPCRIKQFINMWQEVGKAMYAPPIS GQIRCSSNITGLLLTRDGGNNNNGSEIFRPGGGDMRDNWRSELYKY KVVKIEPLGVAPTKGGGGSGGGGSKSLEQIWNNMTWMEWDREIN NYTSLIHSLIEESQNQQEKNEQELLELDKGSGSGGSGHHHHHHHH* |
| 11 | 25710-TSTIP | AVGIGAVFLGFLGAAGSTMGAASITLTVQARQLLSGIVQQQSNLLR APEAQQHLLQLTVWGIKQLQTRVLAIERYLKDQQLLGIWGCSGKLI CTTAVPNLWVTVYYGVPVWKEATTTLFCASDAKAYDKEVHNVW ATHACVPTDPNPQEMVLGNVTENFNMWKNEMVNQMHEDVISLW |
| | | DQSLKPCVKLTPLCVTLECSNVTYNESMKEVKNCSFNLTTELRDKK QKVHALFYRLDIVPLNDTEKKNSSRPYRLINCNTSAITQACPKVTFD PIPIHYCTPAGYAILKCNDKKFNGTGPCHKVSTVQCTHGIKPVVSTQ LLLNGSLAEGEIIIRSENLTNNAKTIIVHLNQSVEIVCARPSNNTRTSI RIGPGQTFYATGAITGDIRQAHCNISKDKWNETLQRVGEKLAEHFP NKTIKFNSSSGGDLEITTHSFNCRGEFFYCNTSGLFNGTFNGTYVSPN STDSNSSSIITIPCRIKQIINMWQEVGRAMYAPPIAGNITCKSNITGLL LVRDGGTGSESNKTEIFRPGGGDMRDNWRSELYKYKVVEIKPLGV APTKRSQDDIWDNMTWMQWDKEISNYTNTIYKLLEDSQIQQEKNE KDLLALDSHHHHHHHH |
| 12 | X1632-TSTIP | MDAMKRGLCCVLLLCGAVFVSPSQEIHARFRRGARAIGLGTVLLGF LGTAGSTMGAASITLTVQVRQLLSGIVQQQSNLLRAPEAQQHLLQLT VWGIKQLQARVLAVERYLKDQQILGIWGCSGKLICTTNVPNLWVTV YYGVPVWEDADTTLFCASDAKAYSTESHNVWATHACVPTDPNPQEI YLENVTEDFNMWENNMVEQMQEDIISLWDESLKPCVKLTPLCVTLT CTNVTNVTDSVGTNSRLKGYKEELKNCSFNTTTEIRDKKKQEYALF YKLDIVPINDNSNNSNGYRLINCNVSTIKQACPKVSFDPIPIHYCAPA GFAILKCRDKEFNGTGTCRNVSTVQCTHGIKPVVSTQLLLNGSLAEG DIVIRSENITDNAKTIIVHLNKTVSITCTRPNNNTRKSIRIGPGQALYAT GAIIGDTRQAHCNINGSEWYEMIQNVKNKLNETFKKNITFNPSSGGD LEITTHSFNCRGEFFYCNTSELFNSSHLFNGSTLSTNGTITLPCRIKQI VRMWQRVGQAMYAPPIAGNITCRSNITGLLLTRDGGTNKDTNEAET FRPGGGDMRDNWRSELYKYKVVKIKPLGVAPTRKSYSDIWDNLTWI QWEREISNYTQQIYTLLEESQNQQEKNEQELLALDKHHHHHHHH |
| 13 | CH119-TSTIP(BC) | MDAMKRGLCCVLLLCGAVFVSPSQEIHARFRRGARAVGIGAVFLGF LGVAGSTMGAASMTLTVQARQLLSGIVQQQSNLLRAPEAQQHLLQ LTVWGIKQLQTRVLAIERYLKDQQLLGIWGCSGKLICTTAVPNLWV TVYYGVPVWKEATTTLFCASDAKAYDTEVHNVWATHACVPTDPS PQELVLENVTENFNMWKNEMVNQMHEDVISLWDQSLKPCVKLTP LCVTLECSKVSNNETDKYNGTEEMKNCSFNATTVVRDRQQKVYAL FYRLDIVPLTEKNSSENSSKYYRLINCNTSAITQACPKVSFEPIPIHYC TPAGYAILKCNDKTFNGTGPCHNVSTVQCTHGIKPVVSTQLLLNGS LAEGEIIIRSENLTNNVKTILVHLNQSVEIVCTRPNNNTRKSIRIGPGQ TFYATGDIIGDIRQAHCNISKWHETLKRVSEKLAEHFPNKTINFTSSS GGDLEITTHSFTCRGEFFYCNTSGLFNSTYMPNGTYLHGDTNSNSSI TIPCRIKQIINMWQEVGRAMYAPPIEGNITCKSNITGLLLVRDGGTES NNTETNNTEIFRPGGGDMRDNWRSELYKYKVVEIKPLGVAPTAKS QKEIWDNMTWMQWDKEISNYTNTIYKLLEDSQNQQESNEKDLLAL DHHHHHHHH |
| 14 | CNE8-TSTIP(AE) | MDAMKRGLCCVLLLCGAVFVSPSQEIHARFRRGARAVGIGAMIFGF LGAAGSTMGAASITLTVQARQLLSGIVQQQSNLLRAPEAQQHLLQL TVWGIKQLQARVLAVERYLKDQKFLGLWGCSGKIICTTAVPNLWV TVYYGVPVWRDADTTLFCASDAKAYDTEVHNVWATHACVPTDPN PQEIHLENVTENFNMWKNKMAEQMQEDVISLWDESLKPCVQLTPL CVTLNCTNANLNATVNASTTIGNITDEVRNCSFNTTTELRDKKQNV YALFYKLDIVPINNNSEYRLINCNTSVIKQACPKVSFDPIPIHYCAPA GYAILRCNDKNFNGTGPCKNVSSVQCTHGIKPVVSTQLLLNGSLAE DEIIIRSENLTDNVKTIIVHLNKSVEINCTRPSNNTRTSVRIGPGQVFY RTGDIIGDIRKAYCEINRTKWHETLKQVATKLREHFNKTIIFQPPSGG DIEITMHHFNCRGEFFYCNTTKLFNSTWGENTTMEGHNDTIVLPCRI KQIVNMWQGVGQAMYAPPIRGSINCVSNITGILLTRDGGTNMSNET FRPGGGNIKDNWRSELYKYKVVEIEPLGIAPTKRSYEEIWDNMTWI EWEREISNYTSQIYEILTESQNQQDRNEKDLLELDKHHHHHHHH |
| 15 | p246F3-TSTIP | MDAMKRGLCCVLLLCGAVFVSPSQEIHARFRRGARAVGIGAVFIGF LGAAGSTMGAASITLTVQARQLLSGIVQQQSNLLRAPEAQQHLLKL TVWGIKQLQARVLAVERYLKDQQLLGIWGCSGKLICTTNVDLWVT VYYGVPVWKDAKTTLFCASDAKAYEKEVHNVWATHACVPTDPNP |
| | | QEIVMANVTEEFNMWKNNMVEQMHTDIISLWDQSLKPCVKLTPLC VTLDCKDYNYSITNNSTGMEGEIKNCSYNITTELRDKRQKVYSLFY RLDVVQINDSNDRNNSQYRLINCNTTTMTQACPKVTFDPIPIHYCAP AGFAILKCNNKTFNGKGPCNNVSSVQCTHGIKPVVSTQLLLNGSLA EKEIVIRSENLTDNVKTIIVHLNESVEINCTRPNNNTRKSVRIGPGQTF YATGDIIGNIRQAHCNVNKTEWNTALTRVSKKLKEYFPNKTIAFQPS SGGDLEITTFSFNCRGEFFYCNTSDLFNGTFNETSGQFNSTFNSTLQC RIKQIINMWQEVGQAMYAPPIAGSITCISNITGLILTRDGGNTNSTKE TFRPGGGNMRDNWRSELYKYKVVKIEPLGVAPTKKSQDEIWDNMT WLQWDKEISNYTQIIYNLIEESQTQQELNERDLLALDHHHHHHHH |
| 16 | Bal.26-TSTIP-gp160 (AA:646-836 are transmembrane region and intracellular region) | MRVTEIRKSYQHWWRWGIMLLGMLMICNAEEKAVGIGAVLLGFL GAAGSTMGAASMTLTVQARLLLSGIVQQQNNLLRAIEAQQHLLQL TVWGIKQLQARVLAVERYLRDQQLLGPWGCSGKLICTTAVPNLWV TVYYGVPVWKEATTTLFCASDAKAYDTEVHNVWATHACVPTDPN PQEVKMENVTENFNMWKNNVVEQMHEDIISLWDQSLKPCVKLTPL CVTLNCTDLKNATNGNNTNTTSSSGGMMGGGEMKNCSFNITTNIR GKVQKEYALFYELDIVPIDNKIDSYRLISCNTSVITQACPKVSFEPIPI HYCAPAGFAILKCKDKKFNGKGPCSNVSTVQCTHGIRPVVSTQLLL NGSLAEEEVVIRSENFTNNAKIIVVQLNESVEINCTRPNNNTRKSIHI GPGRAFYTTGEIIGDIRQAHCNLSRAKWNDTLNKIVIKLREQFGNKT IVFKHSSGGDPEIVTHSFNCGGEFFYCNSTQLFNSTWNVTEESNNTV ENNTITLPCRIKQIINMWQEVGRAMYAPPIRGQIRCSSNITGLLLTRD GGPEDDKTEVFRPGGGDMRDNWRSELYKYKVVKIEPLGVAPTKKS LNKIWDNMTWMEWDREINNYTSIIYSLIEESQNQQEKNEQELLELD KWASLWNWFDITKWLWYIKIFIMIVGGLIGLRIVFSVLSIVNRVRQG YSPLSFQTHLPASRGPDRPGGIEEEGGERDRDRSGPLVNGFLTLIWV DLRSLFLFSYHRLRDLLLIVTRIVELLGRRGWEVLKYWWNLLQYWS QELKNSAVSLLNTIAIAVAEGTDRVIEVVQRAVRAILHIPRRIRQGLE RALL |
| 17 | NL4-3 TSTIP-gp160 (aa646-837 are transmembrane region and intracellular region) | MRVKEKYQHLWRWGWKWGTMLLGILMICSATEKAVGIGALFLGF LGAAGSTMGAASMTLTVQARQLLSDIVQQQNNLLRAPEAQQHLLQ LTVWGIKQLQARILAVERYLKDQQLLGIWGCSGKLICTTAVPNLWV TVYYGVPVWKEATTTLFCASDAKAYDTEVHNVWATHACVPTDPN PQEVVLVNVTENFNMWKNDMVEQMHEDIISLWDQSLKPCVKLTPL CVSLKCTDLKNDTNTNSSSGRMIMEKGEIKNCSFNISTSIRDKVQKE YAFFYKLDIVPIDNTSYRLISCNTSVITQACPKVSFEPIPIHYCAPAGF AILKCNNKTFNGTGPCTNVSTVQCTHGIRPVVSTQLLLNGSLAEEDV VIRSANFTDNAKTIIVQLNTSVEINCTRPNNNTRKSIRIQRGPGRAFV TIGKIGNMRQAHCNISRAKWNATLKQIASKLREQFGNNKTIIFKQSS GGDPEIVTHSFNCGGEFFYCNSTQLFNSTWFNSTWSTEGSNNTEGSD TITLPCRIKQFINMWQEVGKAMYAPPISGQIRCSSNITGLLLTRDGGN NNNGSEIFRPGGGDMRDNWRSELYKYKVVKIEPLGVAPTKKSLEQI WNNMTWMEWDREINNYTSLIHSLIEESQNQQEKNEQELLELDKWA SLWNWFNITNWLWYIKLFIMIVGGLVGLRIVFAVLSIVNRVRQGYS PLSFQTHLPIPRGPDRPEGIEEEGGERDRDRSIRLVNGSLALIWDDLR SLCLFSYHRLRDLLLIVTRIVELLGRRGWEALKYWWNLLQYWSQE LKNSAVNLLNATAIAVAEGTDRVIEVLQAAYRAIRHIPRRIRQGLERI LL |
| 18 | BG505 gp160 (aa662-859 are transmembrane region and intracellular region) | MRVMGIQRNCQHLFRWGTMILGMIIICSAAENLWVTVYYGVPVWK DAETTLFCASDAKAYETEKHNVWATHACVPTDPNPQEIHLENVTEE FNMWKNNMVEQMHTDIISLWDQSLKPCVKLTPLCVTLQCTNVTNN ITDDMRGELKNCSFNMTTELRDKKQKVYSLFYRLDVVQINENQGN RSNNSNKEYRLINCNTSAITQACPKVSFEPIPIHYCAPAGFAILKCKD KKFNGTGPCPSVSTVQCTHGIKPVVSTQLLLNGSLAEEEVMIRSENI TNNAKNILVQFNTPVQINCTRPNNNTRKSIRIGPGQAFYATGDIIGDI RQAHCTVSKATWNETLGKVVKQLRKHFGNNTIIRFANSSGGDLEVT THSFNCGGEFFYCNTSGLFNSTWISNTSVQGSNSTGSNDSITLPCRIK QIINMWQRIGQAMYAPPIQGVIRCVSNITGLILTRDGGSTNSTTETFR PGGGDMRDNWRSELYKYKVVKIEPLGVAPTRAKRRVVGREKRAV GIGAVFLGFLGAAGSTMGAASMTLTVQARNLLSGIVQQQSNLLRAI |
| | | EAQQHLLKLTVWGIKQLQARVLAVERYLRDQQLLGIWGCSGKLIC TTNVPWNSSWSNRNLSEIWDNMTWLQWDKEISNYTQIIYGLLEESQ NQQEKNEQDLLALDKWASLWNWFDISNWLWYIKIFIMIVGGLIGLR IVFAVLSVIHRVRQGYSPLSFQTHTPNPRGLDRPERIEEEDGEQDRGR STRLVSGFLALAWDDLRSLCLFCYHRLRDFILIAARIVELLGHSSLK GLRLGWEGLKYLWNLLAYWGRELKISAINLFDTIAIAVAEWTDRVI EIGQRLCRAFLHIPRRIRQGLERAL |
| 19 | NL4-3 gp160 (aa663-854 are transmembrane region and intracellular region) | MRVKEKYQHLWRWGWKWGTMLLGILMICSATEKLWVTVYYGVP VWKEATTTLFCASDAKAYDTEVHNVWATHACVPTDPNPQEVVLV NVTENFNMWKNDMVEQMHEDIISLWDQSLKPCVKLTPLCVSLKCT DLKNDTNTNSSSGRMIMEKGEIKNCSFNISTSIRDKVQKEYAFFYKL DIVPIDNTSYRLISCNTSVITQACPKVSFEPIPIHYCAPAGFAILKCNN KTFNGTGPCTNVSTVQCTHGIRPVVSTQLLLNGSLAEEDVVIRSANF TDNAKTIIVQLNTSVEINCTRPNNNTRKSIRIQRGPGRAFVTIGKIGN MRQAHCNISRAKWNATLKQIASKLREQFGNNKTIIFKQSSGGDPEIV THSFNCGGEFFYCNSTQLFNSTWFNSTWSTEGSNNTEGSDTITLPCRI KQFINMWQEVGKAMYAPPISGQIRCSSNITGLLLTRDGGNNNNGSEI FRPGGGDMRDNWRSELYKYKVVKIEPLGVAPTKAKRRVVQREKR AVGIGALFLGFLGAAGSTMGAASMTLTVQARQLLSDIVQQQNNLL RAIEAQQHLLQLTVWGIKQLQARILAVERYLKDQQLLGIWGCSGKL ICTTAVPWNASWSNKSLEQIWNNMTWMEWDREINNYTSLIHSLIEE SQNQQEKNEQELLELDKWASLWNWFNITNWLWYIKLFIMIVGGLV GLRIVFAVLSIVNRVRQGYSPLSFQTHLPIPRGPDRPEGIEEEGGERD RDRSIRLVNGSLALIWDDLRSLCLFSYHRLRDLLLIVTRIVELLGRRG WEALKYWWNLLQYWSQELKNSAVNLLNATAIAVAEGTDRVIEVL QAAYRAIRHIPRRIRQGLERILL* |
| 20 | 25710 gp160 (aa657-855 are transmembrane region and intracellular region) | MRVRGTLRNYQQWWIWGVLGFWMLMICNVGGNLWVTVYYGVP VWKEATTTLFCASDAKAYDKEVHNVWATHACVPTDPNPQEMVLG NVTENFNMWKNEMVNQMHEDVISLWDQSLKPCVKLTPLCVTLEC SNVTYNESMKEVKNCSFNLTTELRDKKQKVHALFYRLDIVPLNDTE KKNSSRPYRLINCNTSAITQACPKVTFDPIPIHYCTPAGYAILKCNDK KFNGTGPCHKVSTVQCTHGIKPVVSTQLLLNGSLAEGEIIIRSENLTN NAKTIIVHLNQSVEIVCARPSNNTRTSIRIGPGQTFYATGAITGDIRQA HCNISKDKWNETLQRVGEKLAEHFPNKTIKFNSSSGGDLEITTHSFN CRGEFFYCNTSGLFNGTFNGTYVSPNSTDSNSSSIITIPCRIKQIINMW QEVGRAMYAPPIAGNITCKSNITGLLLVRDGGTGSESNKTEIFRPGG GDMRDNWRSELYKYKVVEIKPLGVAPTKAKRRVVEREKRAVGIGA VFLGFLGAAGSTMGAASITLTVQARQLLSGIVQQQSNLLRAIEAQQ HLLQLTVWGIKQLQTRVLAIERYLKDQQLLGIWGCSGKLICTTAVP WNYSWSNRSQDDIWDNMTWMQWDKEISNYTNTIYKLLEDSQIQQ EKNEKDLLALDSWENLWNWFNITNWLWYIKIFIIIVGGLIGLRIIFAV LPIVNRVRQGYSPLSFQTHTPTPGGPDRLGRIEEEGGEQDNVRSIRLV NGFLALAWDDLRNLCLFSYHRLRDFILVAARVVELLGRNSLRGLQK GWEALKYLGSLVQYWGLELKRSAISLLDTIAIAVAEGTDRIIQLGQG ICRAICNIPRRIRQGLEAALQ |
| 21 | X1632 gp160 (aa660-858 are transmembrane region and intracellular region) | MKVKGTQRDWHSLWNWGILILGLVIICSASNNLWVTVYYGVPVW EDADTTLFCASDAKAYSTESHNVWATHACVPTDPNPQEIYLENVTE DFNMWENNMVEQMQEDIISLWDESLKPCVKLTPLCVTLTCTNVTN VTDSVGTNSRLKGYKEELKNCSFNTTTEIRDKKKQEYALFYKLDIV PINDNSNNSNGYRLINCNVSTIKQACPKVSFDPIPIHYCAPAGFAILK CRDKEFNGTGTCRNVSTVQCTHGIKPVVSTQLLLNGSLAEGDIVIRS ENITDNAKTIIVHLNKTVSITCTRPNNNTRKSIRIGPGQALYATGAIIG DTRQAHCNINGSEWYEMIQNVKNKLNETFKKNITFNPSSGGDLEITT HSFNCRGEFFYCNTSELFNSSHLFNGSTLSTNGTITLPCRIKQIVRMW QRVGQAMYAPPIAGNITCRSNITGLLLTRDGGTNKDTNEAETFRPG GGDMRDNWRSELYKYKVVKIKPLGVAPTRARRRVVEREKRAIGLG TVLLGFLGTAGSTMGAASITLTVQVRQLLSGIVQQQSNLLRAIEAQQ HLLQLTVWGIKQLQARVLAVERYLKDQQILGIWGCSGKLICTTNVP WNSSWSNKSYSDIWDNLTWIQWEREISNYTQQIYTLLEESQNQQEK |
| | | NEQELLALDKWASLWNWFDITNWLWYIKIFIMIVGGLIGLRIVFAV LSIINRVRKGYSPLSFQTLTRHQREPDRPGGIEEEDGEQDRDKSVRFV SGFLSPVWDDLRSLCLFSYRRLRDFILVAARTVELLGRSSLKGLRLG WEGLKYLWNLLLYWGRELKSSAINLLDTTAIAVANWTDRVIEVGQ RIVRAFLHIPVRIRQGLERALL |
| 22 | CH119 gp160 (aa663-862 are transmembrane region and intracellular region) | MRVTGIRKNYRHLWRWGTMLLGMLMICSAVGNLWVTVYYGVPV WKEATTTLFCASDAKAYDTEVHNVWATHACVPTDPSPQELVLENVT ENFNMWKNEMVNQMHEDVISLWDQSLKPCVKLTPLCVTLECSKVS NNETDKYNGTEEMKNCSFNATTVVRDRQQKVYALFYRLDIVPLTEK NSSENSSKYYRLINCNTSAITQACPKVSFEPIPIHYCTPAGYAILKCND KTFNGTGPCHNVSTVQCTHGIKPVVSTQLLLNGSLAEGEIIIRSENLT NNVKTILVHLNQSVEIVCTRPNNNTRKSIRIGPGQTFYATGDIIGDIRQ AHCNISKWHETLKRVSEKLAEHFPNKTINFTSSSGGDLEITTHSFTCR GEFFYCNTSGLFNSTYMPNGTYLHGDTNSNSSITIPCRIKQIINMWQE VGRAMYAPPIEGNITCKSNITGLLLVRDGGTESNNTETNNTEIFRPGG GDMRDNWRSELYKYKVVEIKPLGVAPTAAKRRVVEREKRAVGIGA VFLGFLGVAGSTMGAASMTLTVQARQLLSGIVQQQSNLLRAIEAQQ HLLQLTVWGIKQLQTRVLAIERYLKDQQLLGIWGCSGKLICTTAVPW NSSWSNKSQKEIWDNMTWMQWDKEISNYTNTIYKLLEDSQNQQES NEKDLLALDSWNNLWNWFNITQWLWYIKIFIIIVGGLIGLRIIFAVLSI VNRVRQGYSPLSFQTLTPTSGGRPDRLERIEEEGGEQDRDRSIRLVNG FLALAWDDLRNLCLFSYHRLRDFILVAARVVELLGRTSLRGLQRGW EALKYLGSLVQYWGQELKKSAISLVDTIAIVVAEGTDRIIDIVQAFCR AIYNIPRRIRQGFEAALQ |
| 23 | CNE8 gp160 (aa652-850 are transmembrane region and intracellular region) | MRVKETQMNWPNLWKWGTLILGLVIICSASDNLWVTVYYGVPVW RDADTTLFCASDAKAYDTEVHNVWATHACVPTDPNPQEIHLENVT ENFNMWKNKMAEQMQEDVISLWDESLKPCVQLTPLCVTLNCTNA NLNATVNASTTIGNITDEVRNCSFNTTTELRDKKQNVYALFYKLDIV PINNNSEYRLINCNTSVIKQACPKVSFDPIPIHYCAPAGYAILRCNDK NFNGTGPCKNVSSVQCTHGIKPVVSTQLLLNGSLAEDEIIIRSENLTD NVKTIIVHLNKSVEINCTRPSNNTRTSVRIGPGQVFYRTGDIIGDIRK AYCEINRTKWHETLKQVATKLREHFNKTIIFQPPSGGDIEITMHHFN CRGEFFYCNTTKLFNSTWGENTTMEGHNDTIVLPCRIKQIVNMWQG VGQAMYAPPIRGSINCVSNITGILLTRDGGTNMSNETFRPGGGNIKD NWRSELYKYKVVEIEPLGIAPTKAKRRVVEREKRAVGIGAMIFGFL GAAGSTMGAASITLTVQARQLLSGIVQQQSNLLRAPEAQQHLLQLT VWGIKQLQARVLAVERYLKDQKFLGLWGCSGKIICTTAVPWNSTW SNRSYEEIWDNMTWIEWEREISNYTSQIYEILTESQNQQDRNEKDLL ELDKWASLWNWFDITRWLWYIKIFIMIVGGLIGLRIIFAVLSIVNRVR QGYSPLSFQTPTHHQREPDRPERIEEGGGEQDRDRSVRLVSGFLALA WDDLRSLCLFSYHRLRDLILIAVRTVELLGHGGLKGLRRGWEGLKY LGNLLLYWGQELKISAISLLDATAIAVAGWTDRIIEVAQRAWRAILH IPRRIRQGLERSLL |
| 24 | p246F3 gp160 (aa654-852 are transmembrane region and intracellular region) | MRARGMLRTWQHWWIWGILGFWMLMICNMQDLWVTVYYGVPV WKDAKTTLFCASDAKAYEKEVHNVWATHACVPTDPNPQEIVMAN VTEEFNMWKNNMVEQMHTDIISLWDQSLKPCVKLTPLCVTLDCKD YNYSITNNSTGMEGEIKNCSYNITTELRDKRQKVYSLFYRLDVVQIN DSNDRNNSQYRLINCNTTTMTQACPKVTFDPIPIHYCAPAGFAILKC NNKTFNGKGPCNNVSSVQCTHGIKPVVSTQLLLNGSLAEKEIVIRSE NLTDNVKTIIVHLNESVEINCTRPNNNTRKSVRIGPGQTFYATGDIIG NIRQAHCTVNKTEWNTALTRVSKKLKEYFPNKTIAFQPSSGGDLEIT TFSFNCRGEFFYCNTSDLFNGTFNETSGQFNSTFNSTLQCRIKQIINM WQEVGQAMYAPPIAGSITCISNITGLILTRDGGNTNSTKETFRPGGGN MRDNWRSELYKYKVVKIEPLGVAPTKARRRVVEREKRAVGIGAVFI GFLGAAGSTMGAASITLTVQARQLLSGIVQQQSNLLRAIEAQQHLL KLTVWGIKQLQARVLAVERYLKDQQLLGIWGCSGKLICTTNVPWNS SWSNKSQDEIWDNMTWLQWDKEISNYTQIIYNLIEESQTQQELNER DLLALDKWANLWNWFDITKWLWYIKIFIMIVGGLIGLRIIFAVLSIVN RVRQGYSPLSFQTLTPNPRGPDRPGGIEEEGGEQGRNSYTRLVSGFLP |
| | | LAWDDLRSLCLFSYHLLRDFILIAARAAELLGRSSLRGLQRGWETLK YLGSLVQYWGLELKKSAISLLDTIAIQVAEGTDRIIELIQGIYRAIRNIP RRIRQGAETALV |
| 25 | Bal.26 gp160 (aa662-853 are transmembrane region and intracellular region) | MRVTEIRKSYQHWWRWGIMLLGMLMICNAEEKLWVTVYYGVPV WKEATTTLFCASDAKAYDTEVHNVWATHACVPTDPNPQEVKMEN VTENFNMWKNNVVEQMHEDIISLWDQSLKPCVKLTPLCVTLNCTD LKNATNGNNTNTTSSSGGMMGGGEMKNCSFNITTNIRGKVQKEYA LFYELDIVPIDNKIDSYRLISCNTSVITQACPKVSFEPIPIHYCAPAGFA ILKCKDKKFNGKGPCSNVSTVQCTHGIRPVVSTQLLLNGSLAEEEV VIRSENFTNNAKIIVVQLNESVEINCTRPNNNTRKSIHIGPGRAFYTT GEIIGDIRQAHCNLSRAKWNDTLNKIVIKLREQFGNKTIVFKHSSGG DPEIVTHSFNCGGEFFYCNSTQLFNSTWNVTEESNNTVENNTITLPC RIKQIINMWQEVGRAMYAPPIRGQIRCSSNITGLLLTRDGGPEDDKT EVFRPGGGDMRDNWRSELYKYKVVKIEPLGVAPTKAKRRVVQRE KRAVGIGAVLLGFLGAAGSTMGAASMTLTVQARLLLSGIVQQQNN LLRAIEAQQHLLQLTVWGIKQLQARVLAVERYLRDQQLLGIWGCS GKLICTTAVPWNASWSNKSLNKIWDNMTWMEWDREINNYTSIIYS LIEESQNQQEKNEQELLELDKWASLWNWFDITKWLWYIKIFIMIVG GLIGLRIVFSVLSIVNRVRQGYSPLSFQTHLPASRGPDRPGGIEEEGG ERDRDRSGPLVNGFLTLIWVDLRSLFLFSYHRLRDLLLIVTRIVELLG RRGWEVLKYWWNLLQYWSQELKNSAVSLLNTIAIAVAEGTDRVIE VVQRAVRAILHIPRRIRQGLERALL* |
| 26 | BG505- deleted sequence from gp41 | WNSSWSN |
| 27 | BG505- deleted sequence from gp120 | AKRRVVGREKR |
| 28 | Linker-1 | GGGGS |
| 29 | Linker-2 | GGGGSGGGGS |
| 30 | BG-B1(1/1) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSAENLWVTVYYGVPVWKDAETTLFCASDAKAYETEKHNVWATH ACVPTDPNPQEIHLENVTEEFNMWKNNMVEQMHTDIISLWDQSLK PCVKLTPLCVTLQCTNVTNNITDDMRGELKNCSFNMTTELRDKKQ KVYSLFYRLDVVQINENQGNRSNNSNKEYRLINCNTSAITQACPKV SFEPIPIHYCAPAGFAILKCKDKKFNGTGPCPSVSTVQCTHGIKPVVS TQLLLNGSLAEEEVMIRSENITNNAKNILVQFNTPVQINCTRPNNNT RKSIRIGPGQAFYATGDIIGDIRQAHCNVSKATWNETLGKVVKQLR KHFGNNTIIRFANSSGGDLEVTTHSFNCGGEFFYCNTSGLFNSTWIS NTSVQGSNSTGSNDSITLPCRIKQIINMWQRIGQAMYAPPIQGVIRCV SNITGLILTRDGGSTNSTTETFRPGGGDMRDNWRSELYKYKVVKIEP LGVAPTRAKRRVVGGGGGSWNSSWSNRNLSEIWDNMTWLQWDK EISNYTQIIYGLLEESQNQQEKNEQDLLALDHHHHHHHH |
| 31 | BG-B 1(1/2) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSAENLWVTVYYGVPVWKDAETTLFCASDAKAYETEKHNVWATH ACVPTDPNPQEIHLENVTEEFNMWKNNMVEQMHTDIISLWDQSLK PCVKLTPLCVTLQCTNVTNNITDDMRGELKNCSFNMTTELRDKKQ KVYSLFYRLDVVQINENQGNRSNNSNKEYRLINCNTSAITQACPKV SFEPIPIHYCAPAGFAILKCKDKKFNGTGPCPSVSTVQCTHGIKPVVS TQLLLNGSLAEEEVMIRSENITNNAKNILVQFNTPVQINCTRPNNNT RKSIRIGPGQAFYATGDIIGDIRQAHCNVSKATWNETLGKVVKQLR KHFGNNTIIRFANSSGGDLEVTTHSFNCGGEFFYCNTSGLFNSTWIS NTSVQGSNSTGSNDSITLPCRIKQIINMWQRIGQAMYAPPIQGVIRCV SNITGLILTRDGGSTNSTTETFRPGGGDMRDNWRSELYKYKVVKIEP |
| | | LGVAPTRAKRRVVGGGGGSGGGGSWNSSWSNRNLSEIWDNMTWL QWDKEISNYTQIIYGLLEESQNQQEKNEQDLLALDHHHHHHHH |
| 32 | BG-B 1(2/2) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSGGGGSAENLWVTVYYGVPVWKDAETTLFCASDAKAYETEKHN VWATHACVPTDPNPQEIHLENVTEEFNMWKNNMVEQMHTDIISLW DQSLKPCVKLTPLCVTLQCTNVTNNITDDMRGELKNCSFNMTTELR DKKQKVYSLFYRLDVVQINENQGNRSNNSNKEYRLINCNTSAITQA CPKVSFEPIPIHYCAPAGFAILKCKDKKFNGTGPCPSVSTVQCTHGIK PVVSTQLLLNGSLAEEEVMIRSENITNNAKNILVQFNTPVQINCTRPN NNTRKSIRIGPGQAFYATGDIIGDIRQAHCNVSKATWNETLGKVVK QLRKHFGNNTIIRFANSSGGDLEVTTHSFNCGGEFFYCNTSGLFNST WISNTSVQGSNSTGSNDSITLPCRIKQIINMWQRIGQAMYAPPIQGVI RCVSNITGLILTRDGGSTNSTTETFRPGGGDMRDNWRSELYKYKVV KIEPLGVAPTRAKRRVVGGGGGSGGGGSWNSSWSNRNLSEIWDNM TWLQWDKEISNYTQIIYGLLEESQNQQEKNEQDLLALDHHHHHH H |
| 33 | BG-B1(2/1) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSGGGGSAENLWVTVYYGVPVWKDAETTLFCASDAKAYETEKHN VWATHACVPTDPNPQEIHLENVTEEFNMWKNNMVEQMHTDIISLW DQSLKPCVKLTPLCVTLQCTNVTNNITDDMRGELKNCSFNMTTELR DKKQKVYSLFYRLDVVQINENQGNRSNNSNKEYRLINCNTSAITQA CPKVSFEPIPIHYCAPAGFAILKCKDKKFNGTGPCPSVSTVQCTHGIK PVVSTQLLLNGSLAEEEVMIRSENITNNAKNILVQFNTPVQINCTRPN NNTRKSIRIGPGQAFYATGDIIGDIRQAHCNVSKATWNETLGKVVK QLRKHFGNNTIIRFANSSGGDLEVTTHSFNCGGEFFYCNTSGLFNST WISNTSVQGSNSTGSNDSITLPCRIKQIINMWQRIGQAMYAPPIQGVI RCVSNITGLILTRDGGSTNSTTETFRPGGGDMRDNWRSELYKYKVV KIEPLGVAPTRAKRRVVGGGGGSWNSSWSNRNLSEIWDNMTWLQ WDKEISNYTQIIYGLLEESQNQQEKNEQDLLALDHHHHHHHH |
| 34 | BG-B 1 (2/3) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSGGGGSAENLWVTVYYGVPVWKDAETTLFCASDAKAYETEKHN VWATHACVPTDPNPQEIHLENVTEEFNMWKNNMVEQMHTDIISLW DQSLKPCVKLTPLCVTLQCTNVTNNITDDMRGELKNCSFNMTTELR DKKQKVYSLFYRLDVVQINENQGNRSNNSNKEYRLINCNTSAITQA CPKVSFEPIPIHYCAPAGFAILKCKDKKFNGTGPCPSVSTVQCTHGIK PVVSTQLLLNGSLAEEEVMIRSENITNNAKNILVQFNTPVQINCTRPN NNTRKSIRIGPGQAFYATGDIIGDIRQAHCNVSKATWNETLGKVVK QLRKHFGNNTIIRFANSSGGDLEVTTHSFNCGGEFFYCNTSGLFNST WISNTSVQGSNSTGSNDSITLPCRIKQIINMWQRIGQAMYAPPIQGVI RCVSNITGLILTRDGGSTNSTTETFRPGGGDMRDNWRSELYKYKVV KIEPLGVAPTRAKRRVVGGGGGSGGGGSGGGGSWNSSWSNRNLSE IWDNMTWLQWDKEISNYTQIIYGLLEESQNQQEKNEQDLLALDHH HHHHHH |
| 35 | BG-B2(1-1) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSLWVTVYYGVPVWKDAETTLFCASDAKAYETEKHNVWATHAC VPTDPNPQEIHLENVTEEFNMWKNNMVEQMHTDIISLWDQSLKPC VKLTPLCVTLQCTNVTNNITDDMRGELKNCSFNMTTELRDKKQKV YSLFYRLDVVQINENQGNRSNNSNKEYRLINCNTSAITQACPKVSFE PIPIHYCAPAGFAILKCKDKKFNGTGPCPSVSTVQCTHGIKPVVSTQL |
| | | LLNGSLAEEEVMIRSENITNNAKNILVQFNTPVQINCTRPNNNTRKSI RIGPGQAFYATGDIIGDIRQAHCNVSKATWNETLGKVVKQLRKHFG NNTIIRFANSSGGDLEVTTHSFNCGGEFFYCNTSGLFNSTWISNTSVQ GSNSTGSNDSITLPCRIKQIINMWQRIGQAMYAPPIQGVIRCVSNITG LILTRDGGSTNSTTETFRPGGGDMRDNWRSELYKYKVVKIEPLGVA PTRAKRRVVGGGGGSWNSSWSNRNLSEIWDNMTWLQWDKEISNY TQIIYGLLEESQNQQEKNEQDLLALDHHHHHHHH |
| 36 | BG-B2(1-2) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSLWVTVYYGVPVWKDAETTLFCASDAKAYETEKHNVWATHAC VPTDPNPQEIHLENVTEEFNMWKNNMVEQMHTDIISLWDQSLKPC VKLTPLCVTLQCTNVTNNITDDMRGELKNCSFNMTTELRDKKQKV YSLFYRLDVVQINENQGNRSNNSNKEYRLINCNTSAITQACPKVSFE PIPIHYCAPAGFAILKCKDKKFNGTGPCPSVSTVQCTHGIKPVVSTQL LLNGSLAEEEVMIRSENITNNAKNILVQFNTPVQINCTRPNNNTRKSI RIGPGQAFYATGDIIGDIRQAHCNVSKATWNETLGKVVKQLRKHFG NNTIIRFANSSGGDLEVTTHSFNCGGEFFYCNTSGLFNSTWISNTSVQ GSNSTGSNDSITLPCRIKQIINMWQRIGQAMYAPPIQGVIRCVSNITG LILTRDGGSTNSTTETFRPGGGDMRDNWRSELYKYKVVKIEPLGVA PTRAKRRVVGGGGGSGGGGGSWNSSWSNRNLSEIWDNMTWLQW DKEISNYTQIIYGLLEESQNQQEKNEQDLLALDHHHHHHHH |
| 37 | BG-B2(1-3) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPGGG GSLWVTVYYGVPVWKDAETTLFCASDAKAYETEKHNVWATHAC VPTDPNPQEIHLENVTEEFNMWKNNMVEQMHTDIISLWDQSLKPC VKLTPLCVTLQCTNVTNNITDDMRGELKNCSFNMTTELRDKKQKV YSLFYRLDVVQINENQGNRSNNSNKEYRLINCNTSAITQACPKVSFE PIPIHYCAPAGFAILKCKDKKFNGTGPCPSVSTVQCTHGIKPVVSTQL LLNGSLAEEEVMIRSENITNNAKNILVQFNTPVQINCTRPNNNTRKSI RIGPGQAFYATGDIIGDIRQAHCNVSKATWNETLGKVVKQLRKHFG NNTIIRFANSSGGDLEVTTHSFNCGGEFFYCNTSGLFNSTWISNTSVQ GSNSTGSNDSITLPCRIKQIINMWQRIGQAMYAPPIQGVIRCVSNITG LILTRDGGSTNSTTETFRPGGGDMRDNWRSELYKYKVVKIEPLGVA PTRAKRRVVGGGGGSGGGGSGGGGSWNSSWSNRNLSEIWDNMTW LQWDKEISNYTQIIYGLLEESQNQQEKNEQDLLALDHHHHHHHH |
| 38 | BG-C1(1/1) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPWNS SWSNGGGGSAENLWVTVYYGVPVWKDAETTLFCASDAKAYETEK HNVWATHACVPTDPNPQEIHLENVTEEFNMWKNNMVEQMHTDIIS LWDQSLKPCVKLTPLCVTLQCTNVTNNITDDMRGELKNCSFNMTT ELRDKKQKVYSLFYRLDVVQINENQGNRSNNSNKEYRLINCNTSAI TQACPKVSFEPIPIHYCAPAGFAILKCKDKKFNGTGPCPSVSTVQCT HGIKPVVSTQLLLNGSLAEEEVMIRSENITNNAKNILVQFNTPVQINC TRPNNNTRKSIRIGPGQAFYATGDIIGDIRQAHCNVSKATWNETLGK VVKQLRKHFGNNTIIRFANSSGGDLEVTTHSFNCGGEFFYCNTSGLF NSTWISNTSVQGSNSTGSNDSITLPCRIKQIINMWQRIGQAMYAPPIQ GVIRCVSNITGLILTRDGGSTNSTTETFRPGGGDMRDNWRSELYKY KVVKIEPLGVAPTRAKRRVVGGGGGSRNLSEIWDNMTWLQWDKEI SNYTQIIYGLLEESQNQQEKNEQDLLALDHHHHHHHH |
| 39 | BG-C1(1/2) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPWNS SWSNGGGGSAENLWVTVYYGVPVWKDAETTLFCASDAKAYETEK HNVWATHACVPTDPNPQEIHLENVTEEFNMWKNNMVEQMHTDIIS LWDQSLKPCVKLTPLCVTLQCTNVTNNITDDMRGELKNCSFNMTT ELRDKKQKVYSLFYRLDVVQINENQGNRSNNSNKEYRLINCNTSAI |
| | | TQACPKVSFEPIPIHYCAPAGFAILKCKDKKFNGTGPCPSVSTVQCT HGIKPVVSTQLLLNGSLAEEEVMIRSENITNNAKNILVQFNTPVQINC TRPNNNTRKSIRIGPGQAFYATGDIIGDIRQAHCNVSKATWNETLGK VVKQLRKHFGNNTIIRFANSSGGDLEVTTHSFNCGGEFFYCNTSGLF NSTWISNTSVQGSNSTGSNDSITLPCRIKQIINMWQRIGQAMYAPPIQ GVIRCVSNITGLILTRDGGSTNSTTETFRPGGGDMRDNWRSELYKY KVVKIEPLGVAPTRAKRRVVGGGGGSGGGGSRNLSEIWDNMTWLQ WDKEISNYTQIIYGLLEESQNQQEKNEQDLLALDHHHHHHHH |
| 40 | BG-C1(1/3) | MDAMKRGLCCVLLLCGAVFVSPSQEIHARFRRGARAVGIGAVFLGF LGAAGSTMGAASMTLTVQARNLLSGIVQQQSNLLRAPEAQQHLLK LTVWGIKQLQARVLAVERYLRDQQLLGIWGCSGKLICTTNVPWNS SWSNGGGGSAENLWVTVYYGVPVWKDAETTLFCASDAKAYETEK HNVWATHACVPTDPNPQEIHLENVTEEFNMWKNNMVEQMHTDIIS LWDQSLKPCVKLTPLCVTLQCTNVTNNITDDMRGELKNCSFNMTT ELRDKKQKVYSLFYRLDVVQINENQGNRSNNSNKEYRLINCNTSAI TQACPKVSFEPIPIHYCAPAGFAILKCKDKKFNGTGPCPSVSTVQCT HGIKPVVSTQLLLNGSLAEEEVMIRSENITNNAKNILVQFNTPVQINC TRPNNNTRKSIRIGPGQAFYATGDIIGDIRQAHCNVSKATWNETLGK VVKQLRKHFGNNTIIRFANSSGGDLEVTTHSFNCGGEFFYCNTSGLF NSTWISNTSVQGSNSTGSNDSITLPCRIKQIINMWQRIGQAMYAPPIQ GVIRCVSNITGLILTRDGGSTNSTTETFRPGGGDMRDNWRSELYKY KVVKIEPLGVAPTRAKRRVVGGGGGSGGGGSGGGGSRNLSEIWDN MTWLQWDKEISNYTQIIYGLLEESQNQQEKNEQDLLALDHHHHHH HH |
| 41 | gp160 protein-furin cleavage site | REKR |

### EXAMPLES

The present invention will now be described with reference to the following examples, which are intended to illustrate the present invention, but not to limit it.

Unless otherwise specified, the molecular biology experiment methods and immunoassay methods used in the present invention were basically referred to the methods described by J. Sambrook et al., Molecular Cloning: A Laboratory Manual, 2nd Edition, Cold Spring Harbor Laboratory Press, 1989, and F. M. Ausubel et al., Short protocols in Molecular Biology, 3rd Edition, John Wiley & Sons, Inc., 1995; the restriction enzymes were used in accordance with the conditions recommended by the product manufacturer. Those skilled in the art understand that the examples describe the present invention by way of example and are not intended to limit the scope of the present invention.

### Example 1: Expression of TSTIP proteins of two strains of NL4-3/BG505

### Modification design of 4-3/BG505 TSTIP

The base and amino acid sequences of gp160 of the two strains BG505/pNL4-3 on NCBI were used as templates for modification. Taking the modification of BG505 as an example, according to the three-dimensional cryoelectron microscope structure of BG505-SOSIP (PDB: 4tvp), part of the loop sequence (aa610-616, WNSSWSN) at the C-terminal of β27 of BG505 gp41 was removed, and the sequence of 10 amino acids including furin cleavage site at the C-terminal of gp120 (aa501-511, AKRRVVGREKR) was also removed. Subsequently, the truncated C-terminal of β27 of gp41 was linked to the N-terminal of gp120, and the truncated C-terminal of gp120 was linked to the N-terminal of the α8 domain of gp41. The amino acids of the complete gp140 after modification were arranged in sequence as follows: α6/α7/β27 (501-606) + part of the loop region between β27 and α8 (607-609) + gp120 (33-500) + part of the C-terminal sequence of the loop region between β27 and α8 (617-618) + α8/α9 (619-664).

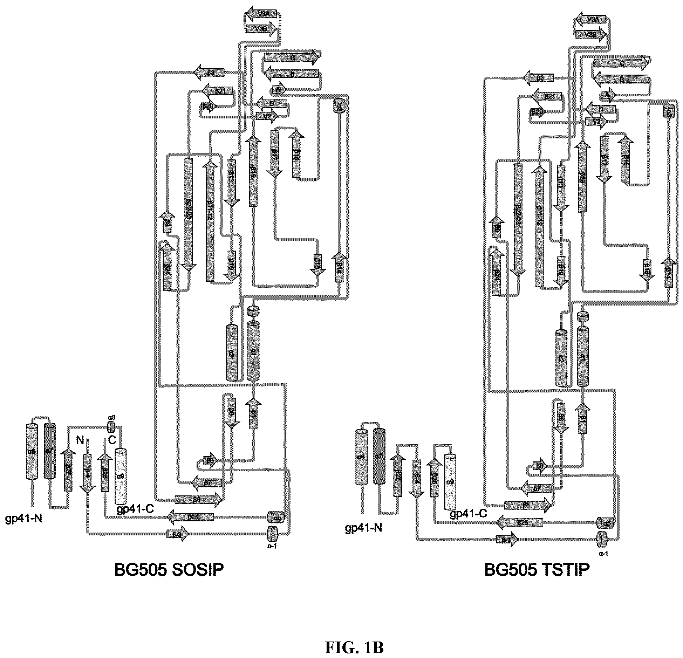

In addition, 8His tag was added to the C-terminal of the above sequence to facilitate purification, and tPA signal peptide and kozak sequence were added in front of the sequence to promote secretion and expression. The protein designed above was named as BG505-TST (SEQ ID NO: 1). On the basis of the above structure, I559P and T332N mutations were introduced, and the protein designed was named as BG505-TSTIP (SEQ ID NO: 3). NL4-3 strain was modified in the same way to obtain NL4-3-TST (SEQ ID NO: 2) and NL4-3-TSTIP (SEQ ID NO: 4), respectively. The structure of each of the above proteins is shown in FIG. 1.

The designed amino acid sequences of BG505/pNL4-3-TSTIP were converted into base sequences suitable for mammalian cell expression, and then sent to Sangon Biotech (Shanghai) Co., Ltd. for gene synthesis, and constructed between the ECORI and Xbal restriction sites of pcDNA3.1 vector. 1µl of the synthesized pcDNA3.1-BG505SOSIP/4-3TSTIP plasmid was taken to transform 50µl of DH5α competent cells (purchased from Shenzhen Kangti Life Technology Co., Ltd.), which were plated on ampicillin-containing solid medium; after static culture at 37°C for 10-12 hours, single colony was clearly visible, and was picked and placed into a test tube containing 3ml of ampicillin-containing LB medium, and cultured under shaking at 220 rpm at 37°C for 10 hours; 500µl of the bacterial solution was taken and mixed with 500µl of 50% glycerol, then cryopreserved at -20°C.

### Extraction of PcDNA3.1 NL4-3/BG505 TSTIP plasmid:

10 µl of the pcDA3.1 NL4-3/BG505 TSTIP bacterial solution cryopreserved at -20°C was taken, transferred into a test tube containing 3ml of ampicillin-containing LB medium, cultured for 10-12h, then inoculated in a conical flask containing 500ml of ampicillin-containing LB medium and cultured at 37°C for 12 hours; the bacterial solution was collected and centrifuged at 7000g for 10 minutes, the supernatant was discarded. The pcDNA3.1-BG505/NL4-3TSTIP plasmid was extracted by using Tiangen Endotoxin-Free Maxi Plasmid Kit.

### Culture and passage of 293F cells

293F cells cryopreserved in -80°C refrigerator were taken, thawed at 37°C, then centrifuged at 1300rpm for 4min; the supernatant was discarded in an ultra-clean bench, the cells were lightly flicked and resuspended in 293freestyle medium warmed at 37°C in advance, then transferred into a conical flask containing 50ml of warm culture medium, for suspension culture at 37°C, 5% CO₂, 120 rpm; the cells were passaged when the cell density reached 2.0^{∗}10⁶, to gradually expand the culture system.

### Transient transfection

The 293F cells were transiently transfected with PEG2000. After the cell density reached 2.0^{∗}10⁶, the cells were harvested in a sterile 50ml tube, centrifuged at 1300rpm for 4min; the cells were flicked, then resuspended in medium incubated at 37°C, transferred to a conical flask containing 450ml of medium incubated at 37°C, and placed on a shaker at 37°C for later use.

The extracted pcDNA3.1-BG505/NL4-3TSTIP plasmid and PEG2000 in a ratio of 1:2 was added into 50ml of culture medium, mixed well and allowed to stand for 18min, then transferred into the above 450ml of culture medium, for suspension-culture at 37°C, 5% CO₂, 120rpm for 6 days to express BG505/NL4-3TSTIP protein. It should be aware the PEG operation should be carried out in the dark during transfection.

### Example 2: Purification of NL4-3/BG505 TSTIP protein

After 6 days of transient transfection, the cell culture medium was collected, centrifuged at 7000g for 10 minutes with JA-14 rotor; the cell supernatant was collected, centrifuged at 20000g for 10 minutes; the supernatant was taken and filtered twice with a 0.22µm pore size membrane filter, and this sample was used for the next step of Ni-excel column purification.
Purification by Ni affinity chromatography using the AKTA system;
Instrument system: AKTA Pure type preparative liquid chromatograph;
Purification medium: Ni Sepharose excel affinity medium; buffer: buffers A and B, buffer A was 1 ×PBS buffer, buffer B was 1×PBS + 250 mmol/L imidazole buffer;
System sample-loading flow rate: 8mL/min; detection wavelength: UV@280 nm
System elution flow rate: 4ml/min; detection wavelength: UV@280 nm
Elution conditions: 20mM imidazole was used to elute impurity proteins, and the product eluted by 250mM imidazole was collected. The eluate was dialyzed against 1× PBS overnight with two dialysate changes. About 30ml of low-concentration target protein was harvested, and concentrated in Vivaspin 20ml, 100KD ultrafiltration concentrator tube to 5ml for later use.

### Example 3: Identification of biochemical properties of NL4-3/BG505 TSTIP protein SDS-PAGE:

The concentrated sample in Example 2 and BG505/4-3SOSIP protein were diluted to 1µg/µl, two tubes of 50µl samples were then taken, added with 10µl of reducing 6Loading Buffer and 10µl non-reducing 6Loading Buffer, respectively, to prepare reduced samples and non-reduced samples, the reduced samples were placed in a boiling water bath at 100°C for 10 minutes. 10µl of the reduced and non-reduced samples were electrophoresed in 8% SDS-PAGE at a voltage of 80V for 120min, and the electrophoresis bands were displayed after staining with Coomassie brilliant blue. The electrophoresis results are shown in FIGS. 2A to 2B. SDS-PAGE analysis showed that after one-step Ni-EXCEL purification, high-purity BG505/NL4-3TSTIP protein could be obtained, and the TSTIP protein showed a complete 140KD band in the presence of DTT, while the BG505/NL4-3SOSIP protein presented a 120KD band under reducing conditions, indicating that the gp120 and gp41 extracellular regions were stably and covalently linked in the protein designed by the inventors as expected.

### Molecular sieve purification:

Instrument system: AKTA Pure type preparative liquid chromatograph;
Chromatographic column: superdex 200 16/600
Column volume: 120ml
Buffer: PBS (20mM phosphate buffer, pH7.5, 150mM NaCl)
Detector wavelength: 280nm
Flow rate: 1ml/min
The sample was the purified concentrated protein of Example 2.

The purification procedure comprised: superdex200 16/600 was equilibrated with 1 column volume of PBS, the target protein purified in Example 2 was loaded using a 5ml loading loop, and the molecular sieve purification of the purified sample was performed at inject mode, and the components of the sample would be eluted in sequence according to their molecular weights, from high to low, and the elution peaks at different elution volumes were collected, which are the target proteins in different multimer forms.

The molecular sieve purification results are shown in FIG. 3. It could be seen that the components of BG505/NL4-3TSTIP designed by the inventors were more simple than those of BG505/NL4-3, while the molecular sieve purification pattern of BG505/NL4-3TSTIP showed obvious peaks for monomers and multimers, indicating that the TSTIP protein and TST protein of the present invention had a higher trimer content.

### SDS-PAGE of molecular sieve purified samples:

The samples of different elution volumes collected during the molecular sieve purification were concentrated in Vivaspin 20ml, 100KD concentrator tubes to 1µg/µl, and marked as 1, 2, 3, 4 according to the order of elution. 50µl of each of the samples was taken and added with 10µl of non-reducing 6Loading Buffer, mixed well, 10µl of non-reduced sample was taken and electrophoresed at 80V voltage for 120min in 8% SDS-PAGE, and the electrophoresis bands were displayed after staining with Coomassie brilliant blue. The electrophoresis results were shown in FIGS. 4A to 4C. It could be seen that the main component of the BG505/NL4-3TSTIP was gp140 trimer after superdex200-16/600 purification, while the BG505/NL4-3SOSIP contained a large amount of gp140 monomer.

### Thermal stability analysis by differential scanning calorimetry (DSC)

### Instrument system: VP-Capillary produced by GE Healthcare

The sample was the purified concentrated protein of Example 2.

The sample-loading tank was washed once with an acidic or weakly alkaline washing solution, and washed 3 times with deionized water; 400 µL of the sample and corresponding buffer solution thereof each was pipetted into EP tube, and centrifuged to remove sediment and air bubbles; 300 µL of buffer solution was pipetted into the sample-loading tank and control tank, respectively, and rinsed 3 times, then 300 µL of buffer solution was added to the sample-loading tank and control tank with avoiding air bubbles during the addition process; the DSC software and instrument were turned on, the number of scan cycles was set, and when the DP value in the scan cycle was stabilized between ±0.2, it was allowed to perform circular scanning and the baseline equilibration was carried out for no less than 3 times; when the last circular scanning was completed, and the temperature dropped to between 30°C and 10°C, the buffer in the sample tank was sucked out, and 300µl of protein sample to be tested was added quickly, and the scanning test was continued; the scan rate was set as: 90°C/h. After scanning, Origin7.0 was used to process the data. The results were shown in FIG. 5. The Tm of the protein BG505-TSTIP of the present invention was 74.51°C, the Tm of the control protein BG505-SOSIP was significantly lower than that of BG505-TSTIP, which was 68.18°C. The Tm of NL4-3-TSTIP was 63.41°C, and the Tm of NL4-3-SOSIP was 62.00°C; although the Tm of the one based on NL4-3 strain did not increase significantly, it was improved to a certain extent compared with the reported optimal design NL4-3SOSIP. These results indicated that our design could improve the thermal stability of the protein to a certain extent, and its thermal stability could be greatly improved in some strains.

### Example 4: Identification of BG505/NL4-3TSTIP antigenicity and immunogenicity

### Enzyme-linked immunosorbent assay (ELISA)

Broad-spectrum neutralizing antibodies such as PGT121, PGT125, VRC01, 2G12, B12 and non-neutralizing antibodies such as 17b, 447-52D, F105, F240 were selected to carry out ELISA antigenicity analysis on BG505/NL4-3TSTIP and BG505/NL4-3SOSIP, and the specific process was as follows:
(1) BG505/NL4-3TSTIP and BG505/NL4-3SOSIP trimer proteins were diluted with 1×CB to 1 µg/Ml, coated at 100 µL/well on a 96-well plate, and allowed to stand in a 37°C incubator for 2 h;
(2) the plate was washed once and spin-dried, and blocked with ED (180 µL/well) in a 37°C incubator for 2 h;
(3) the plate was washed once and spin-dried. The 96-well u-bottom plate was taken, the antibody was diluted to 1µg/ml or 10µg/ml, and added to the first well of the U-bottom plates, repeats of two wells were performed for each antibody, with 150µl at first well, and 3-fold dilution with 11 gradients. 100 µl of the diluted antibody was transferred into the 96-well ELISA plates coated with BG505/NL4-3TSTIP and BG505/NL4-3SOSIP, and reacted at 37°C for 1 hour.
(4) the plate was washed 5 times and spin-dried. The secondary antibody GAH-HRP (1:5000) was added into the 96-well plate, 100 µL/well, and reacted at 37°C for 45 minutes;
(5) the plate was washed 5 times, color development was performed at room temperature for 10 min, then stopped, and detection was carried out at wavelength of 450 nm on a microplate reader; GraphPad Prism 5 (GraphPad, USA) software was used for data analysis. The results are shown in FIG. 6.

It could be seen that BG505/NL4-3TSTIP had strong reactivity with broad-spectrum neutralizing antibodies such as PGT121, PGT125, VRC01, 2G12, and B12, but weak reactivity with non-neutralizing antibodies such as 17b, F105, and F240. 17b, F105, and F240 were antibodies targeting non-neutralizing epitopes such as CDi, CD4bs, and gp41, respectively, which indicated that BG505/NL4-3TSTIP did not expose such non-neutralizing epitopes, but well presented key epitopes such as CD4bs, out Glycan, V3, etc. Overall, the reactivities of BG505/NL4-3TSTIP with various broad-spectrum neutralizing antibodies and non-neutralizing antibodies remained comparable to those of the control protein BG505/NL4-3SOSIP.

### Immunological evaluation of BG505/NL4-3TSTIP in animals

White mice: female, 6 weeks old, purchased from Shanghai Slack Experimental Animal Co., Ltd. Four groups of immunized mice were set up, with 6 mice in each group, and BG505/NL4-3TSTIP and BG505/NL4-3SOSIP prepared in Example 2 were used as immune proteins. The proteins were diluted with normal saline, mixed with aluminum hydroxide adjuvant at a ratio of 1:1 by volume, so that the proteins were adsorbed on the adjuvant, and the mice were immunized intraperitoneally. The mouse immunization protocols were shown in Table 1.

**Table 1: Mouse immunization protocol**

| Group | Immunogen | Dose | Period (weeks) |
|---|---|---|---|
| A | NL4-3TSTIP | 10µg/animal | 2 |
| B | NL4-3SOSIP | 10µg/animal | 2 |
| C | BG505/NL4-3TSTIP | 10µg/animal | 2 |
| D | BG505/NL4-3SOSIP | 10µg/animal | 2 |

The blood was collected from mouse eyeball before immunization, and immunization was carried out according to the immunization protocol in Table 1. The blood was collected from mouse eyeball before each immunization, and after the sixth injection of immunization, the blood was collected from mouse eyeball and the mice were treated with neck dislocation. After the blood sample was placed at 37°C for 30 minutes, it was centrifuged at 13300 rpm for 10 minutes, and the serum was collected for HIV-1 pseudovirus neutralization and antibody titer determination.

The serum sample collected after the sixth injection was used to perform the virus neutralization experiment, and the results are shown in FIG. 7. For all four groups of mice, a strong neutralization response against NL4-3 was detected, but no neutralization response to the tier2 strain TRO11 of the same subtype was detected. The results showed that the BG505/NL4-3TSTIP of the present invention could induce a strong neutralizing antibody response against the corresponding strain, but it was difficult to generate a neutralizing response against other strains. In general, the protein of the present invention maintained a level of immunogenicity comparable to that of the control protein SOSIP. Those skilled in the art know that it is possible to induce virus cross-neutralizing antibodies by introducing amino acid mutations of cross-reactivity epitopes based on the protein of the present invention.

### Example 5: Production and identification of Bal.26-TSTIP pseudoviral particle

### Construction of Bal.26-TSTIP and NL4-3-TSTIP pseudoviruses

The Bal26 TSTIP pseudovirus produced by the present inventors was obtained by co-transfecting 293FT cells (Invitrogen) with HIV-1 backbone plasmid pfNL43-dGPE-EGFP (Addgene) and envelope plasmid VRC8400-Bal26-TSTIP-gp160.

### Acquisition of envelope gene and backbone gene

The backbone plasmid was pfNL43-dGPE-EGFP, purchased from addgene. The plasmid was obtained by modifying NL4-3 infectious clone, and contained gag, pol, tat, rev and other genes necessary for virus packaging, but its Env gene had been partially replaced by the EGFP gene, resulting in the silencing of the Env gene, so that the production of pseudovirus could be performed by co-transfecting only one expression plasmid expressing Env. The expression vector of the envelope gene used in the present invention was VRC8400 which was preserved in our laboratory. The inventor carried out the TSTIP design for the full length of the Env gene of Bal.26 based on the design of BG505 TSTIP protein, and cloned the full-length Bal.26 TSTIP gene (which encoded SEQ ID NO: 16) carrying the virus's own signal peptide between the EcoRV and BglII restriction sites of the VRC8400 vector, to construct a VRC8400-Bal.26-TSTIP-gp160 expression plasmid. The obtained pseudovirus was called BaL.26-SD-FS. In addition, a pseudovirus (BaL.26-WT) obtained based on the wild-type gp160 sequence of the Bal.26 strain was used as a control.

### Production of pseudovirus:

The production of pseudovirus adopted the method of PEI transient transfection, the mixture of pfNL43-dGPE-EGFP plasmid and envelope plasmid of VRC8400-Bal.26 TSTIP gp160 plasmid, and PEI were diluted in 90µl of normal saline respectively, and the two were fully mixed and allowed to stand for 18 minutes to form a plasmid-PEI complex, and co-transfected into 293FT cells at ratios of PEI:plasmid=2:1, pfNL43-dGPE-EGFP:VRC8400 Bal.26 TSTIP gp160=1:1, with the amount of plasmid of 20µg/plate. After 6 hours of transfection, fresh complete DMEM medium was replaced, and culturing was performed at 37°C, 5% CO₂ for 48 hours to obtain a supernatant as a virus liquid.

### Verification of pseudovirus packaging capability

The above-mentioned transfected 293FT cells and cell transfection supernatant were harvested, lysed with cell lysis buffer for 1 hour, the cell lysate supernatant was collected by centrifugation, and subjected to western blot, and the specific steps were as follows:
(1) 20µl of cell lysate and virus liquid supernatant were taken and electrophoresed at 80V on 8% SDS polyacrylamide gel.
(2) after 2 hours, the gel was transferred to a nitrocellulose membrane with two layers of filter paper placed on each of its upside and downside, 1xTrans-Blot^{®} Turbo^{™} Transfer Buffer (SDS) was used as transfer buffer for semi-dry transfer, with the transferring time of 30min.
(3) the membrane was washed with deionized water, and the membrane was blocked with blocking solution 1 (purchased from Xiamen Wantai) for 2h.
(4) the monoclonal antibody 3A7 screened in the laboratory was used as the primary antibody, the primary antibody was diluted in ED11 (purchased from Xiamen Wantai) at a concentration of 1 µg/ml, and incubated for 1 hour at room temperature on a shaker.
(5) the membrane was washed with 1×PBST washing solution for 3 times, 5min for each time, and washed with deionized water once, the secondary antibody GAM-HRP was diluted in ED11 (purchased from Beijing Wantai) at a ratio of 1:5000, and incubated at room temperature for 45 min on a shaker.
(6) the membrane was washed with 1×PBST washing solution for 3 times, 5min for each time, and washed with deionized water once, and then exposed for color development. The results of Western blot are shown in FIG. 8A, in which the Env protein was detected in both cell lysate and virus liquid.

At the same time, the virus liquid was diluted by 5-fold dilution for 6 gradients, and the p24 content in the virus liquid supernatant was detected by ELISA method, and the fitting curve was made according to the reaction of the p24 standard substance in the gradient dilution, and the fitting curve was Y=3618*X +151.3 (FIG. 8B), R² was 0.996, which had a good linear relationship. The quantitative results of p24 were shown in Table 2. At the same time, the samples of the harvested pseudovirus liquid were observed by negative staining electron microscope, and the results were shown in FIG. 9. Based on the above results, the p24 content of the pseudovirus produced by the present invention had decreased to a certain extent, but within an acceptable range, the expression of Env protein could be detected in the supernatant, and obvious pseudoviral particles could be observed under negative staining electron microscope, indicating that the pseudovirus of the present invention could be normally packaged to form pseudoviral particles.

**Table 2: Detection of p24 content**

| Sample | Dilution fold | AOD630-450 | p24 (ng/mL) |
|---|---|---|---|
| BaL.26-SD-FS | 25 | 0.402 | 40.08 |
| BaL.26-WT | 25 | 0.729 | 69.72 |
| Negative control | 1 | 0.008 | 0.18 |

### Verification of pseudovirus infection ability

The harvested virus liquid was diluted in a U-bottom 96-well plate, 100µl of virus liquid was added in the first well, 5-fold dilution was performed for 5 times in sequence, and each well contained 15µg/ml DEAE to promote the infectivity of pseudovirus to cells. TZM-b1 cells were cultured in a 96-well cell culture plate in advance at 37°C, 5% CO₂ until the cell density reached 80%, the diluted pseudovirus liquid was transferred to TZM-b1 cells, and after the cells were infected with the virus for 40-48h, two methods (i.e., chemiluminescence and ELISPOT) were used to detect the infectivity of the produced pseudovirus to the target cells. The results were shown in FIG. 10, and both detection methods showed that the pseudovirus modified by TSTIP design almost completely lost its ability to infect, while the natural pseudovirus could normally infect TZM-b 1 cells.

The verification of the production, packaging and infectivity of the NL4-3TSTIP and NL4-3WT pseudoviral particles were carried out according to the verification methods for the production, packaging and infectivity of the above-mentioned Bal26 pseudovirus.

### Example 6: Identification of pseudoviral particle immunogenicity

### Production and purification of pseudoviral particle

According to the method for producing pseudovirus in Example 5, large scale production of pseudoviral particles (e.g., NL4-3-TSTIP and un-mutated NL4-3-WT pseudoviral particles) was performed by transfection of 293FT cells, and the virus supernatant was harvested and subjected to density gradient centrifugation and purification to obtain pseudoviral particles, and the specific process was as follows:
(1) 20% sucrose solution was prepared in advance and filtered with a 0.22µm syringe filter into a sterile tube;
(2) a set of internal and external tubes and the cap were cleaned and wiped with 75% alcohol, dried by tapping and irradiated with ultraviolet light for 30 minutes in a safety cabinet; the pre-concentrated virus was mixed and centrifuged at 3000 rpm for 7 minutes;
(3) 0.45µm syringe filter and 10ml syringe were used to filter the virus supernatant into the ultracentrifuge internal tube (30ml of virus liquid/tube); a 10ml syringe was used to draw 5ml/tube of 20% sucrose solution and connected with a 5cm needle, and then it was inserted to the bottom of the tube set containing the virus liquid, to add sucrose solution slowly;
(4) the tube set was arranged in pairs 1-4, 2-5, 3-6 correspondingly, and labeled with sample signs. After addition of the samples, balance with electronic analytical balance was performed so that the error between the corresponding two tubes was less than 0.0005g;
(5) the caps of tube sets were tightened after confirming there was no wrong, the tube sets were hung on the corresponding position on sw28 rotor; vacuuming was performed, and the parameters were set as 25000rpm, 2.5h, 4°C, the maximum up speed, and down speed with brakes;
(6) after centrifugation, the vacuum button was pressed to introduce air, the centrifuge tubes were gently taken out, the supernatant was discarded in a safety cabinet, sterile PBS was added to dissolve overnight, and the liquid was collected as a concentrated virus sample.

The obtained HIV-1 virus-like particles were resuspended in PBS, and the p24 and Env of the virus were quantified by the following methods.

### Quantification of p254

1. Antibody coating: 16G12 antibody was diluted with PB7.4, and coated at 100 ng/well (100 µL) 16G12 on a 96-well plate, and the coating was carried out overnight at 4°C.
2. Blocking: the 96-well plate was washed once with PBST, spin-dried, added with 180 µL of ED solution, and blocked for 2 h at 37°C or overnight at 4°C.
3. Virus incubation: PBS was used as buffer solution to serially dilute the virus in a U bottom plate (p24 standard substance was added to the plate at 350, 700, 1400 and 2800pg/mL), 100 µL (volume) per well, then added with lysis buffer, 20µl (volume) per well. It was transferred to a 96-well plate and incubated at 37 °C for 1 h.
4. Secondary antibody reaction: the 96-well plate was washed 5 times with PBST washing solution, spin-dried. 100 µL of 2F2-HRP secondary antibody solution (1: 10000 diluted in ED11 solution) was added to each well, and incubated in a 37°C incubator for 45 min.
5. Color development: the 96-well plate was washed 5 times with PBST washing solution, spin-dried, and 100 µL of the A/B color development solutions mixed at equal volume was added to each well, and incubated at 37°C for 10 min.
6. Stopping: 50 µL of sulfuric acid stopping solution was added to each well, the 96-well plate was placed in a microplate reader to read OD650-450nm.
7. The p24 standard substance was used as a reference to make a standard curve to calculate the p24 content in the virus.

### Quantification of Env

1. GNL coating: GNL at 500ng/well (100µl) was coated on a 96-well plate, the buffer was PBS, and the coating was performed overnight at 4°C.
2. Blocking: the plate was washed once with PBST, spin-dried, added with 180 µL of ED solution, and blocked for 2 hours at 37°C or overnight at 4°C.
3. Virus coating: the virus liquid was diluted with ED as diluent solution (gp140 at a starting concentration of 1 µg/ml was used as the standard), transferred into the 96-well plate, and incubated at 37°C for 1 hour after sealing the plate.
4. Primary antibody reaction: the plate was washed 5 times with PBST washing solution, and spin-dried. 100 µl of VRC01 (1µg/ml diluted in ED11 solution) was added to each well, and incubated at 37 °C for 1 hour after sealing the plate.
4. Secondary antibody reaction: the plate was washed 5 times with PBST washing solution, and spin-dried. 100 µL of GAH-HRP secondary antibody solution (1:5000 diluted in ED11 solution) was added to each well, and incubated at 37°C for 45 min after sealing the plate.
5. Color development: the 96-well plate was washed 5 times with PBST washing solution, and spin-dried. 100 µL of the A/B color development solutions mixed at equal volume was added to each well, and incubated to react in a 37°C incubator for 10 min.
6. Stopping: 50 µL of sulfuric acid stopping solution was added to each well, the 96-well plate was place in a microplate reader to read OD650-450nm.
7. The gp140 standard substance was used as a reference to make a standard curve to calculate the Env content in the virus.

### Identification of pseudovirus immunogenicity

After quantification of p24 and Env of the NL4-3 pseudoviral particles, the mouse immunization experiment of the NL4-3 pseudoviral particles were performed with the immunization protocol shown in Table 3. The 6-week-old female mice, purchased from Shanghai Slack Experimental Animal Co., Ltd., were divided into five experimental groups, A/B/C/D/E, 5 mice in each group. Combined with aluminum adjuvant, a total of five injections of intraperitoneal immunization were performed, and the immunization period was 2 weeks/injection. The blood samples were collected from mouse eyeball before immunization at 0/2/4/6/8 weeks and at the 10^{th} week, and then the mice were treated with neck dislocation. The blood samples were placed at 37°C for 30 minutes, then centrifuged at 13300 rpm for 10 minutes, and the serum samples were collected for determination of Env and P24 specific binding antibody titers and HIV-1 pseudovirus neutralizing antibody titers. The experimental results showed that the pseudovirus of the present invention could induce neutralizing antibody responses and exhibited good immunogenicity.

**Table 3: Immunization protocol of NL4-3 pseudoviral particle**

| Injection No. | Group A | Group B | Group C | Group D | Group E |
|---|---|---|---|---|---|
| 1 | NL4-3TSTIP pseudovirus 2µg(Env) | NL4-3 WT pseudovirus 2µg(Env) | NL4-3TSTIP gp140(2µg) | NL4-3SOSIP gp140(2µg) | NL4-3TSTIP pseudovirus 2µg(Env)+NL4-3TSTIP gp140(2µg) |
| 2 | NL4-3TSTIP pseudovirus 2µg(Env) | NL4-3 WT pseudovirus 2µg(Env) | NL4-3TSTIP gp140(2µg) | NL4-3SOSIP gp140(2µg) | NL4-3TSTIP pseudovirus 2µg(Env)+NL4-3TSTIP gp140(2µg) |
| 3 | NL4-3TSTIP pseudovirus 2µg(Env) | NL4-3 WT pseudovirus 2µg(Env) | NL4-3TSTIP gp140(2µg) | NL4-3SOSIP gp140(2µg) | NL4-3TSTIP pseudovirus 2µg(Env)+NL4-3TSTIP gp140(2µg) |
| 4 | NL4-3TSTIP pseudovirus 2µg(Env) | NL4-3 WT pseudovirus 2µg(Env) | NL4-3TSTIP gp140(2µg) | NL4-3SOSIP gp140(2µg) | NL4-3TSTIP pseudovirus 2µg(Env)+NL4-3TSTIP gp140(2µg) |
| 5 | NL4-3TSTIP pseudovirus 2µg(Env) | NL4-3 WT pseudovirus 2µg(Env) | NL4-3TSTIP gp140(2µg) | NL4-3SOSIP gp140(2µg) | NL4-3TSTIP pseudovirus 2µg(Env)+NL4-3TSTIP gp140(2µg) |

### Example 7: Production and characterization of NL4-3 virus particles

The full-length Env sequence of NL4-3 strain was designed with reference to the design of TSTIP protein, and the obtained full-length TSTIP gp160 gene (which encoded the amino acid sequence set forth in SEQ ID NO: 17) was codon-optimized to obtain base sequence suitable for expression in mammalian cells, and then sent to Sangon Biotech (Shanghai) Co., Ltd for gene synthesis, and cloned into the genomes of two strains to replace the wild-type Env gene. 1 µl of each of the synthesized plasmids was taken to transform Stbl3 competent cell (purchased from Shanghai Weidi Biotechnology Co., Ltd.), then coated on an ampicillin-containing solid medium, and cultured at 30°C for 12-14 hours; after a single colony was clearly visible, it was picked and placed in a test tube containing 3ml of ampicillin-containing LB medium, cultured under shaking at 220 rpm at 30°C for 12 hours; 500µl of the bacterial liquid was taken and mixed with 500µl of 50% glycerol, and then cryopreserved at -20°C. The bacterial liquid was inoculated into 500ml of LB medium and cultured, for plasmid extraction. The extraction process of plasmid referred to the extraction process of pcDNA3.1 NL4-3/BG505 TSTIP plasmid in Example 1. The extracted plasmid was transiently transfected into 293FT adherent cells, with the transfection reagent of PEI, and by using the transfection method referring to the pseudovirus production process as mentioned in the production and identification of Bal.26-TSTIP pseudoviral particle in Example 5. After 48 hours of transfection, the transfection supernatant was collected, the NL4-3 TSTIP virus particles were purified, and the collected virus particles were verified for their packaging ability and infectivity, and the specific methods referred to the methods described in Example 5. The experimental results showed that the virus obtained by the above method could be normally packaged to form virus particles, and the virus modified by TSTIP design almost completely lost its ability to infect.

### Example 8: Identification of immunogenicity of virus particles

According to the method of Example 7, a sufficient amount of virus particles was obtained, and detected for the p24 and Env content by using the p24 and Env quantification method in Example 6. NL4-3 TSTIP virus particle immunization experiment was carried out according to the immunization protocol shown in Table 4. Six-week-old female white mice were purchased from Shanghai Slack Experimental Animal Co., Ltd., and two experimental groups A/B were set up, five mice in each group. Taking Env content as a reference, the group A was immunized with virus particles containing 2µg-Env, and the group B was immunized with 2µg of purified gp140 protein. Combined with aluminum adjuvant, intraperitoneal immunization was carried out, the immunization period was 2 weeks/injection, and there was a total of 5 injections for the immunization. The blood samples of mice were collected from eyeball before the immunization at 0/2/4/6/8 weeks and at the 10^{th} week, and then the mice were treated with neck dislocation. After the blood samples were placed at 37°C for 30 minutes, they were centrifuged at 13300 rpm for 10 minutes, and the serum samples were collected for the determination of Env and P24-specific binding antibody titers and HIV-1 pseudovirus neutralizing antibody titers. The experimental results showed that the virus of the present invention could induce neutralizing antibody responses and exhibited good immunogenicity.

**Table 4: Immunization protocol of NL4-3 virus particle**

| Injection No. | Group A | Group B |
|---|---|---|
| 1 | NL4-3TSTIP pseudovirus 2µg(Env) | NL4-3TSTIP gp140(2µg) |
| 2 | NL4-3TSTIP pseudovirus 2µg(Env) | NL4-3TSTIP gp140(2µg) |
| 3 | NL4-3TSTIP pseudovirus 2µg(Env) | NL4-3TSTIP gp140(2µg) |
| 4 | NL4-3TSTIP pseudovirus 2µg(Env) | NL4-3TSTIP gp140(2µg) |
| 5 | NL4-3TSTIP pseudovirus 2µg(Env) | NL4-3TSTIP gp140(2µg) |

### Example 9: Design and characterization of various TSTIP-based proteins

Based on the amino acid sequence of BG505 TSTIP (SEQ ID NO: 3), WNSSWSN and AKRRVVGREKR, which needed to be deleted due to the design of TSTIP, were introduced at the linkage region of β27 and gp120 and the linkage region of gp120 and α8 of BG505 TSTIP respectively to obtain SEQ ID NO: 5 (BGTSTIP-Full).

Based on the amino acid sequence of NL4-3TSTIP (SEQ ID NO: 4), WNSSWSN and AKRRVVGREKR, which needed to be deleted due to the design of TSTIP, were introduced at the linkage region of β27 and gp120 and the linkage region of gp120 and α8 of NL4-3TSTIP respectively to obtain SEQ ID NO: 6 (NL4-3TSTIP Full).

GGGGS (SEQ ID NO: 28) was introduced at the linkage region of β27 and gp120 and the linkage region of gp120 and α8 of BG505 TSTIP, respectively, to obtain SEQ ID NO: 7 (BG505-TSTIP G1).

GGGGS was introduced at the linkage region of β27 and gp120 and the linkage region of gp120 and α8 of NL4-3TSTIP, respectively, to obtain SEQ ID NO: 8 (NL4-3-TSTIP G1).

GGGGSGGGGS (SEQ ID NO: 29) was introduced at the linkage region of β27 and gp120 and the linkage region of gp120 and α8 of BG505 TSTIP, respectively, to obtain SEQ ID NO: 9 (BG505-TSTIP G2).

GGGGSGGGGS was introduced at the linkage region of β27 and gp120 and the linkage region of gp120 and α8 of NL4-3TSTIP, respectively, to obtain SEQ ID NO: 10 (NL4-3-TSTIP G2).

According to the steps of Examples 1, 2, 3, and 4, a series of studies were carried out on the six proteins obtained above. The results of SDS-polyacrylamide gel electrophoresis of the six proteins were shown in FIG. 11A, in which the purity of BGTSTIP-Full, NL4-3TSTIP Full and 8NL4-3-TSTIP G1 proteins decreased in some extent after one-step Ni column purification; the Coomassie brilliant blue staining of BGTSTIP-G1 and NL4 -3 TSTIP G2 under reducing conditions showed >180KD bands, which might be the dimer forms thereof, while BG505-TSTIP G2 showed similar purity and molecular weight to BG505 TSTIP.

The four high-purity proteins were subjected to analytical ultracentrifugation, and the results were shown in FIG. 11B, in which this result was consistent with the results of SDS polyacrylamide gel electrophoresis in the previous step (14A), that was, the molecular weights of BGTSTIP G1 and NL4-3TSTIP G2 were greater than the molecular weight of monomer gp140, while the purity of BG505 TSTIP was significantly lower than that of BGTSTIP. This showed that the linkage region of β27 and gp120, and the linkage region of gp120 and α8 had obvious effects on the forms of Env protein. Moreover, different modifications of the same strain could result in differences of expression level, and the mutation of Ile→Pro at position 559 could effectively increase the protein production. Specifically, the expression levels were summarized in Table 3.

The ELISA experiment results of the six proteins with various reported human monoclonal antibodies were shown in FIG. 12. Even some proteins had a relatively lower purity, the purified six proteins could all be well recognized by various antibodies, which proved their activity.

According to the above immunization protocol, we immunized BABL/C mice with the six purified proteins. The results of animal immunization experiments showed that all the six proteins could induce neutralizing antibody responses in mice, as shown in FIG. 13. In addition, pseudoviral particles and virus particles were further constructed based on these proteins, and mouse immunization experiments were carried out to detect their immunogenicity.

**Table 3: Summary of protein expression levels**

| BG505 Env | BGTSTIP | BGSOSIP | BGTSTIP-G1 | BGTSTIP-G2 | BGTSTIP-full |
|---|---|---|---|---|---|
| Expression level (mg/L) | 40 | 20 | 9 | 27 | 6 |
| NL4-3 Env | 4-3TSTIP | 4-3SOSIP | 4-3TSTIP-G1 | 4-3TSTIP-G2 | 4-3TSTIP-full |
| Expression level (mg/L) | 37 | 19 | 3 | 25 | 5 |

### Example 10: Application of TSTIP modification in strains of multiple subtypes

In order to further confirm the effect of the TSTIP design on different strains, the inventors applied a design method similar to BG505/NL4-3-TSTIP to the Env proteins of various strains.

Table 4 showed 12 global-representative strains. Since the expression and identification of proteins of subtype A and subtype B strains had been completed, one strain of each subtype except subtype A and subtype B was selected from the 12 global pseudovirus strains, to carry out the TSTIP modification of Env protein in the gp140 proteins of the selected 5 strains, including 25710 (C), X1632 (G), CH119 (BC), CNE8 (AE), and 246F3 (AC). According to a series of experimental steps and methods such as cloning, expression and purification, and characterization as described above, the TSTIP-gp140 proteins of the five strains were studied, and the series of results were shown in FIGS. 14A to 14D.

The results of SDS polyacrylamide gel electrophoresis in FIG. 14A showed that the TSTIP proteins of the five strains all had a relatively higher purity after one-step Ni column purification, and a molecular weight of about 140KD under the reducing SDS PAGE, indicating that the subunits in the proteins of the present invention were linked together by stable covalent interaction as expected.

FIG. 14B showed the superdex200 16/600 molecular sieve chromatography results of the five TSTIP proteins, which showed that the elution peaks were relatively single, indicating that the components of the five proteins of the present invention were relatively uniform in natural state, with trimers as majority.

FIG. 14C showed the enzyme-linked immunoassay (ELISA) results of the five proteins with various reported antibodies, which showed that the proteins of different strains had different recognition and binding abilities to the antibody of the same type. The results showed that the TSTIP proteins of the five strains produced by the inventors all had good activity.

FIG. 14D showed the neutralization experiment of serum samples after immunizing BALB/C mice with the five proteins, and the results indicated that the immunization with TSTIP proteins of different strains could stimulate mice to produce a neutralizing response.

**Table 4: Twelve global pseudoviruses**

| Isolate strain | Subtype | Region |
|---|---|---|
| TRO11 | B | Italy |
| 25710 | C | India |
| 398F1 | A | Tanzania |
| CNE8 | CRF01_AE | China |
| X2278 | B | Spain |
| BJOX002000 | CRF07_BC | China |
| X1632 | G | Spain |
| CE1176 | C | Malawi |
| 246F3 | AC | Tanzania |
| CH119 | CRF07_BC | China |
| CE0217 | C | Malawi |
| CNE55 | CRF01_AE | China |

**Table 5: Summary of TSTIP protein expression levels of five subtypes**

| | | | | | |
|---|---|---|---|---|---|
| TSTIP | 246F3 | 25710 | CH119 | CNE8 | X1632 |
| Expression level (mg/L) | 14.7 | 24 | 60 | 27 | 36 |

### Example 11: Design and activity identification of other TSTIP-based proteins

### 11.1 Protein design

### 1. BG-B1(1/1)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of GGGGS after it, and WNSSWSN was added back before α8 with further insertion of GGGGS before it. The modified sequence based on the BG505 strain was set forth in SEQ ID NO:30.

### 2. BG-B1(1/2)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of GGGGS after it, and WNSSWSN was added back before α8 with further insertion of (GGGGS)₂ before it. The modified sequence based on the BG505 strain was set forth in SEQ ID NO:31.

### 3. BG-B1(2/2)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of (GGGGS)₂ after it, and WNSSWSN was added back before α8 with further insertion of (GGGGS)₂ before it. The modified sequence based on the BG505 strain was set forth in SEQ ID NO:32.

### 4. BG-B1(2/1)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of (GGGGS)₂ after it, and WNSSWSN was added back before α8 with further insertion of GGGGS before it. The modified sequence based on the BG505 strain was set forth in SEQ ID NO:33.

### 5. BG-B1(2/3)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of (GGGGS)₂ after it, and WNSSWSN was added back before α8 with further insertion of (GGGGS)₃ before it. The modified sequence based on the BG505 strain was set forth in SEQ ID NO:34.

### 6. BG-B2(1-1)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of GGGGS after it, and WNSSWSN was added back before α8 with further insertion of GGGGS before it; in addition, a cys mutation was introduced between gp120 and gp41 subunits by referring to the design of SOSIP in order to expect the formation of a disulfide bond, which specifically comprised that Ala at position 501 was mutated to cys, and Thr at position 605 was mutated to cys. The modified sequence based on the BG505 strain was set forth in SEQ ID NO:35.

### 7. BG-B2(1-2)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of GGGGS after it, and WNSSWSN was added back before α8 with further insertion of (GGGGS)₂ before it; in addition, a cys mutation was introduced between gp120 and gp41 subunits by referring to the design of SOSIP in order to expect the formation of a disulfide bond, which specifically comprised that Ala at position 501 was mutated to cys, and Thr at position 605 was mutated to cys. The modified sequence based on the BG505 strain was set forth in SEQ ID NO:36.

### 8. BG-B2(1-3)

The amino acid sequence AKRRVVG (not including the furin cleavage site) deleted after β26 and the sequence WNSSWSN (or homologous sequence) deleted after β27 were added back, that was, AKRRVVG was added back after β26 with further insertion of GGGGS after it, and WNSSWSN was added back before α8 with further insertion of (GGGGS)₃ before it; in addition, a cys mutation was introduced between gp120 and gp41 subunits by referring to the design of SOSIP in order to expect the formation of a disulfide bond, which specifically comprised that Ala at position 501 was mutated to cys, and Thr at position 605 was mutated to cys. The modified sequence based on the BG505 strain was set forth in SEQ ID NO:37.

### 9. BG-C1(1/1)

The amino acid sequence AKRRVVG (or its homologous sequence, not including the furin cleavage site) deleted after β26 and the sequence WNSSWSN (or homologous sequence) deleted after β27 were added back, that was, WNSSWSN was added back after β27 with further insertion of GGGGS after it, and AKRRVVG was added back before α8 with further insertion of GGGGS after it. The modified sequence based on the BG505 strain was set forth in SEQ ID NO:38.

### 10. BG-C1(1/2)

The amino acid sequence AKRRVVG (or its homologous sequence, not including the furin cleavage site) deleted after β26 and the sequence WNSSWSN (or homologous sequence) deleted after β27 were added back, that was, WNSSWSN was added back after β27 with further insertion of GGGGS after it, and AKRRVVG was added back before α8 with further insertion of (GGGGS)₂ after it. The modified sequence based on the BG505 strain was set forth in SEQ ID NO:39.

### 11. BG-C1(1/3)

The amino acid sequence AKRRVVG (or its homologous sequence, not including the furin cleavage site) deleted after β26 and the sequence WNSSWSN (or homologous sequence) deleted after β27 were added back, that was, WNSSWSN was added back after β27 with further insertion of GGGGS after it, and AKRRVVG was added back before α8 with further insertion of (GGGGS)₃ after it. The modified sequence based on the BG505 strain was set forth in SEQ ID NO:40.

### 11.2 SDS-PAGE:

These proteins were prepared and purified using the methods described in the above Examples 1-2, the concentrated samples were diluted to 1µg/µl, then two tubes of 50µl samples were taken, and 10µl of reducing loading buffer and 10µl of non-reducing loading buffer were added, respectively, to prepare reduced samples and non-reduced samples, and the reduced samples were placed in a boiling water bath at 100°C for 10 minutes. The reduced samples and non-reduced samples in an amount of 10µl were taken and electrophoresed at 80V for 120min in 8% SDS PAGE, and the electrophoresis bands were displayed after Coomassie brilliant blue staining.

The results of electrophoresis were shown in the panel (I) of FIGS. 15A to 15K. The SDS-PAGE analysis showed that after one-step Ni-EXCEL purification, the above-mentioned designs could obtain high-purity proteins; under the condition of adding reducing loading buffer, except for BG-C1(1/1), the main bands showed by other optimized proteins were all 140KD, while bands of multimer were showed under the condition of non-reducing loading buffer. This indicated that in the optimized designed proteins, similar to the original TSTIP protein, the gp120 and gp41 extracellular regions were still connected via a stable covalent linkage.

### 11.3 Molecular sieve purification:

Instrument system: AKTA Pure type preparative liquid chromatograph;
Chromatographic column: superdex 200 10/300
Column volume: 24ml
Buffer: PBS (20mM phosphate buffer, pH7.5, 150mM NaCl)
Detector wavelength: 280nm
Flow rate: 0.5ml/min

The samples were purified and concentrated proteins.

The purification procedure comprised: the superdex200 10/300 was equilibrated with 1 column volume of PBS, a target protein to be purified was loaded with 500µl sample loop, and molecular sieve purification of the sample to be purified was performed at inject mode. The components of the sample would be eluted according to molecular weights thereof in order, from high to low, and a diagram of peaks after purification was saved. The molecular sieve purification results were shown in the panel (II) of FIGS. 15A to 15K. Each of the proteins showed an obvious single elution peak on the molecular sieve spectrum. Among them, BG-B1(1/1), BG-B1(1/2), BG-B1(2-1), BG-B1(2-2), BG-B1(2-3), BG-B2(1-1), BG-B2(1-2), BG-B2(1-3), BG-C1(1/2), BG-B 1(1/3) all had main elution peaks at an elution volume of about 9.5ml, which were peaks of trimer, while BG-C1 (1/1) had an elution volume of 14ml showing a peak of dimer.

### 11.4 Homogeneity analysis and molecular weight prediction of proteins after analytical ultracentrifugation (AUC)

The above-mentioned purified proteins were subjected to analytical ultracentrifugation, and the AUC results were shown in the panel (III) of FIGS. 15A to 15K. The results showed that BG-B1(1/1), BG-B1(1/2), BG-B1(2-1), BG-B1(2-2), BG-B1(2-3), BG-B2(1-1), BG-B2(1-2), BG-B2(1-3), BG-C1(1/2), BG-B1(1/3) were mainly trimers, while BG- C1(1/1) was mainly dimers, and this result was consistent with result of molecular sieve.

### 11.5 Enzyme-linked immunosorbent assay (ELISA)

Using the enzyme-linked immunosorbent assay described in Example 4, the antigenicity of these above-mentioned purified proteins was identified. The results were shown in the panel (IV) of FIGS. 15A to 15K. The results showed that these proteins had antigenicity, and the modification of linker at the linkage region could adjust their antigenic activity.

Although the specific embodiments of the present invention have been described in detail, those skilled in the art will understand that: according to all the teachings that have been disclosed, various modifications and changes can be made to the details, and these changes are all within the protection scope of the present invention. The full scope of the present invention is given by the claims appended hereto and any equivalents thereof.

## Claims

1. A recombinant protein, which comprises gp120 and gp41 ectodomain (gp41ECTO), wherein the gp120 is located between β27 and α8 of the gp41ECTO;
preferably, the recombinant protein comprises: α6, α7, β27 of gp41ECTO; gp120; α8, α9 of gp41ECTO, from its N-terminal to C-terminal.

2. The recombinant protein according to claim 1, wherein the gp41ECTO comprises a substitution of one or more (e.g., 1-12, 5-12, 5-10; e.g., 1, 2, 3, 4, 5, 6, 7, 8, 9, 10, 11, or 12) consecutive amino acids in a linkage region between β27 and α8with gp120;
preferably, the linkage region corresponds to amino acid positions 607-618 of a gp160 sequence of isolate HXB2.

3. The recombinant protein according to claim 2, wherein the gp41ECTO comprises a substitution of one or more (e.g., 1-7, 5-7; e.g., 1, 2, 3, 4, 5, 6, or 7) consecutive amino acids in a region corresponding to amino acid positions 610-616 of the gp160 sequence of isolate HXB2 with gp120;
preferably, the gp120 is located between amino acid positions corresponding to positions 606 and 619 of the gp 160 sequence of isolate HXB2.

4. The recombinant protein according to claim 1, wherein the gp120 is inserted between adjacent amino acids in a linkage region between β27 and α8 of gp41ECTO;
preferably, the linkage region corresponds to amino acid positions 607-618 of a gp160 sequence of isolate HXB2;
preferably, the gp120 is located between amino acid positions corresponding to positions 609 and 610 of the gp 160 sequence of isolate HXB2;
preferably, the gp120 is located between amino acid positions corresponding to positions 616 and 617 of the gp 160 sequence of isolate HXB2.

5. The recombinant protein according to any one of claims 1-4, wherein the gp120 is a modified gp120 that has a furin cleavage site containing a mutation to prevent being cleaved compared to a natural gp120;
preferably, the mutation is selected from amino acid substitution, insertion or deletion;
preferably, the furin cleavage site corresponds to amino acid positions 508-511 of a gp160 sequence of isolate HXB2;
preferably, the furin cleavage site is deleted in the modified gp120 compared to the natural gp 120.

6. The recombinant protein according to any one of claims 1-4, wherein the gp120 is a modified gp120, that has C-terminal truncation of 1-11 (e.g., 4-11; e.g., 1, 2, 3, 4, 5, 6, 7, 8, 9, 10, or 11) amino acids compared with a natural gp120;
preferably, the modified gp120 has a deletion of one or more (e.g., 1-11, 4-11; e.g.,1, 2, 3, 4, 5, 6, 7, 8, 9, 10, or 11) consecutive amino acids in a region corresponding to amino acid positions 501-511 of a gp160 sequence of isolate HXB2;
preferably, the modified gp120 has a deletion of the region corresponding to amino acid positions 501-511 of the gp160 sequence of isolate HXB2.

7. The recombinant protein according to any one of claims 1-6, wherein, a disulfide bond is contained between gp120 and gp41ECTO of the recombinant protein;
preferably, the recombinant protein comprises a disulfide bond between amino acid positions corresponding to positions 37 and 605 of a gp 160 sequence of isolate HXB2;
preferably, the recombinant protein comprises residue Cys at amino acid positions corresponding to positions 37 and 605 of the gp160 sequence of isolate HXB2.

8. The recombinant protein according to any one of claims 1-4, wherein the gp 120 is a natural gp 120.

9. The recombinant protein according to any one of claims 1-8, wherein the N-terminal and/or C-terminal of the gp120 is linked to the gp41ECTO optionally via a peptide linker;
preferably, the peptide linker is (GmS)n, wherein m is an integer selected from 1-4, and n is an integer selected from 1-3.

10. The recombinant protein according to any one of claims 1-9, which further possesses one or more of the following features:
(1) the gp41ECTO comprises the following amino acid substitution: I559P;
(2) the gp120 comprises the following amino acid substitution: T332N;
(3) the gp120 comprises the following amino acid substitutions: E64K and H66R;
(4) the gp120 comprises the following amino acid substitution: A316W;
(5) the N-linked glycosylation site (PNGS) near the CD4bs epitope of the gp120 is replaced to prevent glycosylation; preferably, the PNGS is selected from the group consisting of N276, N301, N360, N463;
(6) the gp120 comprises an internal disulfide bond; preferably, the gp120 comprises an internal disulfide bond between I201C and A433C;
(7) a disulfide bond is further contained between the gp120 and gp41ECTO; for example, the recombinant protein comprises a disulfide bond between E49C and L555C;
the numbering of the above positions is according to the numbering in gp160 of HIV-1 isolate HXB2.

11. The recombinant protein according to any one of claims 1-10, wherein the gp41ECTO and gp120 are derived from the same or different HIV-1 strains;
preferably, the gp41ECTO and gp120 are derived from the same HIV-1 strain.

12. The recombinant protein according to any one of claims 1-11, comprising an amino acid sequence selected from:
(1) an amino acid sequence consisting of amino acid residues at positions 40 to 651 of the sequence set forth in SEQ ID NO: 1;
(2) an amino acid sequence consisting of amino acid residues at positions 40 to 652 of the sequence set forth in SEQ ID NO:2;
(3) an amino acid sequence consisting of amino acid residues at positions 40 to 651 of the sequence set forth in SEQ ID NO:3;
(4) an amino acid sequence consisting of amino acid residues at positions 40 to 652 of the sequence set forth in SEQ ID NO:4;
(5) an amino acid sequence consisting of amino acid residues at positions 40 to 665 of the sequence set forth in SEQ ID NO:5;
(6) an amino acid sequence consisting of amino acid residues at positions 40 to 678 of the sequence set forth in SEQ ID NO:6;
(7) an amino acid sequence consisting of amino acid residues at positions 40 to 661 of the sequence set forth in SEQ ID NO:7;
(8) an amino acid sequence consisting of amino acid residues at positions 40 to 666 of the sequence set forth in SEQ ID NO:8;
(9) an amino acid sequence consisting of amino acid residues at positions 40 to 671 of the sequence set forth in SEQ ID NO:9;
(10) an amino acid sequence consisting of amino acid residues at positions 40 to 676 of the sequence set forth in SEQ ID NO:10;
(11) an amino acid sequence consisting of amino acid residues at positions 36 to 607 of the sequence set forth in SEQ ID NO:11;
(12) an amino acid sequence consisting of amino acid residues at positions 36 to 646 of the sequence set forth in SEQ ID NO:12;
(13) an amino acid sequence consisting of amino acid residues at positions 36 to 648 of the sequence set forth in SEQ ID NO:13;
(14) an amino acid sequence consisting of amino acid residues at positions 36 to 638 of the sequence set forth in SEQ ID NO:14;
(15) an amino acid sequence consisting of amino acid residues at positions 36 to 639 of the sequence set forth in SEQ ID NO:15;
(16) an amino acid sequence consisting of amino acid residues at positions 36 to 836 of the sequence set forth in SEQ ID NO:16;
(17) an amino acid sequence consisting of amino acid residues at positions 36 to 673 of the sequence set forth in SEQ ID NO:30;
(18) an amino acid sequence consisting of amino acid residues at positions 36 to 678 of the sequence set forth in SEQ ID NO:31;
(19) an amino acid sequence consisting of amino acid residues at positions 36 to 683 of the sequence set forth in SEQ ID NO:32;
(20) an amino acid sequence consisting of amino acid residues at positions 36 to 678 of the sequence set forth in SEQ ID NO:33;
(21) an amino acid sequence consisting of amino acid residues at positions 36 to 688 of the sequence set forth in SEQ ID NO:34;
(22) an amino acid sequence consisting of amino acid residues at positions 36 to 670 of the sequence set forth in SEQ ID NO:35;
(23) an amino acid sequence consisting of amino acid residues at positions 36 to 676 of the sequence set forth in SEQ ID NO:36;
(24) an amino acid sequence consisting of amino acid residues at positions 36 to 680 of the sequence set forth in SEQ ID NO:37;
(25) an amino acid sequence consisting of amino acid residues at positions 36 to 673 of the sequence set forth in SEQ ID NO:38;
(26) an amino acid sequence consisting of amino acid residues at positions 36 to 678 of the sequence set forth in SEQ ID NO:39;
(27) an amino acid sequence consisting of amino acid residues at positions 36 to 683 of the sequence set forth in SEQ ID NO:40; or
(28) a variant of the sequence described in any one of (1) to (27), in which the variant has a substitution, deletion or addition of one or several amino acids (e.g., a substitution, deletion or addition of 1, 2, 3, 4, 5, 6, 7, 8, 9 or 10 amino acids) or has a sequence identity of at least 80%, at least 85%, at least 90%, at least 91%, at least 92%, at least 93%, at least 94%, at least 95%, at least 96%, at least 97%, at least 98%, or at least 99% compared to the sequence from which it is derived, and the variant retains the properties of the sequence from which it is derived.

13. The recombinant protein according to any one of claims 1-12, which optionally comprises one or more sequences selected from the following at its N-terminal or C-terminal: a signal peptide, a translation initiation sequence (e.g., a Kozak consensus sequence), a tag sequence;
preferably, the recombinant protein optionally comprises a signal peptide and/or a translation initiation sequence (e.g., a Kozak consensus sequence) at its N-terminal;
preferably, the recombinant protein optionally comprises a tag sequence at its C-terminal.

14. A fusion protein, which comprises the recombinant protein according to any one of claim 1-13, and transmembrane region and intracellular region sequences of gp41 linked to its C-terminal;
preferably, the transmembrane region and intracellular region sequences of gp41 and the gp41ECTO in the recombinant protein are derived from the same HIV-1 strain.

15. The fusion protein according to claim 14, comprising an amino acid sequence selected from:
(1) an amino acid sequence consisting of amino acid residues at positions 33 to 836 of the sequence set forth in SEQ ID NO: 16;
(2) an amino acid sequence consisting of amino acid residues at positions 34 to 837 of the sequence set forth in SEQ ID NO: 17;
(3) a variant of the sequence described in any one of (1) to (2), in which the variant has a substitution, deletion or addition of one or several amino acids (e.g., a substitution, deletion or addition of 1, 2, 3, 4, 5, 6, 7, 8, 9 or 10 amino acids) or has a sequence identity of at least 80%, at least 85%, at least 90%, at least 91%, at least 92%, at least 93%, at least 94%, at least 95%, at least 96%, at least 97%, at least 98%, or at least 99% compared to the sequence from which it is derived, and the variant retains the properties of the sequence from which it is derived.

16. A multimer comprising a plurality of monomers, wherein each monomer is independently selected from the recombinant protein according to any one of claims 1-13, or independently selected from the fusion protein according to in claim 14 or 15;
preferably, the monomers are identical to each other;
preferably, the multimer is a trimer or a dimer.

17. An isolated nucleic acid molecule, which comprises a nucleotide sequence encoding the recombinant protein according to any one of claims 1-13, the fusion protein according to claim 14 or 15, or the multimer according to claim 16.

18. A vector, which comprises the isolated nucleic acid molecule according to claim 17.

19. A host cell, which comprises the isolated nucleic acid molecule according to claim 17 or the vector according to claim 18;
preferably, the host cell is a mammalian cell.

20. A method for preparing the recombinant protein according to any one of claims 1-13, the fusion protein according to claim 14 or 15 or the multimer according to claim 16, which comprises cultivating the host cell according to claim 19 under appropriate conditions, and recovering the recombinant protein, the fusion protein or the multimer from a cell culture;
preferably, the recombinant protein or the fusion protein exists in the form of a multimer (e.g., trimer or dimer).

21. A particle, displaying on its surface the recombinant protein according to any one of claims 1-13, the fusion protein according to claim 14 or 15, or the multimer according to claim 16;
preferably, the particle is a liposome or a nanoparticle.

22. A pseudoviral particle, comprising on its surface the recombinant protein according to any one of claims 1-13, the fusion protein according to claim 14 or 15, or the multimer according to claim 16;
preferably, the pseudoviral particle is obtained by co-expressing (i) a vector comprising the nucleic acid molecule according to claim 17, and (ii) a packaging vector (e.g., a backbone plasmid) in a host cell.

23. A packaging system for producing the pseudoviral particle according to claim 22, which comprises: (i) an expression vector comprising the nucleic acid molecule according to claim 17, and (ii) a packaging vector (e.g., a backbone plasmid).

24. A modified HIV virus, which expresses the fusion protein according to claim 14 or 15 as its envelope protein;
preferably, the modified HIV virus has a genome comprising the following modifications: substitution of wild-type env gene with a nucleotide sequence encoding the fusion protein according to claim 14 or 15;
preferably, the HIV is HIV-1.

25. An isolated nucleic acid molecule, which comprises a nucleotide sequence encoding the genome of the modified HIV virus according to claim 24.

26. A vector, which comprises the isolated nucleic acid molecule according to claim 25.

27. A composition, which comprises the recombinant protein according to any one of claim 1-13, the fusion protein according to claim 14 or 15 or the multimer according to claim 16, the isolated nucleic acid molecule according to claim 17, the vector according to claim 18, the host cell according to claim 19, the particle according to claim 21, the pseudoviral particle according to claim 22, the packaging system according to claim 23, the modified HIV virus according to claim 24, the isolated nucleic acid molecule according to claim 25, or the vector according to claim 26;
preferably, the composition further comprises a pharmaceutically acceptable carrier and/or excipient.

28. The composition according to claim 27, wherein the composition is an immunogenic composition or a vaccine;
preferably, the composition comprises an adjuvant.

29. The composition according to claim 28, wherein the composition is a protein vaccine, which comprises the recombinant protein according to any one of claims 1-13, the fusion protein according to claim 14 or 15, or the multimer according to claim 16, or the particle according to claim 21.

30. The composition according to claim 28, wherein the composition is a virus-based vaccine, which comprises the pseudoviral particle according to claim 22 or the modified HIV virus according to claim 23.

31. The composition according to claim 28, wherein the composition is a nucleic acid vaccine, which comprises the isolated nucleic acid molecule according to claim 17, the vector according to claim 18, the isolated nucleic acid molecule according to claim 25, or the vector according to claim 26;
preferably, the nucleic acid vaccine comprises DNA or RNA;
preferably, the DNA or RNA may be naked or encapsulated in a shell with delivery and/or protection functions.

32. The composition according to any one of claims 27-31, which optionally comprises an antiretroviral agent, such as a nucleoside reverse transcriptase inhibitor, a non-nucleoside reverse transcriptase inhibitor, a protease inhibitor, or a fusion protein inhibitor.

33. Use of the recombinant protein according to any one of claims 1-13, the fusion protein according to claim 14 or 15 or the multimer according to claim 16, the isolated nucleic acid molecule according to claim 17, the vector according to claim 18, the host cell according to claim 19, the particle according to claim 21, the pseudoviral particle according to claim 22, the packaging system according to claim 23, the modified HIV virus according to claim 24, the isolated nucleic acid molecule according to claim 25, the vector according to claim 26, or the composition according to any one of claims 27-32, for inducing an immune response against HIV in a subject and/or for preventing and/or treating an HIV infection in a subject, or in the manufacture of a medicament for inducing an immune response against HIV in a subject and/or for preventing and/or treating an HIV-infection in a subject;
preferably, the medicament is a vaccine;
preferably, the subject is a human;
preferably, the HIV is HIV-1;
optionally, the recombinant protein, the fusion protein, the multimer, the isolated nucleic acid molecule, the vector, the host cell, the particle, the pseudoviral particle, the packaging system, the modified HIV virus, the composition is administrated, for example simultaneously, separately or sequentially in combination with an antiretroviral agent; preferably, the antiretroviral agent is selected from a nucleoside reverse transcriptase inhibitor, a non-nucleoside reverse transcriptase inhibitor, a protease inhibitor, or a fusion protein inhibitor.

34. A method for inducing an immune response against HIV in a subject or for preventing and/or treating an HIV infection in a subject, which comprises administering to a subject in need thereof an immunologically effective amount of the recombinant protein according to any one of claims 1-13, the fusion protein according to claim 14 or 15 or the multimer according to claim 16, the isolated nucleic acid molecule according to claim 17, the vector according to claim 18, the host cell according to claim 19, the particle according to claim 21, the pseudoviral particle according to claim 22, the packaging system according to claim 23, the modified HIV virus according to claim 24, the isolated nucleic acid molecule according to claim 25, the vector according to claim 26, or the composition according to any one of claims 27-32;
preferably, the method comprises administering an immunogenic composition or vaccine (e.g., a protein vaccine) comprising the recombinant protein according to any one of claims 1-13, the fusion protein according to claim 14 or 15 or the multimer according to claim 16, or the particle according to claim 21;
preferably, the method comprises administering an immunogenic composition or vaccine (e.g., a virus-based vaccine) comprising the pseudoviral particle according to claim 22 or the modified HIV virus according to claim 23;
preferably, the method comprises administering an immunogenic composition or vaccine (e.g., a nucleic acid vaccine) comprising the isolated nucleic acid molecule according to claim 17, the vector according to claim 18, the isolated nucleic acid molecule according to claim 25, or the vector according to claim 26;
preferably, the subject is a human;
preferably, the HIV is HIV-1;
optionally, the recombinant protein, the fusion protein, the multimer, the isolated nucleic acid molecule, the vector, the host cell, the particle, the pseudoviral particle, the packaging system, the modified HIV virus, the composition is administrated, for example simultaneously, separately or sequentially in combination with an antiretroviral agent; preferably, the antiretroviral agent is selected from a nucleoside reverse transcriptase inhibitor, a non-nucleoside reverse transcriptase inhibitor, a protease inhibitor, or a fusion protein inhibitor.
